(19) Europäisches Patentamt
European Patent Office
Office européen des brevets

(11) **EP 1 971 257 B1**

(12) **EUROPEAN PATENT SPECIFICATION**

(45) Date of publication and mention
of the grant of the patent:
**14.10.2015 Bulletin 2015/42**

(51) Int Cl.:
**A61B 6/00** (2006.01)

(21) Application number: **06832278.3**

(22) Date of filing: **28.12.2006**

(86) International application number:
**PCT/IL2006/001511**

(87) International publication number:
**WO 2007/074467 (05.07.2007 Gazette 2007/27)**

(54) **GATING WITH ANATOMICALLY VARYING DURATIONS**

GATING MIT ANATOMISCH VARIIERENDER DAUER

SYNCHRONISATION (GATING) AVEC DUREES VARIABLES EN FONCTION DE L'ANATOMIE

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.12.2005 US 754199 P**
**15.01.2006 PCT/IL2006/000059**
**31.01.2006 US 763458 P**
**11.05.2006 US 799688 P**
**11.05.2006 PCT/IL2006/000562**
**17.05.2006 US 800845 P**
**17.05.2006 US 800846 P**
**28.06.2006 US 816970 P**
**19.07.2006 PCT/IL2006/000840**
**19.07.2006 PCT/IL2006/000834**
**09.11.2006 PCT/IL2006/001291**
**01.12.2006 US 607075**

(43) Date of publication of application:
**24.09.2008 Bulletin 2008/39**

(73) Proprietor: **Biosensors International Group, Ltd.**
**Hamilton HM 11 (BM)**

(72) Inventors:
• **ZIV, Omer**
**76470 Rechovot (IL)**
• **RAVHON, Ran**
**27252 Kiryat-Bialik (IL)**
• **DICHTERMAN, Eli**
**34756 Haifa (IL)**
• **GLUHOVSKY, Leonid**
**34814 Haifa (IL)**
• **BEN-HAIM, Shlomo**
**London W8 4QP (IL)**

• **ROUSSO, Benny**
**75801 Rishon-LeZion (IL)**

(74) Representative: **Dennemeyer & Associates S.A.**
**Poccistrasse 11**
**80336 München (DE)**

(56) References cited:
**WO-A1-00/10034          US-A1- 2004 015 075**
**US-B1- 6 346 706**

• **GILLAND D R ET AL: "A 3D model of non-uniform attenuation and detector response for efficient iterative reconstruction in SPECT" PHYSICS IN MEDICINE AND BIOLOGY UK, vol. 39, no. 3, March 1994 (1994-03), pages 547-561, XP002558623 ISSN: 0031-9155**
• **DAVID R GILLAND ET AL: "Simultaneous Reconstruction and MotionEstimation for Gated Cardiac ECT" IEEE TRANSACTIONS ON NUCLEAR SCIENCE, IEEE SERVICE CENTER, NEW YORK, NY, US, vol. 49, no. 5, 1 October 2002 (2002-10-01), XP011077797 ISSN: 0018-9499**
• **HONGDI LI ET AL: "A HOTlink/networked PC data acquisition and image reconstruction system for a high resolution whole-body PET with respiratory or ECG-gated performance ho" 2002 IEEE NUCLEAR SCIENCE SYMPOSIUM CONFERENCE RECORD. / 2002 IEEE NUCLEAR SCIENCE SYMPOSIUM AND MEDICAL IMAGING CONFERENCE. NORFOLK, VA, NOV. 10 - 16, 2002; [IEEE NUCLEAR SCIENCE SYMPOSIUM CONFERENCE RECORD], NEW YORK, NY : IEEE, US, vol. 2, 10 November 2002 (2002-11-10), pages 1135-1139, XP010663724 ISBN: 978-0-7803-7636-6**

• **XIAOLONG OUYANG ET AL: "Incorporation of correlated structural images in PET image reconstruction", IEEE TRANSACTIONS ON MEDICAL IMAGING USA, vol. 13, no. 4, December 1994 (1994-12), pages 627-640, ISSN: 0278-0062**

**Description**

Some implementations include a method and an apparatus for image reconstruction in nuclear medicine imaging and, some implementations include image reconstruction in nuclear medicine imaging using gating techniques.

Radionuclide imaging aims at obtaining an image of a radioactively labeled substance, that is, a radiopharmaceutical, within the body, following administration, generally, by injection. The substance is chosen so as to be picked up by active pathologies to a different extent from the amount picked up by the surrounding, healthy tissue in consequence; the pathologies are operative as radioactive-emission sources and may be detected by radioactive-emission imaging. Pathology may appear as a concentrated source of high radiation, that is, a hot region, as may be associated with a tumor, or as a region of low-level radiation, which is nonetheless above the background level, as may be associated with carcinoma.

A reversed situation is similarly possible. Dead tissue has practically no pick up of radiopharmaceuticals, and is thus operative as a cold region.

The mechanism of localization of a radiopharmaceutical in a particular organ of interest depends on various processes in the organ of interest such as antigen-antibody reactions, physical trapping of particles, receptor site binding, removal of intentionally damaged cells from circulation, and transport of a chemical species across a cell membrane and into the cell by a normally operative metabolic process. A summary of the mechanisms of localization by radiopharmaceuticals is found in http://www.lunis.luc.edu/nucmed/tutorial/radpharm/i.htm.

The particular choice of a radionuclide for labeling antibodies depends upon the chemistry of the labeling procedure and the isotope nuclear properties, such as the number of gamma rays emitted, their respective energies, the emission of other particles such as beta or positrons, the isotope half-life, and the decay scheme.

In PET imaging, positron-emitting radioisotopes are used for labeling, and the imaging camera detects coincidence photons, the gamma pair of 0.511 Mev, traveling in opposite directions. Each one of the coincident detections defines a line of sight, along which annihilation takes place. As such, PET imaging collects emission events, which occurred in an imaginary tubular section enclosed by the PET detectors. A gold standard for PET imaging is PET $NH_3$ rest myocardial perfusion imaging with N-13-ammonia ($NH_3$), at a dose level of 740 MBq, with attenuation correction. Yet, since the annihilation gamma is of 0.511 Mev, regardless of the radioisotope, PET imaging does not provide spectral information, and does not differentiate between radioisotopes.

In SPECT imaging, primarily gamma emitting radioisotopes are used for labeling, and the imaging camera is designed to detect the actual gamma emission, generally, in an energy range of approximately 11- 511 KeV. Generally, each detecting unit, which represents a single image pixel, has a collimator that defines the solid angle from which radioactive emission events may be detected.

Because PET imaging collects emission events, in the imaginary tubular section enclosed by the PET detectors, while SPECT imaging is limited to the solid collection angles defined by the collimators, generally, PET imaging has a higher sensitivity and spatial resolution than does SPECT. Therefore, the gold standard for spatial and time resolutions in nuclear imaging is defined for PET. For example, there is a gold standard for PET imaging for at rest myocardial perfusion with N-13-ammonia ($NH_3$), at a dose of 740 MBq with attenuation correction."

Conventional SPECT cameras generally employ an Anger camera, in which a single-pixel scintillation detector, such as NaI(Tl), LSO, GSO, CsI, CaF, or the like, is associated with a plurality of photomultipliers. Dedicated algorithms provide a two dimensional image of the scintillations in the single pixel scintillation detector. There are several disadvantages to this system, for example:

1. The dedicated algorithms associated with the single pixel cannot reach the accuracy of a two-dimensional image of a plurality of single pixel detectors;
2. The single-pixel detector is a rigid unit, which does not have the flexibility of motion of a plurality of small detectors, each with independent motion; and
3. A single hot spot may cause the single pixel detector of the Anger camera to saturate, whereas when a plurality of single pixel detectors is employed, saturation is localized to a few pixels and does not affect the whole image.

Other SPECT cameras which employ a plurality of single pixel detectors are also known.

US Patent 6,628,984, to Weinberg, issued on September 30, 2003 and entitled, "Handheld camera with tomographic capability," describes a tomographic imaging system, which includes a moveable detector or detectors capable of detecting gamma radiation; one or more position sensors for determining the position and angulation of the detector(s) in relation to a gamma ray emitting source; and a computational device for integrating the position and angulation of the detector(s) with information as to the energy and distribution of gamma rays detected by the detector and deriving a three dimensional representation of the source based on the integration. A method of imaging a radiation emitting lesion located in a volumetric region of interest also is disclosed.

US Patent 6,242,743, to DeVito, et al., issued on June 5, 2001 and entitled, "Non-orbiting tomographic imaging system," describes a tomographic imaging system which images ionizing radiation such as gamma rays or x rays and which: 1) can produce tomographic images without requiring an orbiting motion of the detector(s) or collimator(s) around the object of interest, 2) produces smaller tomographic systems with enhanced system mobility, and 3) is capable of observing the object of interest from sufficiently many directions to allow multiple time-sequenced tomographic images to be produced. The system consists of a plurality of detector modules which are distributed about or around the object of interest and which fully or partially encircle it. The detector modules are positioned close to the object of interest thereby improving spatial resolution and image quality. The plurality of detectors view a portion of the patient or object of interest simultaneously from a plurality of positions. These attributes are achieved by configuring small modular radiation detector with high-resolution collimators in a combination of application-specific acquisition geometries and non-orbital detector module motion sequences composed of tilting, swiveling and translating motions, and combinations of such motions. Various kinds of module geometry and module or collimator motion sequences are possible, and several combinations of such geometry and motion are shown. The geometric configurations may be fixed or variable during the acquisition or between acquisition intervals. Clinical applications of various embodiments of US Patent 6,242,743 include imaging of the human heart, breast, brain or limbs, or small animals. Methods of using the non-orbiting tomographic imaging system are also included.

US Patent 5,939,724, to Eisen, et al., issued on August 17, 1999, and entitled, "Light weight-camera head and-camera assemblies containing it," describes a lightweight gamma-camera head, assemblies, and kits that embody it. The gamma-camera head has a detector assembly which includes an array of room temperature, solid state spectroscopy grade detectors each associated with a collimator and preamplifier, which detectors and associated collimators and preamplifiers are arranged in parallel rows extending in a first direction and suitably spaced from each other in a second direction normal to the first direction, each of the parallel detector rows holding a plurality of detectors. The head may optionally have an electric motor for moving the detector in the second direction and optionally also in the first direction, either stepwise or continuously.

US Patent 6,525,320, to Juni, issued on February 25, 2003, and entitled, single photon emission computed tomography system, describes a single photon emission computed tomography system, which produces multiple tomographic images of the type representing a three-dimensional distribution of a photon-emitting radioisotope. The system has a base including a patient support for supporting a patient such that a portion of the patient is located in a field of view. A longitudinal axis is defined through the field of view. A detector module is adjacent the field of view and includes a photon-responsive detector. The detector is an elongated strip with a central axis that is generally parallel to the longitudinal axis. The detector is operable to detect if a photon strikes the detector. The detector can also determine a position along the length of the strip where a photon is detected. A photon-blocking member is positioned between the field of view and the detector. The blocking member has an aperture slot for passage of photons aligned with the aperture slot. The slot is generally parallel to the longitudinal axis. A line of response is defined from the detector through the aperture. A displacement device moves either the detector module or the photon-blocking member relative to the other so that the aperture is displaced relative to the detector and the line of response is swept across at least a portion of the field of view.

US Patent 6,271,525, to Majewski, et al., issued on August 7, 2001, and entitled, "Mini gamma camera, camera system and method of use," describes a gamma camera, which comprises essentially and in order from the front outer or gamma ray impinging surface: 1) a collimator, 2) a scintillator layer, 3) a light guide, 4) an array of position sensitive, high resolution photomultiplier tubes, and 5) printed circuitry for receipt of the output of the photomultipliers. There is also described, a system wherein the output supplied by the high resolution, position sensitive photomultipiler tubes is communicated to: a) a digitizer and b) a computer where it is processed using advanced image processing techniques and a specific algorithm to calculate the center of gravity of any abnormality observed during imaging, and c) optional image display and telecommunications ports.

US Patent 6,271,524, to Wainer, et al., issued on August 7, 2001 and entitled, "Gamma ray collimator," describes a gamma ray collimator assembly comprising collimators of different gamma ray acceptance angles. For example, the acceptance angle of a first collimator may be between 0.2 and 5 degrees, and the acceptance angle of a second collimator may be between about 5 and 30 degrees.

US Patent 6,212,423, to Krakovitz, issued on April 3, 2001 and entitled, "Diagnostic hybrid probes," describes a hybrid nuclear and ultrasonic probe, comprising a cylindrical outer casing surrounding a nuclear probe, which comprises two scintillator plates intersecting perpendicularly, each of the scintillator plates having a plurality of parallel collimators; and an ultrasonic probe situated between said casing at the intersection of said scintillator plates.

GILLAND 0 R ET AL: "A 3D model of non-uniform attenuation and detector response for efficient iterative reconstruction in SPECT" PHYSICS IN MEDICINE AND BIOLOGY UK, vol. 39, no. 3, March 1994 (1994-03), pages 547-561, describes a 3D model which is a refinement of previously described models, incorporating three significant improvements. First. non-uniform tissue density information, like that obtained from reconstructed transmission computed tomography data, has been incorporated into the model. Second, a means of automatically identifying the active voxel region (section 2.4) has been implemented. Finally, the artifact problem inherent in the active voxel approach has been

**EP 1 971 257 B1**

remedied 25through a new technique called region-dependent reconstruction (IIDR)

International Publication Number WO 00/10034 describes a method of applying scatter and attenuation correction to emission tomography images of a region of interest of a patient under observation comprises the steps of aligning a three-dimensional computer model representing the density distribution within the region of interest with the emission tomography images and applying scatter and attenuation correction to the emission tomography images using the aligned computer model as a guide.

DAVID R GILLAND ET AL: "Simultaneous Reconstruction and Motion Estimation for Gated Cardiac ECT" IEEE TRANSACTIONS ON NUCLEAR SCIENCE, IEEE SERVICE CENTER, NEW YORK, NY, US, vol. 49, no. 5, 1 October 2002 (2002-10-01), describes a processing method for gated cardiac emission computed tomography that simultaneously reconstructs the pixel intensities of the gated images and estimates the motion of the cardiac wall. The simultaneous reconstruction and motion estimation is achieved using conjugate gradient optimization with an objective function that is dependent on the gated reconstructed images at two time frames and the estimated motion of the object between the two frames.

HONGDI LI ET AL: "A HOTlinkinetworked PC data acquisition and image reconstruction system for a high resolution whole-body PET with respiratory or ECG gated performance ho" 2002 IEEE NUCLEAR SCIENCE SYMPOSIUM CONFERENCE RECORD/2002 IEEE NUCLEAR SCIENCE SYMPOSIUM AND MEDICAL IMAGING CONFERENCE. NORFOLK, VA, NOV. 10 -16, 2002; [IEEE NUCLEAR SCIENCE SYMPOSIUM CONFERENCE RECORD], NEW YORK, NY: IEEE, US, vol. 2, 10 November 2002 (2002-11-10), pages 1135-1139, an ultra high resolution PET camera in whole body scanning or gated imaging study needs super computer processing power for creating a huge sinogram as well as doing image reconstruction.

XIAOLONG OUYANG ET AL: "Incorporation of correlated structural images in the PET image reconstruction", IEEE TRANSACTION ON MEDICAL IMAGING USA, vol. 13, no. 4, December 1994 (1994-12), describes spatially correlated MR or X-ray CT images employed on images reconstructed according to the Bayesian reconstruction.

List mode data acquisition is known in PET studies, and enables the determination of coincidence. It relates to recording every radiation event together with data pertinent to that event, which includes:

    i. the time the radiation event impinged upon a detector pixel, with respect to a clock, with respect to a time bin, or with respect to another time definition, for example, a time interval between two clock signals; and
    ii. the detector pixel location with respect to a coordinate system, at the time of the impinging.

The knowledge of time and location enables the determination of coincidence counts, namely photon counts that arrive substantially simultaneously, 180 degrees apart. The time and location data may be stamped onto the radiation-event data packet, for example, as a header or as a footer, or otherwise associated with the radiation-event data packet, as known.

The time-stamped data available in PET studies may further be used for perfusion studies, where the timing of physiological processes of short durations, that is, durations shorter than about half the time span between heartbeats, is important. Perfusion studies usually involve a sequence of continuous acquisitions, each of which may represent data acquisition duration of about 10 - 30 seconds, although longer durations are sometimes employed. Data from each of the frames is independently reconstructed to form a set of images that can be visualized and used to estimate physiological parameters. This approach involves selection of the set of acquisition times, where one must choose between collecting longer scans with good counting statistics but poor temporal resolution, or shorter scans that are noisy but preserve temporal resolution.

US Patent Application 2003010539, to Turner, et al., published on June 5, 2003, and entitled, "X-ray and gamma ray detector readout system," describes a readout electronics scheme, under development for high resolution, compact PET (positron emission tomography) imagers, using time tagging, based on LSO (lutetium ortho-oxysilicate, Lu.sub.2SiO.sub.5) scintillator and avalanche photodiode (APD) arrays.

There is some work relating to timing data in SPECT systems, employing Anger cameras.

US Patent 5,722,405, to Goldberg, issued on March 3, 1998, and entitled, "Method and apparatus for acquisition and processsing of event data in semi list mode," describes a system for acquisition, processing and display of gated SPECT imaging data for use in diagnosing Coronary Artery Disease (CAD) in nuclear medicine, employing an Anger camera, and provides a physician with two parameters for evaluating CAD: information relating to the distribution of blood flow within the myocardium (perfusion) and information relating to myocardium wall motion (function). One aspect provides the physician with a display of functional images representing quantitative information relating to both perfusion and function with respect to selected regions of interest of the subject heart at end-diastole and end-systole segments of the cardiac cycle. The functional display consists of arcs of varied width depending on wall motion and color coded to illustrate degrees of myocardial perfusion for different pie shaped sections of a selected region of interest within a given short axis slice of reconstructed volumetric region data. Another aspect provides a series of display images allowing facilitated access, display, and comparison of the numerous image frames of the heart that may be collected during gated SPECT sessions. US Patent 5,722,405 also teaches the ability to define and recall parameter files representative of data acquisition and processing parameters and protocol for use in gated SPECT studies and includes a semi-list processing mode to increase efficiency of data acquisition within a camera computer system.

US Patent 7,026,623, to Oaknin, et al., issued on April 11, 2006, and entitled, "Efficient single photon emission imaging," describes a method of diagnostic imaging in a shortened acquisition time for obtaining a reconstructed diagnostic image of a portion of a body of a human patient who has been administered with dosage of radiopharmaceutical substance radiating gamma rays, using SPECT and an Anger camera. The method comprises acquiring photons emitted from said portion of the body, by means of a detector capable of converting the photons into electric signals, wherein the total time of photon acquiring is substantially shorter than the clinically acceptable acquisition time; processing said electric signals by a position logic circuitry and thereby deriving data indicative of positions on said photon detector crystal, where the photons have impinged the detector; andreconstructing an image of a spatial distribution of the pharmaceutical substance within the portion of the body by iteratively processing said data. For example, the method includes effective acquisition time of less than 10 minutes, or less than 8 minutes, and acquiring photons in a list-mode procedure.

Current techniques record data with SPECT and electrocardiogram (ECG), and perform some gating to the data which is captured by the SPECT detectors, to incorporate the global and regional atrial and ventricular function and assessment of the relationship of perfusion to regional function.

Gated images are used to overcome distortions such as motion artifacts, which are caused due to motion of the heart during image acquisition. The images are needed as the physical model used for reconstruction assumes that the imaged objects are static. In gated imaging, photon-counting takes into account the portion of the heart contraction cycle within which a photon is measured. The Gating enables the reconstruction of an anatomical structure which is subject to periodic motion by enabling image acquisition only when the structure has reached the same configuration. Cardiac contraction is usually synchronized to the recorded electrocardiogram (ECG) signal that indicates the current heart pose. The period between a certain repetitive wave, such as R-wave, and a subsequent wave is divided into several time segments, called "frames", which are usually spaced evenly. Each photon which is detected by the PET detectors during one of the frames is collected and associated with the related frame.

In gated imaging, each frame generates a single dataset. The collection of all the datasets belonging to all the frames are defined as a "dynamic" dataset.

The dynamic dataset is created by dividing the time span between one R-wave to the next R-wave into M frames that usually have an identical duration. Each detected photon is accumulated into a dataset of one of the M frames. Each dataset of the M datasets contains data relevant to a defined portion ("snapshot") within the cardiac cycle.

Usually, during the image reconstruction process, each one of the gated datasets of the M frames is processed independently by a suitable reconstruction algorithm, see Leahy R et al., Computer tomography in: Handbook of Image and Video Processing, BovikA, Academic press, 2000, pp. 771-787; J. Kay. The EM algorithm in medical imaging, Stat. Meth. Med. Res., 6(1):55-75, January 1997; J. A. Fessler, Statistical image reconstruction methods for transmission tomography, Handbook of Medical Imaging, Volumetric region 2, pages 1-70. SPIE, Bellingham, 2000; R. M. Leahy et al., Statistical approaches in quantitative positron emission tomography, 10(2):147-65, April 2000; M. Defrise, A short reader's guide to 3D tomographic reconstruction, Computerized Medical Imaging and Graphics, 25(2):1 13-6, March 2001; Vandenberghe, Y. D'Asseler, et al. Iterative reconstruction algorithms in nuclear medicine, Computerized Medical Imaging and Graphics, 25(2):105-11, March 2001; G. L. Zeng. Image reconstruction, a tutorial, Computerized Medical Imaging and Graphics, 25(2):97-103, March 2001; and R. M. Lewitt et al., Overview of methods for image reconstruction from projections in emission computed tomography, Proc. IEEE, 91(9):1588-611, October2003.

A common practice in gated SPECT reconstruction is to divide the gated dynamic dataset into M 'non-gated' data sets. Each one of the datasets includes data from a single frame i. The reconstruction of each volumetric region is performed independently using the relevant data set.

In particular, once the emission data is obtained, the data is processed to reconstruct the intensity distribution within the measured volumetric region. The reconstruction process is generally complex, due to the large quantity of data that must be processed in order to obtain an accurate reconstruction. The following prior art statistical model may be used to perform reconstruction.

We assume an intensity distribution, I, defined over an input overall volume U, where U denotes a set of basic elements, such as pixels in two dimensional overall volumes and voxels in three dimensional overall volumes, and I(u) is the intensity of a given basic element $u \in U$. A detecting unit positioned on a radiation-emission-measuring-probe such as a PET detector or the like takes a series of measurements $y = \left( y_t \right)_{t=1}^{T}$ from different positions and orientations around the volumetric region U. The geometrical and physical properties of the detecting unit, together with its position and orientation in a given measurement t, determine the detection probability $\phi t(u)$ of a photon emitted from location u in time t. Thus, the effective intensity of location u as viewed by the detecting unit during measurement t is $\phi t(u)I(u)$.

The random count Xt(u) of photons that are emitted from location u and detected in measurement t is modeled by a Poisson process with mean $\phi t(u)I(u)$. The total count of photons detected in measurement t is $Yt = \sum_{u \in U} Xt(u)$, and the reconstruction problem is to reconstruct the intensities $(I(u))_{u \in U}$ from the measurements $\left(y_t\right)_{t=1}^{T}$.

The 2-D Radon transform is a mathematical relationship that may be used for reconstructing the emission intensities of volumetric region U when the set of measurements $\left(y_t\right)_{t=1}^{T}$ is unconstrained. The Radon transform is not statistical and does not take into account the Poissonian nature of the counts. In addition, it models the views as line projections. The Radon transform maps the spatial domain (x,y) to the Radon domain (p,$\phi$). For a fixed projection angle, the Radon transform is simply a projection of the object. A technique known in the ART as filtered back-projection (FBP) uses a back-projection operator and the inverse of the Radon transform to reconstruct the intensity distribution in volumetric region U from measurements $\left(y_t\right)_{t=1}^{T}$.

The basic, idealized problem solved by the FBP approach is to reconstruct an image from its Radon transform. The Radon transform, when properly defined, has a well-defined inverse. However, in order to invert the transform one needs measured data spanning 180°. In many medical imaging situations, the positioning of the detecting unit relative to the emitting object is constrained, so that complete measured data is not available. Reconstruction based on filtered back-projection is therefore of limited use for medical imaging. Maximum likelihood (ML) and Maximum A Posteriori (MAP) estimation methods, which address the statistical nature of the counts, have been found to provide better image reconstructions than FBP.

Limited-angle tomography is a reconstruction technique in the related art which reconstructs an image from projections acquired over a limited range of angular directions. The success of the reconstruction process depends upon the extent of the angular range acquired compared with the angular range of the missing projections. Any reconstruction from a limited range of projections potentially results in spatial distortions (artifacts) in the image. Limited angle techniques can be applied for both the Radon transform and the statistical models, but better results are generally achieved within the statistical framework. While it is known that the severity of the artifacts increases with the increasing angular range of the missing projections, limited-angle tomography does not provide information on which projections should be used in order to most effectively reconstruct the image.

ML estimation is a widely used method in the related art for reconstructing an image from a constrained set of measurements. A parameterization of the generative model described above is obtained by assigning an intensity I(u) to every voxel in U. The likelihood of the observed data y = (yt)t, given the set of parameters $\mathbf{I} = \{I(u) : u \in U\}$ is:

$$
\begin{aligned}
L(\mathbf{y}|\mathbf{I}) = \ln P(\mathbf{y}|\mathbf{I}) \quad &= \quad \ln \prod_t P(y_t|\mathbf{I}) = \sum_t \ln P\left(\sum_u x_t(u) \mid \mathbf{I}\right) \\
&= \quad \sum_t \ln \text{Poisson}\left(y_t \mid \sum_u \phi_t(u)I(u)\right) \\
&= \quad \sum_t \left\{ -\sum_u \phi_t(u)I(u) + y_t \ln \sum_u \phi_t(u)I(u) - \ln(y_t!) \right\}
\end{aligned}
$$

$$(1)$$

Note that the lower and upper bound of an indexing variable, such as voxels u and time index t, are omitted in the following description, when they are clear from the context.

There is currently no analytic way to solve Eqn. 1 for the maximum of the likelihood function. However, optimization methods that find local maxima of the likelihood are known. One such method is the Expectation-Maximization (EM) process.

Since the data generated by the model is only partially observable by our measurements, a basic ingredient of the EM formalism is to define a set of random variables that completely define the data generated by the model. In the current case, since $Yt = \sum_u Xt(u)$, the set of variables $\{Xu(t) : u \in U ; t = 1, ..., T\}$ is such a set; the generated data is x = (xt)t, where xt = (xt(u))u, and the observed data y is completely determined by x. The main tool in the EM formalism is the complete data likelihood:

$$
\begin{aligned}
\ln P(\mathbf{x}|I) \quad &= \quad \ln \prod_t P(\mathbf{x}_t|I) = \sum_t \ln \prod_u \text{Poisson}(x_t(u) \mid \phi_t(u)I(u)) \\
&= \quad \sum_t \sum_u \{ -\phi_t(u)I(u) + x_t(u) \ln(\phi_t(u)I(u)) + \ln(x_t(u)!) \}
\end{aligned}
$$

$$(2)$$

Since the likelihood depends on the complete data, which is only partially observable, we take its expectation with respect to the overall volume of the unobserved data, given the current set of hypothesized parameters (i.e. the current estimator). The result is a function Q(I|I') which assigns likelihood to sets I of model parameters, given the current set I', and given the observed data y:

$$Q(\mathbf{I}|\mathbf{I}') \;=\; E[\ln P(\mathbf{x}|\mathbf{I})\,|\,\mathbf{y};\mathbf{I}']$$
$$=\; \sum_t \sum_u \{-\phi_t(u)I(u) + E[x_t(u)|y_t;\mathbf{I}']\ln(\phi_t(u)I(u)) + C\}$$

$$(3)$$

where C is a term which is independent of the intensities I. The function Q(I|I') is maximized by the following new estimates:

$$I(u) = \frac{1}{\sum_t \phi_t(u)} \sum_t E[x_t(u)\,|\,y_t;\mathbf{I}'] \quad ; \quad \forall\, u \in U$$

$$(4)$$

The expectation in Equation 4 is obtained as follows:

$$P_{X_t(u)}(x_t(u)|y_t;\mathbf{I}') = \frac{P_{Y_t}(y_t|x_t(u);\mathbf{I}')\; P_{X_t(u)}(x_t(u)|\mathbf{I}')}{P_{Y_t}(y_t|\mathbf{I}')}$$
$$=\; \frac{\text{Poisson}(y_t - x_t(u)|\sum_{v \neq u}\phi_t(v)I'(v))\; \text{Poisson}(x_t(u)\,|\,\phi_t(u)I'(u))}{\text{Poisson}(y_t\,|\,\sum_v \phi_t(v)I(v))}$$
$$=\; \text{Binomial}(x_t(u)\,|\,\frac{\phi_t(u)I'(u)}{\sum_v \phi_t(v)I'(v)};\,y_t)$$

$$(5)$$

It follows that $E[xt(u)|yt;\mathbf{I}'] = y_t \dfrac{\phi_t(u)I'(u)}{\sum_v \phi_t(v)I'(v)}$, and hence the EM iteration is:

$$I(u) = \frac{1}{\sum_t \phi_t(u)} \sum_t y_t \frac{\phi_t(u)I'(u)}{\sum_v \phi_t(v)I'(v)}$$

$$(6)$$

It is provable that each EM iteration improves the likelihood. Thus, given a random starting estimator, the EM algorithm iterates the above improvement step until it converges to a local maximum of the likelihood. Several random starts increase the chance of finding a globally good estimator.

It is usually desired to maximize the expected posterior probability (given a proper prior) rather than the expected likelihood. In that case we assume a prior probability on the intensities P(I) = ΠuP(I(u)). A proper conjugate prior for the Poisson distribution is the Gamma distribution:

$$P(I(u)) = \text{Gamma}(I(u)\,|\,\alpha_u;\beta_u) = \frac{\beta_u^{\alpha_u+1}}{\Gamma(\alpha_u+1)}I(u)^{\alpha_u}e^{-\beta_u I(u)}$$

$$(7)$$

Now the maximization is done on Q(I|I') = E[lnP(x|I)p(I) |y; I']. Plugging the Gamma prior into Q, and solving for I(u), we get the following EM iteration for the maximum posterior estimation:

$$I(u) \;=\; \frac{\alpha_u + \sum_t E[x_t(u)|y_t;\mathbf{I}']}{\beta_u + \sum \phi_t(u)}$$

$$(8)$$

$$= \frac{1}{\beta_u + \sum_t \phi_t(u)}\left[\alpha_u + \sum_t y_{it}\frac{\phi_t(u)I'(u)}{\sum_v \phi_t(u)I'(v)}\right] \tag{9}$$

The EM update step can be formulated in matrix notation as follows. Let $\Phi$ be the matrix of the projections $[\phi t(u)]t,u$, and let I, I',y,$\alpha$ and $\beta$ be represented as column vectors. Equation 8 can be written in vector and matrix notations as:

$$\mathbf{I} = \frac{\alpha + \mathbf{I'} \cdot \left(\Phi^T \frac{\mathbf{y}}{\Phi \mathbf{I'}}\right)}{\beta + \Phi^T \mathbf{1}} \tag{10}$$

where the explicit multiplication and division denote element-wise operations, and where 1 is a vector (of the appropriate length) consisting solely of 1's.

Limited computational resources (i.e., when the entire projection matrix $\Phi$ cannot be kept in memory) may require breaking the update computation according to a partition of $\Phi$ into a set of sub-matrices ($\Phi i$). In that case the intensities can be updated gradually (using only one sub-matrix at each step) according to the following computation:

$$\mathbf{I} = \frac{\alpha + \mathbf{I'} \cdot \sum_i \Phi_i^T \frac{\mathbf{y_i}}{\Phi_i \mathbf{I'}}}{\beta + \sum_i \Phi_i^T \mathbf{1}} \tag{11}$$

where yi denotes the vector of observations that are obtained using the views of $\Phi i$.

In order to achieve a reconstructed image which is adequate for medical diagnostic and treatment purposes, a high-resolution image of the tested object must be obtained. When high-resolution detecting units are used, their efficiency is relatively low, and the detecting units must remain at each position for a relatively long time in order to achieve a high probability of detection. Since during medical testing, measurements are generally performed at many locations as the detecting unit is moved relative to the observed organ, the testing procedure generally requires a long time and is physically and emotionally difficult for the patient. Additionally, reconstruction is based upon a large quantity of data, and is a lengthy and computationally complex process.

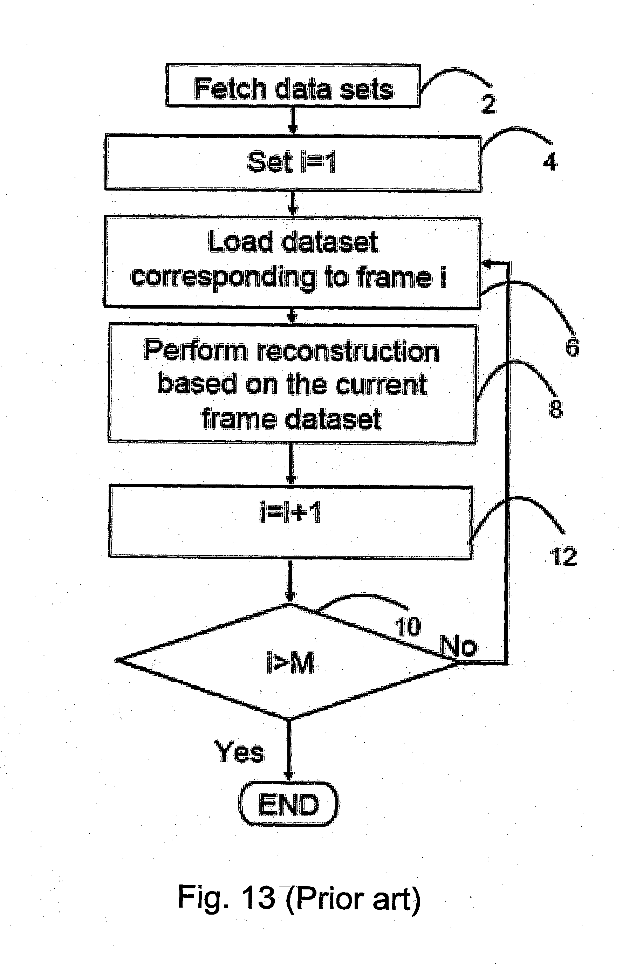

Reference is now made to Figure 13, which is a schematic flowchart that illustrates steps of a typical prior art gated image reconstruction method. In Figure 13, i denotes a frame counter and M denotes the number of frames. Usually, after all the M datasets are fetched, as shown at 2, and i is set with 1, as shown at 4, the dataset that corresponds with frame i is loaded into the processing unit, as shown at 6. During the following step, as shown at 8, the processing unit is used to perform a frame reconstruction according to the dataset that corresponds with frame i using any suitable reconstruction algorithm known in the art, such as the aforementioned EM algorithm, ordered subset expectation maximization (OSEM), and algebraic reconstruction techniques (ART) FBP.

As shown at 12, after the reconstruction is completed, the frame counter i is incremented by 1. If i is larger than M and there are no more frame the process ends. If i is not larger than M, the next dataset that corresponds with the subsequent frame is loading for reconstruction. In such a manner, the generation of a static imaging of the heart in a specific configuration becomes possible.

However, a known problem of such a method is the high computational load that is needed for the execution thereof. Even when using an ordered set method, such as the aforementioned Hudson et al. method, which is capable of reducing the computational load while giving similar results, the computational power needed to obtain a good image reconstruction is still quite high. Such a high computational load results in a longer reconstruction time that reduces the throughput of the processing unit and requires a more expensive processing hardware.

There is thus a widely recognized need for, and it would be highly advantageous to have, a method and an apparatus for image reconstruction in nuclear medicine imaging devoid of the above limitations.

The present invention relates to a method for reconstructing a radioactive emission image of an overall volume (1001) having first and second volumetric regions (1003, 1002), each volumetric region having respectively independent dynamic and static characteristics, as defined in claim 1

Preferably the method of the present invention further comprises representing said initial radioactive emission image by a system (202) of voxels; providing a structural image (200) of a region of interest in said system (202) of voxels; and constructing an anatomical construction (222, 224, 228) of voxels, for said region of interest, in which voxel boundaries are aligned with boundaries of structural objects (202, 204, 206, 208, 218) of said region of interest, based on said structural image (200) wherein said separately reconstructing (1304-1306, 1312-1314) is based on said anatomical construction (222, 224, 228) of voxels.

Preferably said structural image is provided by a source selected from the group consisting of 2-D ultrasound, 3-D ultrasound, planner x-rays, CT x-rays, MRI, and an anatomical atlas.

Preferably said structural image is co-registered to said initial radioactive emission image

Preferably said anatomical construction (222, 224, 228) of voxels includes voxels of varying volumetric regions, depending on their anatomic position and relevance

Preferably said obtaining (1301) radioactive emissions further includes obtaining timing data by a method selected from the group consisting of time stamping, time binning, and a combination thereof.

Preferably said timing data are employed in gating, and further different gating is employed for the first and second volumetric regions (1003, 1002), based on the respectively independent dynamic and static characteristics.

Preferably each of said gating may be of a dynamically varying duration.

Preferably said obtaining (1301) radioactive emissions includes obtaining without timing data, by pre-scanning, for defining the first and second volumetric regions (1003, 1002), and further including:

obtaining a second set of radioactive emissions from the overall volume (1001), including the volumetric regions (1003, 1002), after said reconstructing (1302) said initial radioactive emission image, said second set including timing data, wherein said separately reconstructing (1304-1306, 1312-1314) includes separately reconstructing the first and second volumetric regions (1003, 1002) according to the respectively independent dynamic and static characteristics, from said second set.

Preferably said timing data are employed in gating, and further wherein different gating is employed for the first and second volumetric regions (1003, 1002), based on the respectively independent dynamic and static characteristics.

Preferably each of said gating may be of a dynamically varying duration.

Preferably it further comprises displaying said radioactive emission image and allowing a system user to mark at least one of the first and second volumetric regions (1003, 1002), said segmenting (1301, 1310, 1311) being performed according to said mark.

Preferably said segmenting (1301, 1310, 1311) said initial radioactive emission image is based on the respectively independent dynamic and static characteristics.

Preferably the volumetric regions (1003, 1002) comprise independent sub-volumetric regions, wherein during said separately reconstructing (1304-1306, 1312-1314), each one of said independent sub-volumetric regions is separately reconstructed.

Preferably said radioactive emissions are obtained (1301) using a single photon emission computed tomography (SPECT) camera (10).

Unless otherwise defined, all technical and scientific terms used herein have the same meaning as commonly understood by one of ordinary skill in the art to which this invention belongs. The materials, methods, and examples provided herein are illustrative only and not intended to be limiting.

Implementation of the method and system of the present invention involves performing or completing certain selected tasks or steps manually, automatically, or a combination thereof. Moreover, according to actual instrumentation and equipment of preferred embodiments of the method and system of the present invention, several selected steps may be implemented by hardware or by software on any operating system of any firmware or a combination thereof. For example, as hardware, selected steps of the invention may be implemented as a chip or a circuit. As software, selected steps of the invention may be implemented as a plurality of software instructions being executed by a computer using any suitable operating system. In any case, selected steps of the method and system of the invention may be described as being performed by a data processor, such as a computing platform for executing a plurality of instructions.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

Figures 1A - 1D schematically illustrate a dynamic SPECT camera, in accordance with some implementations;

Figures 2A and 2B schematically illustrate the camera structure with the assemblies, in accordance with an embodiment of the present invention.

Figures 3A - 3D schematically illustrate viewing positions, in accordance with some implementations.

Figures 4A - 4F schematically illustrate stereo views and cross views, in accordance with some embodiments.

Figures 5A and 5B illustrate experimental radiopharmaceutical data, as known;

Figures 5C - 5F illustrate cardiac gating, in accordance with some implementations;

Figure 6A - 6I illustrate an intracorporeal dynamic SPECT camera, in accordance with some implementations;

Figure 7 illustrates assembly-damping parameters, in accordance with some implementations;

Figures 8A and 8B schematically illustrate grid and anatomical construction of voxels, in accordance with some implementations;

Figures 9A - 9J present experimental data, obtained by the dynamic SPECT camera, in accordance with some implementations;

Figure 10 presents experimental data, obtained by the dynamic SPECT camera, in accordance with some implementations;

Figure 11 illustrates components of the dynamic SPECT camera, in accordance with some implementations

Figure 12 illustrates an electrical scheme, in accordance with some implementations;

Figure 13 is a schematic flowchart that illustrates steps of a typical prior art gated image reconstruction method;

Figure 14 is a schematic illustration of an apparatus for reconstructing a radioactive emission image of an input overall volume having dynamic and static volumetric regions, according to a preferred embodiment;

Figure 15 is a schematic isometric view of the input overall volume that is depicted in Figure 14, according to one embodiment;

Figure 16 is a schematic cross-sectional view of the input overall volume of Figure 15, according to one embodiment;

Figure 17 is a flowchart that depicts a method for reconstruction an input overall volume using anatomically varying time-bin lengths, according to one embodiment;

Figure 18 is a graphical representation of a one-dimensional vector of voxels that represents the reconstruction of the input overall volume, according to one embodiment;

Figure 19 is another flowchart that depicts another method for reconstruction an input overall volume using anatomically varying time-bin lengths, according to another embodiment; and

Figure 20 is a graphical representation of a position of two selected sub-regions in two sequential frames, according to another embodiment.

## DESCRIPTION OF THE PREFERRED EMBODIMENTS

In some implementations a radioactive emission image of a overall volume having dynamic and static volumetric regions is reconstructed. The reconstructing is based on gated images with anatomically varying time-bin lengths. The apparatus and the method are designed to allow the segmentation of the radioactive emission image to gated and non-gated regions. In such a manner, the reconstructions radioactive emissions from the dynamic volumetric region and the static volumetric region are carried out separately. Thus, the high computational throughput that is needed in order to reconstruct a dynamic volumetric region, such as the heart, using time binning techniques has less or no effect on the reconstruction of the static volumetric region, as further described below. The disclosed apparatus comprises a number of detectors, such as PET or SPECT detectors, which are designed for obtaining radioactive emissions from the overall volume and an image reconstruction module that is designed for generating radioactive emission images of the overall volume according to the obtained radioactive emissions. The apparatus further comprises a segmentation module that is designed for segmenting an initial radioactive emission image to gated and non-gated regions, according to the dynamic and static volumetric regions of the overall volume. The image reconstruction module is designed to reconstruct separately the gated and non-gated regions in the radioactive emission image respectively according to radioactive emissions the dynamic and static volumetric regions.

The method for reconstructing a radioactive emission image of a overall volume having static and dynamic volumetric regions comprises several steps. During the first step, radioactive emissions are obtained from the overall volume. Then, an initial radioactive emission image of the overall volume is reconstructed according to the radioactive-emission data. In the following step, the initial radioactive emission image is segmented to gated and non-gated regions, respectively according to the dynamic and static volumetric regions. During the last step, the radioactive emission image is reconstructed, wherein the gated region is according to radioactive emissions from said dynamic volumetric region and the non-gated region is separately reconstructed according to radioactive emissions from the static volumetric region.

In another embodiment, only the dynamic volumetric region is reconstructed using time binning. The static volumetric region is reconstructed according to the initial radioactive emission image. Preferably, time binning of different anatomical segments has dynamically varying time-bin lengths, as further described below.

The principles and operation of the dynamic SPECT camera according to some implementations may be better understood with reference to the drawings and accompanying descriptions.

The principles and operation of an apparatus and method according to the present invention may be better understood with reference to the drawings and accompanying description.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

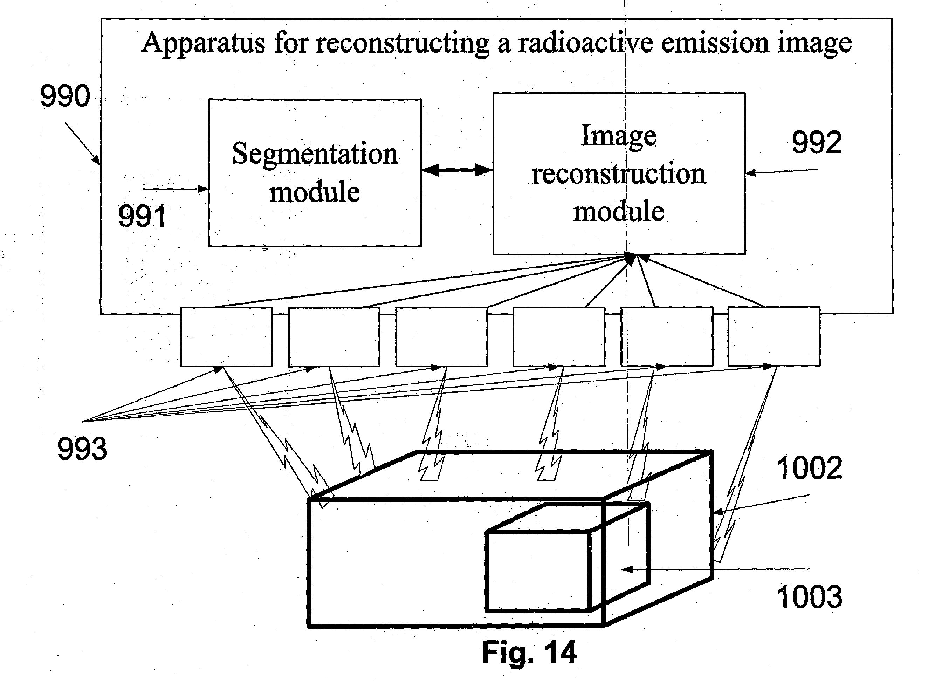

Reference is now made to Figure 14, which is a schematic illustration of an apparatus 990 for reconstructing a radioactive emission image of an input overall volume 1002 having dynamic 1003 and static 1002 volumetric regions, according to an implementation. In one implementation, the input overall volume 1002 is the thorax, the static volumetric region is the related viscus, and the dynamic volumetric region is the heart and the area that confines it. The apparatus 990, which is preferably a SPECT camera, has a number of detecting units 993, such as SPECT detectors. Each one of the detectors 993 is designed for obtaining radiation emission that is emitted from the input overall volume 1002, as described below, and for generating accordingly radioactive-emission data. The apparatus 990 comprises an image reconstruction module 992 that is connected to the detecting units 993. The image reconstruction module 992 is designed for generating radioactive emission images according to the radioactive-emission data. The images are preferably gated, as described below. The apparatus 990 further comprises a segmentation module 991 that is designed for segmenting an initial radioactive emission image, which has been generated by the image reconstruction module 992, to gated and non-gated regions according to the dynamic and static volumetric regions. The gated and non-gated regions are used by the image reconstruction module 992 for separately reconstructing the dynamic and static volumetric regions, as further described below in the anatomically varying time-bin lengths section.

Reference is now made to a more elaborated description of a preferred apparatus 990.

DYNAMIC SPECT CAMERA

Design Description of the Dynamic SPECT Camera

As described above, in one implementation, the apparatus 990 is a dynamic SPECT camera. Hereinbelow a description of a dynamic SPECT camera with temporal and spatial resolutions, which meet and even outperforms those of PET, and with a high spectral resolution not available in PET is given.

Temporal resolution, as used herein, relates to a minimal acquisition time for a tomographic reconstruction image of a predetermined volumetric region, for example 15 X 15 X 15 cubic centimeters, and predetermined spatial resolution, for example, 10 X 10 X 10 cubic millimeters. The minimal acquisition time may be, for example, 30 seconds, 10 seconds, or 1 second.

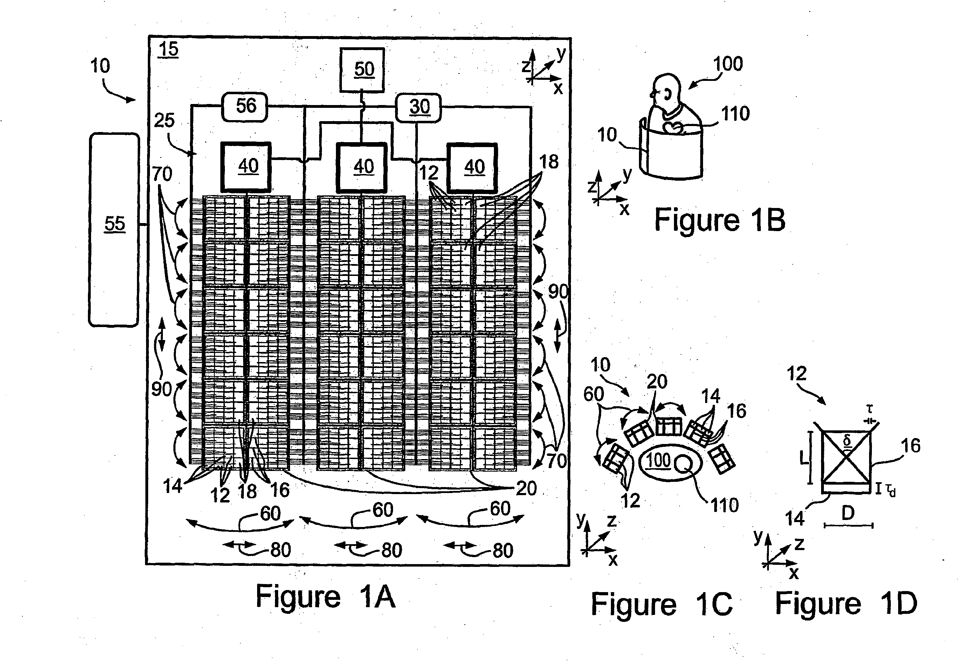

Reference is now made to Figures 1A - 1D, which schematically illustrate a dynamic SPECT camera 10 that is configured for capturing gated images and non-gated image, in accordance with some implementations. The dynamic SPECT camera 10 comprises: an overall structure 15, which defines proximal and distal ends and, with respect to a body 100; a number of assemblies 20, for example, 6, 9, or 16 assemblies 20, arranged on the overall structure 15, forming an array 25 of the assemblies 20. Each one the each assemblies 20 comprises a number of detecting units 12. Each detecting unit 12 includes a single-pixel detector 14 for detecting radioactive emissions and a dedicated collimator 16, attached to the single-pixel detector 14, at the proximal end thereof, for defining a solid collection angle $\delta$ for the detecting unit 12.

Additionally, each assembly 20 comprises an assembly motion provider 40, configured for providing the assembly 20 with individual assembly motion, with respect to the overall structure 15, during the acquisition of radioactive-emission data for a tomographic image.

The dynamic SPECT camera 10 further includes a timing mechanism 30, in communication with each single-pixel detector 14, configured for enabling time binning of the radioactive emissions impinging upon each single-pixel detector 14 to periods, which are not greater than substantially 30 seconds. As the timing mechanism 30 has can control each one of the single-pixel detector 14 separately, each one of the single-pixel detectors 14 can be configured according to a different time binning scheme. In one implementation, the time binning scheme, which is applied to a certain detector, is determined according to the region in the input overall volume that the detector is designed to detect.

The dynamic SPECT camera 10 further includes a position tracker 50, which is designed for providing information on the position and orientation of each detecting unit 12, with respect to the overall structure 15, substantially at all times, during the individual assembly motion.

The dynamic SPECT camera 10 is configured for acquiring a tomographic reconstruction image of a region of interest of about 15 X 15 X 15 cubic centimeters, for example, of a target organ 110, such as a heart or a stomach, during an acquisition period no greater than 300 seconds, at a spatial resolution of at least 10 X 10 X 10 cubic millimeters.

It will be appreciated that the time period may be no greater than 200 seconds, 100 seconds, 60 seconds, 30 seconds, 10 seconds, or 1 second.

Additionally, the dynamic SPECT camera 10 is configured for acquiring a series of tomographic reconstruction images of a region of interest, as a function of time, at a rate of at least a tomographic reconstruction image every 300 seconds.

Again, the rate may further be every 200 seconds, 100 seconds, 60 seconds, 30 seconds, 10 seconds, or 1 second.

In some implementations, the individual assembly motion may be, for example, an assembly oscillatory sweeping motion, as described by an arrow 60. Additionally or alternatively, the individual assembly motion may be a first oscillatory lateral motion, as described by an arrow 80. Additionally or alternatively, the individual assembly motion may be a second oscillatory lateral motion, orthogonal to the first, as described by an arrow 90. Thus, the assembly motion provider 40 may comprise between one and three motion providing units, for the different assembly motions.

Alternatively, the individual assembly motion is an assembly oscillatory sweeping motion, as described by an arrow 60, while the array 25 moves with either the first or the second oscillatory lateral motions, described by the arrows 80 and 90, or with both.

Additionally, the detecting units 12 may be grouped into square or rectangular blocks 18, for example, of 4 X 4 detecting units 12, as seen in Figure 1A, or of 16 X 16, 64 X 64, 64 X 128 or another number of detecting units 12. Furthermore, the blocks 18 may be provided with individual block oscillatory sweeping motion, as described by an arrow 70, with respect to the overall structure 15, during the acquisition of radioactive-emission data for a tomographic image. Preferably, the block oscillatory sweeping motion is orthogonal to, or at an angle to the assembly oscillatory sweeping motion, described by the arrow 60. Thus, the assembly motion provider 40 may further comprise a dedicated block motion providing unit, in communication with each block of an assembly.

A control unit 55 may be integrated with the dynamic SPECT camera 10, to form a single physical unit, or in communication with the dynamic SPECT camera 10.

A spectral selection mechanism 56, in communication with each of the detecting unit 12, is discussed hereinbelow, under the heading, "dynamically varying spectral bins."

The body 100 may be a human or an animal, and the region of interest, or the target organ 110 may be a heart, a brain, a breast, a stomach, a GI tract, a colon, a prostate, a uterus, a cervix, a vagina, a throat, a gland, a lymph node, a portion of skin, a portion of bone, portion of another tissue, or another body portion.

As seen in Figures 1A and 1B, a reference x;y;z coordinate system illustrates a preferred orientation of the dynamic SPECT camera 10 with respect to the body 100, wherein z runs along a length of the body 100. For convenience, the assembly axis along the assembly length will be referred to as the assembly longitudinal axis, and the assembly axis along the assembly width will be referred to as the assembly traverse axis.

Preferably, the assemblies 20 are long and narrow columns, arranged longitudinally against the body 100, wherein the oscillatory sweeping motion, described by an arrow 60, is about the z-axis. It will be appreciated that other arrangements are similarly possible.

As seen in Figure 1C, illustrating a cross-sectional view in the x-y plane, preferably, the assemblies 20 are arranged in an arc or an arc-like structure, about the body 100, maintaining a shape that follows the body contours, so as to keep as close as possible to the body 100.

Figure 1D provides details of the detecting unit 12. The collimator has a length L, a collection angle $\delta$, and a septa thickness $\tau$. The single pixel detector is preferably a square of sides D and a detector thickness $\tau d$.

Preferred dimensions for the detecting unit 12 may be, for example, 2.46 mm X 2.46 mm, and the solid collection angle $\delta$ may be at least 0.005 steradians. Generally, there may be 16 X 64 detecting units 12 per block 18.

The detector 14 is preferably, a room temperature, solid-state CdZnTe (CZT) detector, which is among the more promising that is currently available. It may be obtained, for example, IMARAD IMAGING SYSTEMS LTD., of Rehovot, ISRAEL, 76124, www.imarad.com, or from eV Products, a division of II-VI Corporation, Saxonburg Pa., 16056, or from or from another source. Alternatively, another solid-state detector such as CdTe, HgI, Si, Ge, or the like, or a combination of a scintillation detector (such as NaI(Tl), LSO, GSO, CsI, CaF, or the like) and a photomultiplier, or another detector as known, may be used, preferably with a photomultiplier tube for each single-pixel detector 14 and collimator 16, for accurate spatial resolution.

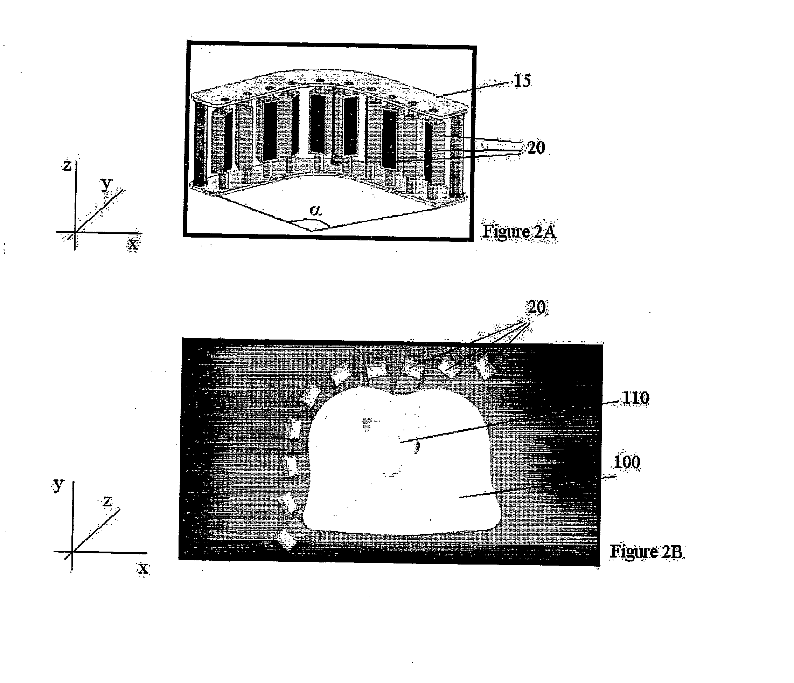

Reference is further made to Figures 2A and 2B that schematically illustrate the structure 15 with the assemblies 20, in accordance with an implementation. As seen, the assemblies 20 are arranged in an arc of an angle $\alpha$, around the body 100, and move in the assembly oscillatory sweeping motion, about the z-axis, so as to provide a plurality of views of the heart 110, from many positions, along the x-y plane.

As seen in Figures 2A and 2B, the dynamic camera 10 is configured for simultaneous acquisition by the assemblies 20, each scanning the same region of interest from a different viewing position, thus achieving both shorter acquisition time and better edge definitions.

Preferably, the structure 15 conforms to the contours of the body 100, to maintain substantial contact or near contact with the body.

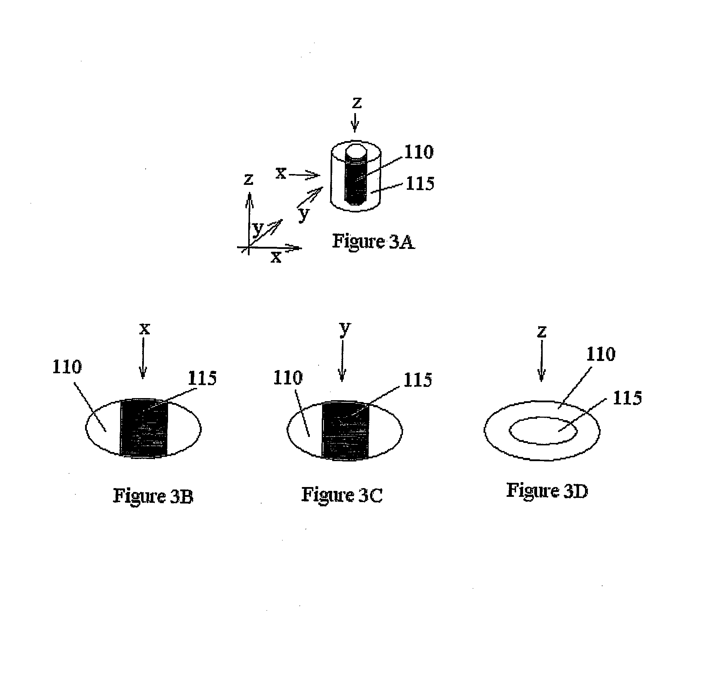

The embodiment of figures 2A and 2B illustrates a single type of motion - assembly oscillatory sweeping motion about the z-axis, as described by the arrow 60 (Figure 1A). In some cases, additional motions or views from additional directions may be desirous, as illustrated in Figures 3A - 3D, hereinbelow.

Reference is further made to Figures 3A - 3D, which schematically illustrate viewing positions, in accordance with some implementations.

Figure 3A illustrates a cylindrical target organ 110, with a cylindrical radioactive emission source 115 therein.

As seen in Figure 3B, a view along the x-axis will observe the cylindrical radioactive emission source 115 as a bar 115.

As seen in Figure 3C, a view along the y-axis will similarly observe the cylindrical radioactive emission source 115 as a bar 115, thus not adding new information to the view along the x-axis.

It will be appreciated that in the present example, any view along the x-y plane will observe the radioactive emission source 115 as a bar 115.

As seen in Figure 3D, a view along the z-axis will observe the cylindrical radioactive emission source 115 as a circle 115, adding new information to the views along the x and y axes.

As Figures 3A - 3D illustrate, at times, views along two axes may be insufficient for a three-dimensional definition of an object, and it may be beneficial to include views with a component along the third axis. For the sake of definition, views along two axes will be referred to stereo views, while views that include a component of the third axis will be referred to as cross views, since they intersect the planer stereo views.

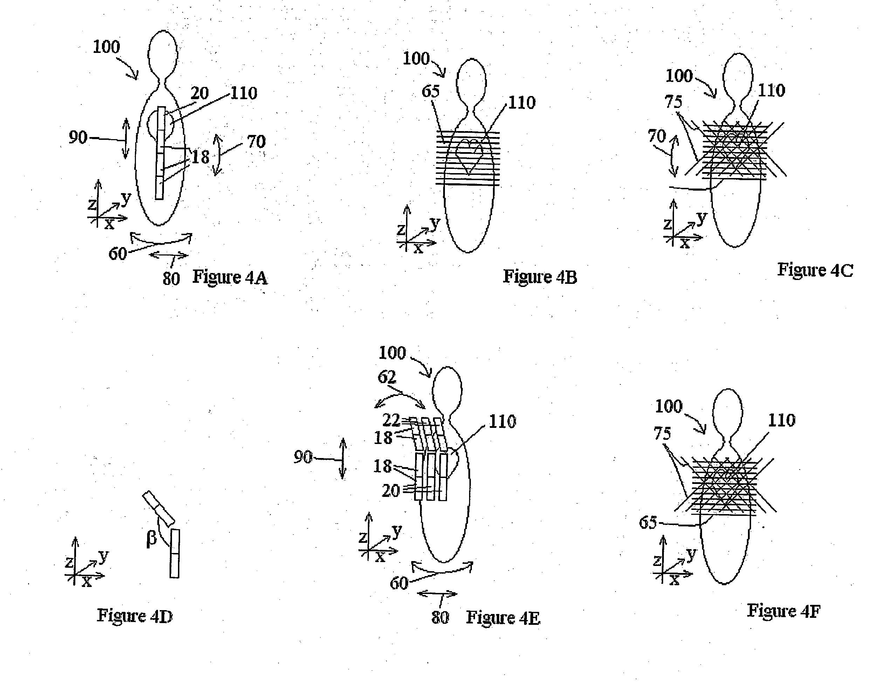

Reference is further made to Figures 4A - 4F, which schematically illustrate stereo views and cross views, in accordance with some implementations.

Figure 4A illustrate the body 100 with a single assembly 20 arranged for viewing, for example, the heart 110. The assembly 20 is afforded with assembly oscillatory sweeping motion along the z-axis, as described by the arrow 60, and preferably first and second preferably orthogonal oscillatory lateral motions, described the arrows 80 and 90, respectively.

As seen in Figures 4B, the assembly oscillatory sweeping motion along the z-axis, described by the arrow 60, produces views 65 in the x-y planes. The first and second orthogonal oscillatory lateral motions, described the arrows 80 and 90, augment these with additional views 65 in the x-y planes. The purpose of the first and second oscillatory lateral motions is to compensate for "dead areas," that is, structural areas and other areas that do not participate in the detection, within the assembly 20 and between the assemblies 20, so as to provide complete coverage of the body 100, by the array 25 (Figure 1A). These motions produce views substantially in the x-y plane. It will be appreciated that there is a component of viewing in a third axis, due to the solid collection angle of the collimator 16. Yet this component is rather small.

Returning to Figure 4A, the blocks 18 of the assembly 20 may be further afforded with block oscillatory sweeping motion, described by the arrow 70 and preferably orthogonal to the assembly oscillatory sweeping motion described by the arrow 60.

As seen in Figure 4C, the block oscillatory sweeping motion, described by the arrow 70, produces cross views 75, which supplement views 65, by providing components of the third axis, namely, the z-axis. As illustrated in Figures 3A - 3D, hereinabove, the views 75 may add additional information, not available or barely available in the views 65 along the x-y planes.

Figures 4D and 4F illustrate an alternative mode for acquiring the cross views 75. Accordingly, the dynamic camera 10 further includes assemblies 22, arranged at an angle β to the assemblies 20, and moving with an assembly oscillatory sweeping motion, described by an arrow 62, so as to provide the cross views 75.

It should be noted that the detectors of the dynamic camera 10 do not have to be arranged in arrays. In one implementation, the detectors are scattered in front of the body so as to provide complete coverage of the body internal overall volume. The detectors can be scattered in a certain structure or in an arbitrary order.

The Position Tracker 50

The position tracker 50 is configured for providing information on the position and orientation of each detecting unit 12, with respect to the overall structure 15, substantially at all times, during the individual assembly motion.

In one implementation, the position tracker 50 relates to software and (or) hardware that receive information from the motion provider 40 and calculate the position and orientation of each detecting unit 12, based on that information. Preferably, the calculation is performed within the control unit 55.

Alternatively, position sensors, as known, may be used for determining the position and angular orientation of each detecting unit 12.

Alternatively still, a combination of information from the motion provider 40 and position sensors may be employed.

The Timing Mechanism 30

The timing mechanism 30 associates timing information with the radioactive emission data impinging the single-pixel detectors 14 of the detecting units 12. Preferably, the timing mechanism 30 includes a single clock used for all of the single-pixel detectors 14 in the dynamic SPECT camera 10, so that timing information is synchronized for the camera as a whole. The timing information is collected at the single-pixel level, so that time binning may be performed for the emission data collected by each pixel. Exemplary methods for associating timing information with the radioactive emission data include:

1) Time stamping - Each event, impinging on a given single-pixel detector 14 at a given time is stamped with a time of detection and a pixel identification. Stamping may be performed by any manner known in the art, for example as a data packet header or footer. The time-stamped, pixel stamped radioactive emission data may be binned, per time and per pixel, by the control unit 55.

2) Time binning - In an alternate approach, timing information is provided for a cumulative count collected from each single-pixel detector 14 over a fixed time interval, for example, 0.001 seconds, 1 second, or 10 seconds, rather than for individual events. Each time bin is then stamped with a time stamp or sequential number and pixel identification. One technique for performing time binning is to insert a periodic clock pulse into the data stream. The interval between the clock pulses equals the minimum bin length. Thus, periodic pulses every 0.001 seconds may lead to bin lengths of 0.001 seconds or greater, for example, 1 second, or 10 seconds.

The timing Mechanism 30 is used by the reconstruction module in order to allow the separate reconstruction of the dynamic and static volumetric regions. The timing Mechanism 30 allows the reconstruction module to apply a time binning with a certain length on the dynamic volumetric region and a time binning with another length on the static volumetric region.

Time Scale Considerations

Dynamic studies, aimed at obtaining kinetic parameters, require the acquisition of full-reconstructed images at a rate that is no greater than about half the frequency of the sampled kinetic parameter. For example, for adult humans, blood circulates through the body at a rate of about 1 cycle per minute. Thus, sampling a process affected by blood circulation should take place at a rate of at least two samplings per minute. Preferably, sampling should be at a much greater rate, for example, 6 samplings or 10 samplings per minute - that is, about every 10 seconds or about every 6 seconds.

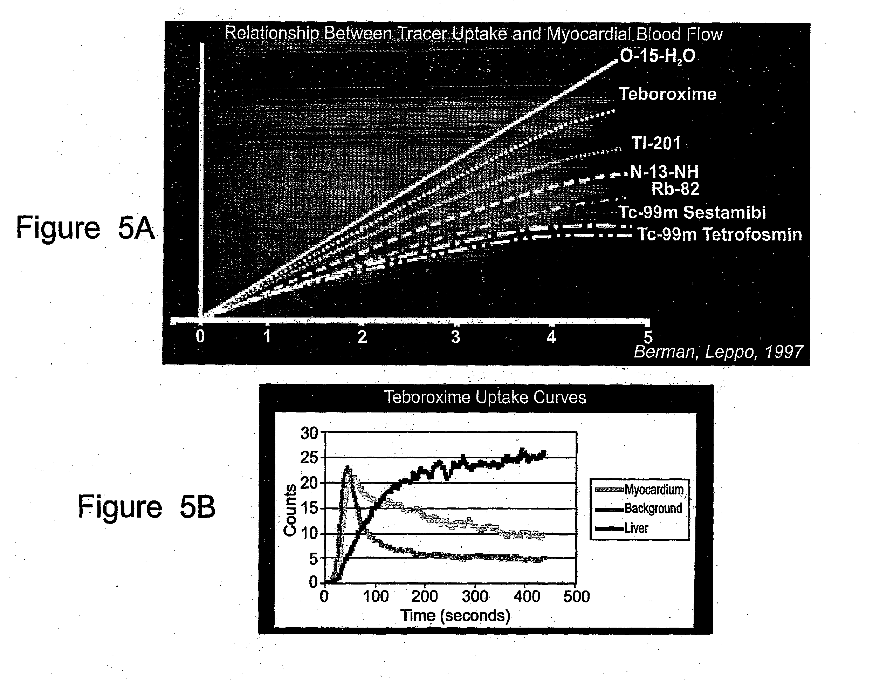

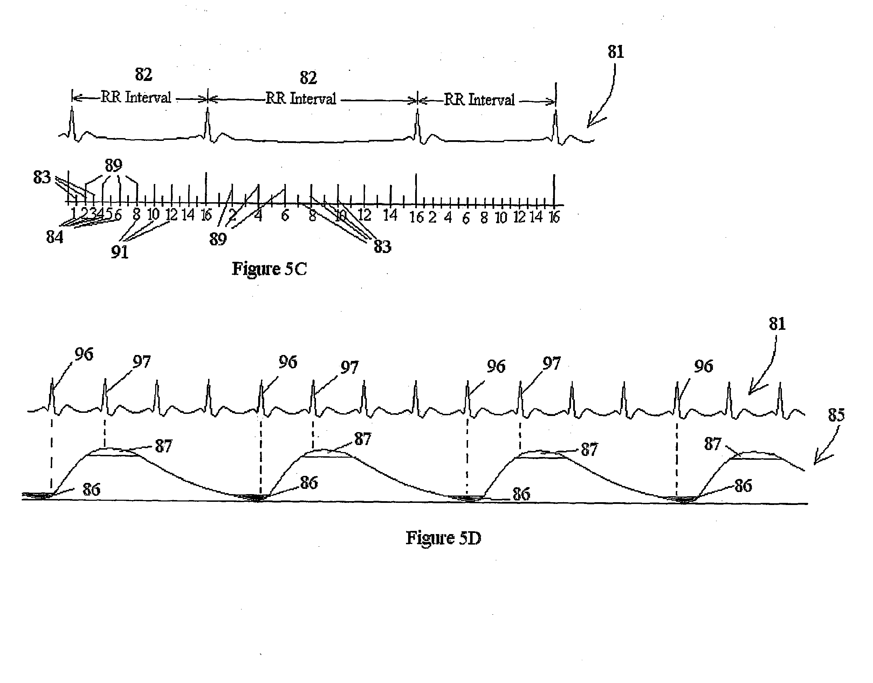

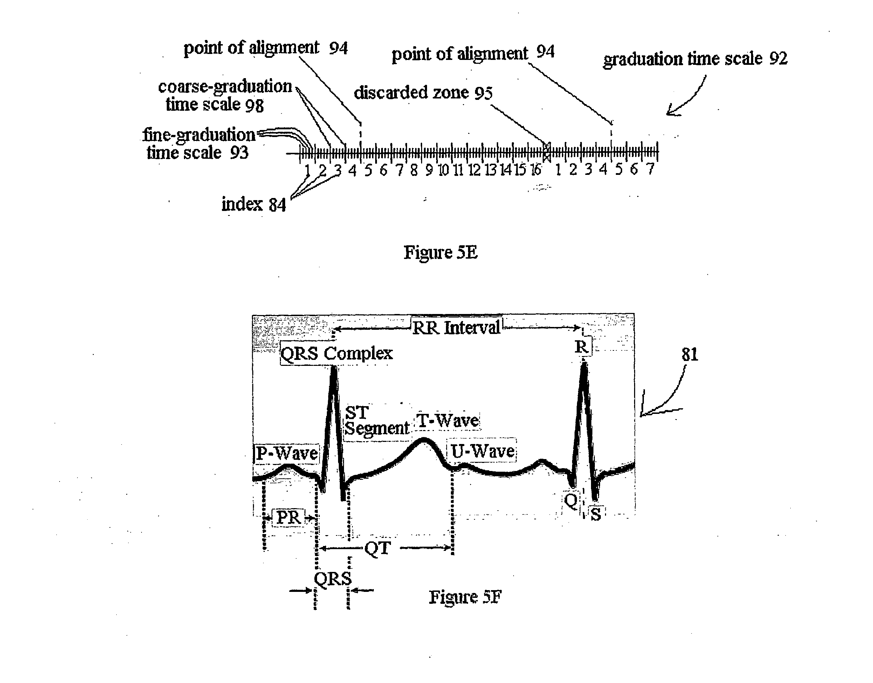

Additionally, based on Figures 5A and 5B, according to Garcia et al. (Am. J. Cardiol. 51st Annual Scientific Session, 2002), showing physiological behavior of different radiopharmaceuticals, dynamic studies for Tc-99m teboroxime are best performed within about the first 100 seconds after administration, and better still, within the first 60 seconds after administration.

Moreover, based on Figures 5A and 5B, the dynamic behavior of a radiopharmaceutical in the body, varies as a function of time, depending on the radiopharmaceutical and on the time elapsed since its administration. For example, myocardial perfusion of Tc-99m teboroxime shows a very steep uptake between about the first 10 - 15 seconds and the first 50 - 60 seconds, followed by a more gradual washout, after the first 60 seconds. The rate of sampling of Tc-99m teboroxime, during the first 60 seconds after administration should be adjusted to the very steep uptake, for example, a sampling rate of every second. For radiopharmaceutical with a slower dynamic behavior, a slower rate may be sufficient.

It will be appreciated that a dynamic analysis requires precise knowledge of the time of administration.

Obtaining the Time of Administration of a Radiopharmaceutical

As noted hereinabove, precise knowledge of the time of administration of a radiopharmaceutical is important both in order to evaluate physiological processes made visible by the radiopharmaceutical, with respect to the time of the radiopharmaceutical's entry to the body and in order to perform the evaluation at an optimal period, with respect to the radiopharmaceutical's cycle in the body.

There are several methods for acquiring precise knowledge of the time of administration of the radiopharmaceutical, as follows:

1. providing communication means between an administration device, for example, a syringe or an IV device, and the dynamic SPECT camera 10, and communicating the precise time of administration, vis-a-vis a clock, by the administration device to the dynamic SPECT camera 10. This method may be employed for example, when administration takes place when the patient is positioned at the dynamic SPECT camera 10, for imaging.

2. providing communication means between the administration device, the dynamic SPECT camera 10, and a third unit, for example, a control system or a hospitals ERP system, communicating the precise time of administration, vis a vis a clock, by the administration device to the third unit, and reporting the precise time of administration by the third unit to the dynamic SPECT camera 10. This method may be employed for example, when administration takes place at a different location than the imaging station.

3. allowing the dynamic SPECT camera 10 to image the site of administration, for example, the arm of the patient, while administration takes place, while employing the timing mechanism 30 of the dynamic SPECT camera 10. A marker, for example, a line of radioactive ink may drawn, for example, on the patient's arm or on the administration device, for defining the time of administration as the time the radiopharmaceutical first crosses the marker. Alternatively, observing a flow of the radiopharmaceutical in the administration device or in the patient's vein may be used to determine the time of administration.

4. Observing a transparent administration device, for example, with a video camera, associated with a clock, may be employed for defining a time of administration based on the radiopharmaceutical distribution in the administration device, or based on the time the radiopharmaceutical first crosses a marker, visible by the video camera. Communication between the video camera and the dynamic SPECT camera 10, or between the video camera, the dynamic SPECT camera 10, and a third unit will provide the information to the dynamic SPECT camera 10.

In some implementations, the administration may include various administration profiles, for example, bolus, continuous drip, or sinusoidal.

Spatial and Temporal Resolution

In order to meet the time scale considerations, described hereinabove, the dynamic SPECT camera 10, according to some implementations, is designed at least for acquiring a tomographic reconstruction image of about 15 X 15 X 15 cubic centimeters, which is approximately the volumetric region of a heart, at a predetermined spatial resolution of at least 10 X 10 X 10 cubic millimeters, at an acquisition time no greater than about 30 seconds. Preferably, the acquisition time is no greater than about 10 seconds, and more preferably, the acquisition time is no greater than about 1 second.

Additionally, the spatial resolution of the tomographic reconstruction image may be at least 7 X 7 X 7 cubic millimeters, or better yet, at least 4 X 4 X 4 cubic millimeters, or better still, at least 1 X 1 X 1 cubic millimeters.

Anatomically Varying Time-Bin Lengths

As discussed in the background section, the time binning is needed in order to generate a clear imaging of a dynamic organ, such as the heart, or a section thereof. Though the time binning allows the acquisition of a clear image of the heart, it has at least one major disadvantage. The reconstruction of the image using time binning requires high computational throughput. Thus, binning images of the input overall volume may provide a clear imaging of the heart however have high computational throughput. Reconstruction using anatomically varying time-bin lengths can be used to reduce to computational throughput of the time binning.

While some body organs, such as the kidney, the lung, or the liver, are relatively static, so as to enable imaging of a period of time that allows acquiring a statistically significant number of counts, the heart moves relatively rapidly, with about 80 - 100 beats per minute, on the average. In one implementation, as the static region does not have to be gated to provide a clear imaging, only the dynamic region that preferably contains the heart is gated. In such an embodiment, fewer voxels are gated and therefore the computational complexity is reduced.

In such an embodiment, different areas in the body can be gated in a rate that is adjusted to according to a respective level of activeness. For example, the heart that has high level of activeness can be gated using a large number of bins, the visceral background, which is relativity static, is gated using one of two bins, and the stomach, that have higher level of activeness than the visceral background but lower level of activeness than the heart is gated using a limited number of bins.

As described below, performing gated image reconstruction using anatomically varying time-bin lengths improves the reconstruction quality, reduces the reconstruction time, or both. The improvement is an outcome of a reduction in the needed computational resources.

Reference is now made to Figures Y and Z, which are respectively a schematic isometric view of the input overall volume 1001 segmented into dynamic volumetric region and static volumetric regions 1003, 1002, as depicted in Figure 14, and a schematic cross-sectional view of the segmented input overall volume 1001A taken along the lines III-III, according to one implementation.

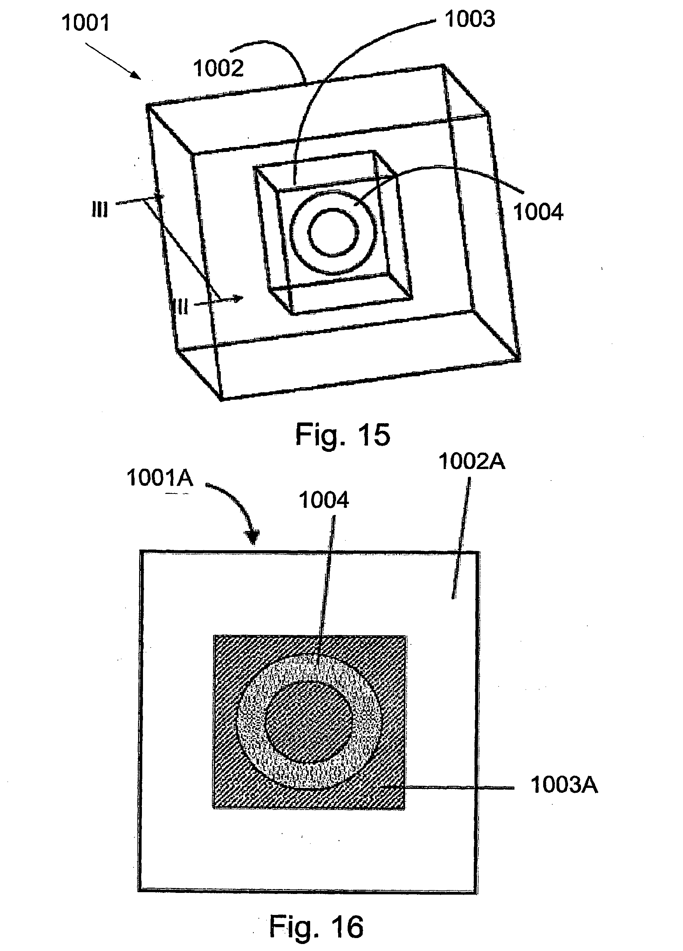

As described below a radioactive emission image of the input overall volume 1001 is segmented into a non-gated region, which includes non-gated voxels, in accordance with the static volumetric region 1002, and to a gated region, which includes gated voxels, in accordance with the dynamic volumetric region 1003. Preferably, the dynamic volumetric region 1003 is adjusted to delimit a dynamic organ, such as the human heart that is schematically represented by a hollow sphere 1004. Preferably, the dynamic volumetric region 1003 is larger than the apparent volumetric region of the heart 1004 to account for segmentation errors. It should be noted that the dynamic volumetric region 1003 may be adjusted to contain other human and animal internal organs such as the stomach. In Figure 16, the hatched region 1003A represents the cross-section of the dynamic volumetric region 1003 and the annular crosshatched region 1004A schematically represents a cross-section through the heart muscle of the heart 1004. The region 1002A represents a cross section of the static volumetric region 1002.

It should be noted that the cubical shape of the dynamic volumetric region 1003 and the static volumetric region 1002 are not obligatory and the segmentation to regions may be performed using differently shaped volumetric regions. Preferably, the dynamic and the static volumetric regions 1003, 1002 have several non-connected parts. For example, the dynamic volumetric region 1003 may be a spherical volumetric region, an ellipsoid of revolution, an ellipsoid with a hole that represents the blood inside the heart, a cylindrical volumetric region or any other type of suitable regularly shaped or non-regularly shaped volumetric region. Preferably, the dynamic volumetric region 1003 comprises non-connected components, which may be referred to as sub-volumetric regions.

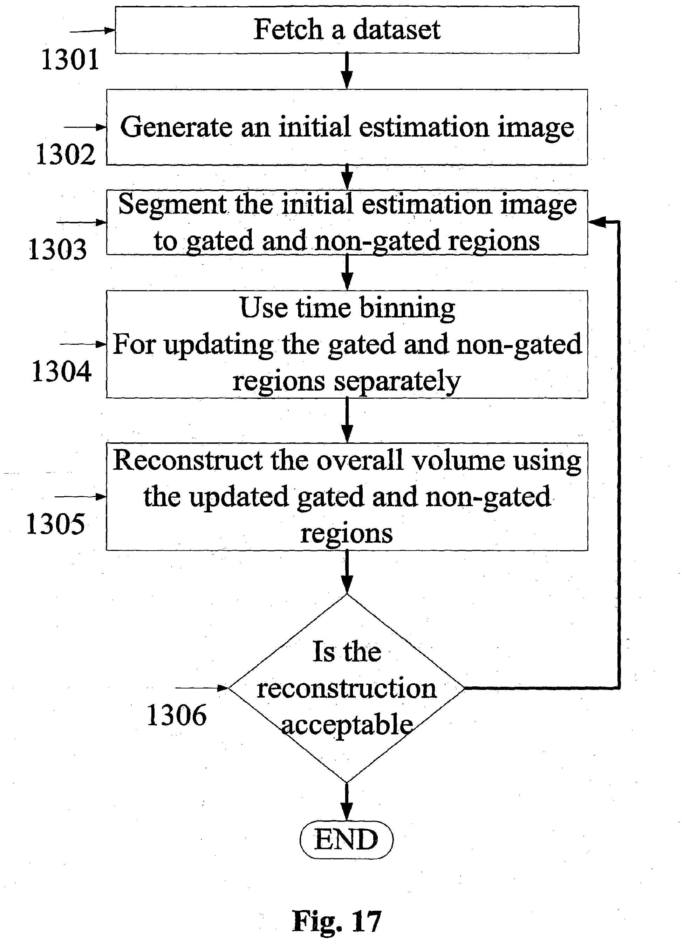

Reference is now made jointly to Figure 15, previously described, and to Figure 17, which is a flowchart that depicts a method for reconstruction an input overall volume using anatomically varying time-bin lengths, according to one implementation.

During the first step, as shown at 1301, radiation emitted from the input overall volume 1001 is captured by the SPECT detectors and recorded, as described above. The captured radiation is used to generate a set of gated images, which are used to overcome distortions such as motion artifacts. As described above, each gated image is generated by a photon counting that takes into account the portion of the heart contraction cycle within which a photon is measured. The number of photons hitting the detector within a specific integration time is calculated and used as raw data, which may be referred to as datasets.

Then, as shown at step 1302, the captured datasets are firstly used in an initial reconstruction process in which an initial estimation image is generated. Preferably, a non-gated reconstruction is used to provide a reconstruction that estimates the static intensity distribution.

In the following step, as shown at step 1303, the initial estimation image is segmented to a gated region and a non-gated region that respectively define the boundaries of the dynamic and static volumetric regions.

The segmentation of the input overall volume 1001 to dynamic and static volumetric regions 1003, 1002 is performed using a suitable image segmentation method or process.

Preferably, the input overall volume 1001 is further segmented to one or more other segments such as the liver. Such segments may be joined to the dynamic or to the static volumetric regions 1003, 1002 according to the nature of the activity level of the segment. For instance, the liver may be joined to the static volumetric region.

The segmentation to static and dynamic volumetric regions may be performed using a number of possible methods. In one embodiment, a system user marks the boundaries of the dynamic volumetric region that comprises the gated voxels. In such an embodiment, the reconstructed image is displayed on a screen of a user interface that allows the system user to delimit the dynamic volumetric region. Though the captured image is blurry, as it is not gated, it provides the system user a perceptual image of the outlines of the internal organs in the input overall volume, including the heart, the liver, the spleen, the kidneys, and the aorta. In such an embodiment, the system user segments the captured image to gated and non-gated regions according to their level of activity, thereby defines the gated and non-gated regions. Preferably, the system user segments the heart as a non-gated region.

In one implementation, the segmentation is based on a voxel value threshold that reflects a certain percentage of the maximal reconstruction value. In such an embodiment, voxels of the reconstructed image having a value above the threshold are presumed to be voxels that depicts the heart and tagged as gated voxels of the dynamic volumetric region and voxels of the input overall volume 1001 having a value below the threshold are tagged as non-gated voxels of the static volumetric region. Preferably, regions in the captured image are segmented according to predefined characteristics. For example, the liver region, which can be characterized as a very large segment residing in the lower part of an image that depicts the thorax, is identified and segmented as a static volumetric region 1003 or a section thereof.

Preferably, the predefined threshold is defined according to the radiation intensity of the visceral background of the input overall volume 1001. In such an embodiment, the radiation intensity of the visceral background is estimated before the segmentation process. Such an initial estimation can be performed using median or linear filters such as Gaussian and moving average filters. Each one of the voxels of the input overall volume 1001 with a value that is well above the estimated background radiation is tagged as a gated voxel. Each one of the voxels of the input overall volume 1001 with a value, which is below the estimated background radiation, is tagged as a non-gated voxel.

In one implementation, the segmentation is performed according to morphological segmentation methods that adjusted according to the volumetric characteristics of the segmented volumetric regions. For example, for the heart that has convex faces can be segmented using top hat transform.

In one implementation, the segmentation is performed according to the growing rate of regions of the input overall volume 1001. In such a manner, regions such as the heart may be indented. In one embodiment, voxels having high growing rate are clustered as a group of voxels that depicts the heart.

In one implementation, the faces of the heart are identified. Such identification may be performed using an objective function with two parts. One part of the objective function is dependent on the organ border smoothness alone and the other part is dependent on the edge strength near a defined border, see M. Kass, A. Witkin, and D. Terzopoulos. Snakes: active contour models, International Conference on Computer Vision, pages 259-268, 1987.

For clarity, $Z_{dyn}(u)$ and $Z_{stat}(u)$ respectively denotes the dynamic volumetric region and the static volumetric region of the captured image. The dynamic and static volumetric regions respectively define the boundaries of gated and non-gated regions in the radioactive emission image that depicts the input overall volume. It should be noted that though only two volumetric regions are exemplified hereinbelow, the overall volume may be segmented according to any number of volumetric regions such as three volumetric region, four volumetric region, ten volumetric region etc.

Preferably, after the gated and non-gated regions of the radioactive emission image have been segmented according to the dynamic and static volumetric regions, different resolutions are used for gated and non-gated voxels. In such an embodiment, the computational load of the reconstruction may be reduced by using large voxels with a low resolution in the static volumetric region 1002 and small voxels in the gated volumetric region.

Preferably, various morphological methods, such as, dilation, closing and the like are used after the initial segmentation to expand the dynamic volumetric region 1003. The broadening of the dynamic volumetric region 1003 is done in order ensure that if the segmentation has been made according to an organ in a contracted state, the dynamic volumetric region 1003 still encompasses the organ in an expanded state.

After the static and dynamic volumetric regions have been segmented during the initial reconstruction process, time binning of the dynamic volumetric region of the input overall volume is performed and a separate reconstruction of the static and the dynamic volumetric regions is enabled. As shown at 1304 - 1306, the reconstruction is based on an iterative process in which the time binning of gated images of the dynamic volumetric region is enabled.

For clarity, $I_0(u)$ denotes an input image I, which is preferably constant, that depicts $u \in U$ voxels, $t$ denotes a certain detector, $g$ denotes a certain gate in a set of G gates, such as 8, 16, and 24, $\phi_t(u)$ denotes a standard functional matrix that depicts the detection probability of a photon emitted from location $u \in U$ to be detected by detector $t$, $s_t$ denotes the sensitivity of the detector $t$, $T_t^g$ denotes the integration time of detector t for gate $g$, $I^g(u)$ denotes a set of $G$ gated reconstructed images, $y_t^g$ denotes the number of photons that are emitted from voxel u and detected in detector t at gate g.

$T_t$ denotes the integration time of detector t and calculated as follows:

$$T_t = \sum_g T_t^g$$

$I_{stat}(u)$ and $I_{dyn}^g(u)$ respectively denote static and dynamic region images, wherein $I_{stat}(u)$ and $I_{dyn}^g(u)$ are mutually exclusive as $I_{stat}(u) \cdot I_{dyn}^g(u) = 0, \; \forall u$.

$Z_{dyn}(u)$ and $Z_{stat}(u)$ respectively denote static and dynamic regions in I, as defined in the aforementioned segmentation process. Preferably, $Z_{dyn}(u)$ and $Z_{stat}(u)$ are defined as follows:

$$Z_{dyn}(u) = \begin{cases} 1, & u \in \text{dynamic region} \\ 0, & \text{otherwise} \end{cases}$$

$$Z_{stat}(u) = 1 - Z_{dyn}(u)$$

Preferably, before the input overall volume $I$ is iteratively reconstructed, few preliminary sub-steps are taken. During the first sub-step, $I_{stat}(u)$ and $I_{dyn}^{g}(u)$ are initialized as follows:

$$I_{stat}(u) = I_0(u) \cdot (1 - Z_{dyn}(u))$$

$$I_{dyn}^{g}(u) = I_0(u) \cdot Z_{dyn}$$

Preferably, if $Z_{dyn}(u) = 0$, the size of $I_{dyn}^{g}(u)$ is reduced.

As described above, $Z_{dyn}(u)$ and $Z_{stat}(u)$, which respectively confine the static and dynamic volumetric regions, are defined at step 1302.

During the second sub-step the scale is calculated as follows:

$$scale_{O.S.}^{g}(u) = \sum_{t \in O.S.} s_t \cdot T_t^{g} \cdot \phi_t(u)$$

$$scale_{O.S.}(u) = \sum_{g} scale_{O.S.}^{g}(u)$$

After the preliminary steps have been completed, the reconstruction of the input overall volume according to time binning process commences. Preferably, during the reconstruction $I_{stat}(u)$ and $I_{dyn}^{g}(u)$ are calculated for each voxel $u \in U$ in the input overall volume.

During each iteration of the time binning process, as shown at 1305, the gated and non-gated regions that represent the static and dynamic images $I_{stat}(u)$, $I_{dyn}^{g}(u)$ are updated. The updating of the regions is calculated according to a deviation between the number of photons that has been detected by the SPECT detectors and an estimation of this number, as described below. During each one of the iterations, the gated voxels of the dynamic volumetric region are binned according to the number of gates and the non-gated voxels are binned only once. As the non-gated voxels are binned only once, the computational complexity of the process is relatively low. The separation between the static and dynamic volumetric regions improves the computational efficiency and reduces the statistical variance.

In particular, in order to calculate $I_{stat}(u)$ and $I_{dyn}^{g}(u)$, a number of sub-iterations take places. First, $\hat{y}_{stat,t}$ is calculated as follows:

$$\hat{y}_{stat,t} = s_t T_t \sum_u \phi_t(u) I_{stat}(u)$$

Where $\hat{y}_{stat,t}$ denotes an estimation of the number of photons that are emitted from the voxels $u \in U$ and detected by detector t, wherein values of voxels from the dynamic volumetric region are zeroed. It should be noted that the sensitivity parameter of and the integration time of detector t are taken into account at some stage in the calculation.

Then, $\hat{y}_{dyn,t}^{g}$ is calculated as follows:

$$\hat{y}_{dyn,t}^{g} = s_t T_t^{g} \sum_u \phi_t(u) I_{dyn}^{g}(u)$$

Where $\hat{y}_{dyn,t}^{g}$ denotes an estimation of the number of photons that are emitted at gate g from voxels $u \in U$ and detected in detector t, wherein values of voxels from the static volumetric region are zeroed. It should be noted that the sensitivity parameter and the integration time of detector t for gate g are taken into account at some stage in the calculation. $\hat{y}_t^{g}$ is calculated according to $\hat{y}_{dyn,t}^{g}$ and $\hat{y}_{stat,t}$, as follows:

$$\hat{y}_t^g = \hat{y}_{stat,t} + \hat{y}_{dyn,t}^g$$

Where $\hat{y}_t^g$ denotes an estimation of the number of photons that are emitted from a certain voxel $u \in U$ and detected by detector $t$ at gate g. It should be noted that unlike the calculation of $y_t^g$, the calculation of $\hat{y}_t^g$ does not take into account the integration time and the sensitivity factor.

Then, for each gate g, the numerator $num^g(u)$ is evaluated as follows:

$$num^g(u) = \sum_t \frac{y_t^g}{\hat{y}_t^g}\left(s_t T_t^g \phi_t(u) - 1\right)$$

Where $num^g(u)$ sums the deviation between the number of photons that are emitted from voxel u and detected in detector t at gate g and the estimation thereof of all the detectors, wherein the sensitivity and the integration time of each detector $t$ are taken into account. It should be noted that the calculation can be directly extended to an ordered sets method or any of its variations by summing the deviation over subsets of the group of detectors.

Based thereupon, the numerator $num(u)$ is evaluated as follows:

$$num(u) = \sum_g num^g(u)$$

Where $num(u)$ is a sum of all the numerators that are evaluated for every g∈G.

$I_{stat}(u)$ and $I_{dyn}^g(u)$ are updated according to the calculation of the aforementioned scales and numerators, as follows:

$$I_{stat}(u) = I_{stat}(u) + \frac{num(u)}{scale(u)} \cdot I_{stat}(u)$$

$$I_{dyn}^g(u) = I_{dyn}^g(u) + \frac{num^g(u)}{scale^g(u)} \cdot I_{dyn}^g(u)$$

The updated $I_{stat}(u)$ and $I_{dyn}^g(u)$ are stored and used during the next iteration, as shown at step 1306. Steps 1303 - 1306 are repeated iteratively until the reconstruction of the input overall volume has reached a desired quality.

Preferably, in order to determine whether the reconstruction has reached a desired quality, as shown at 1306, the number of gated voxels with activity above a predefined threshold is checked. For example, the number of gated voxels with activity level that is in the range between the maximal gated voxel intensity value and 20% therefrom is checked.

When the time binning process has been completed, as shown at 1306, a gated reconstructed image can be generated as follows:

$$I^g(u) = I_{stat}(u) + I_{dyn}^g(u)$$

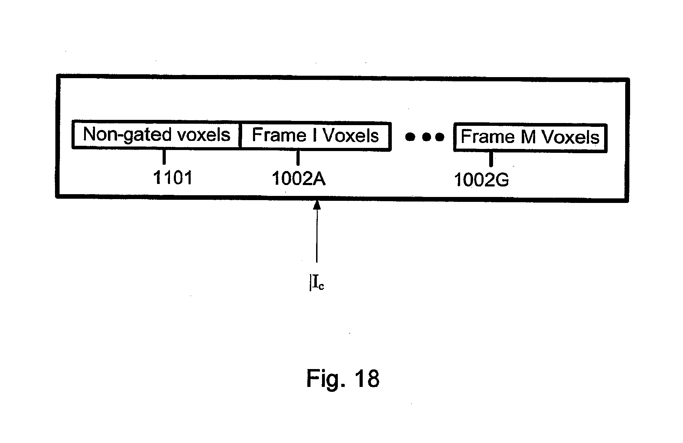

Reference is now made to Figure 18, which is a graphical representation of one dimensional vector $I_c$ of voxels that represents the reconstruction of the input overall volume. Preferably, all the non-gated voxels $I_{stat}(u)$ that represent static regions of the input overall volume U are arranged 1101 first within the vector $I_c$. The non-gated voxels are followed 1101 by gated voxels that comprise a set of different frames in a consecutive order that are arranged in clusters. Each cluster represents the dynamic volumetric region of the input overall volume at a certain frame. The frames are denoted by 1102A,...,1102G. The frames can be arranged in any predefined order.

As described above, $\phi_t(u)$ is a standard functional matrix that depicts the detection probability of a photon emitted from a voxel u ∈ U to be detected by a detector t. Since $\phi_t(u)$ is a sparse matrix, the number of math operations can be reduced by defining $\phi_t^g(u)$ which is zero wherever $I_{dyn}^g(u)$ is zero.

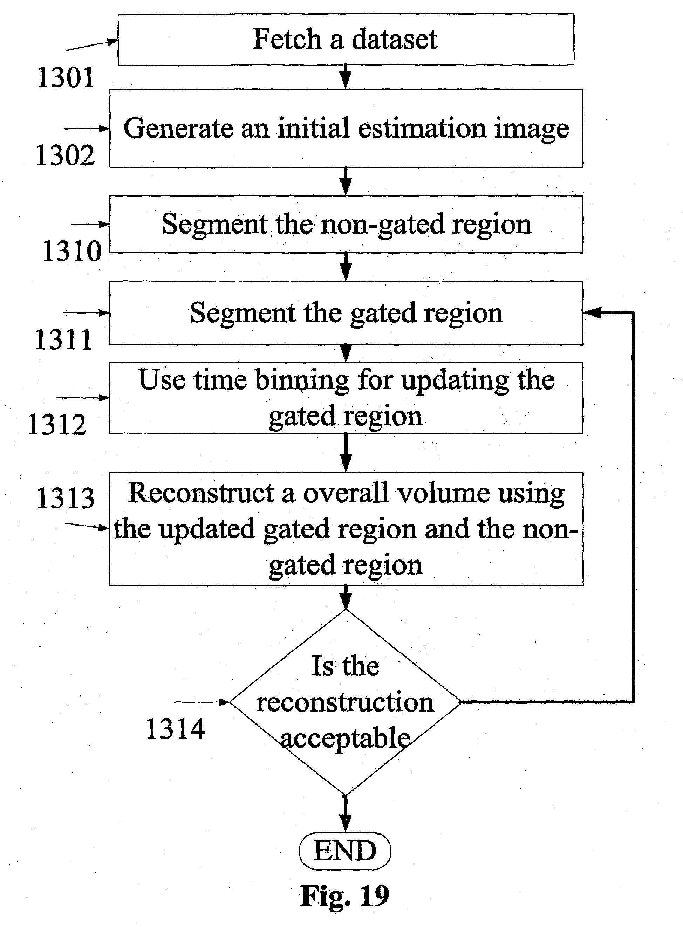

Reference is now made jointly to Figure 15, previously described, and to Figure 19, which is a flowchart that depicts another method for reconstruction an input overall volume using anatomically varying time-bin lengths, according to another implementation. In the method depicted in Figure 19, the static region is estimated only once according to the initial reconstruction process step.

The method depicted in Figure 19 is based on the assumption that the non-gated static region equals to the average of the gated dynamic region images reconstructions. Though the assumption is not accurate, it is expected to be sufficient for the reconstruction of the input overall volume. As the static region is calculated only once, the memory usage and the computational complexity decrease.

Steps 1301 and 1302 are as depicted in Figure 17. During steps 1301 and 1302 the first step I(u) is obtained. Then, as shown at 1310 and 1311, I(u) is segmented to current and static regions, preferably according to the following equations:

$$I_{stat}(u) = I(u) \cdot (1 - Z_{dyn}(u))$$

$$I_{dyn}^g(u) = I(u) \cdot Z_{dyn}(u),$$

for each g ∈ G

In order to reduce the computational complexity of the following iterative process, $\hat{y}_{stat,t}$ is evaluated in advance as follows:

$$\hat{y}_{stat,t} = s_t T_t \sum_u \phi_t(u) I_{stat}(u)$$

The previously described method uses the standard functional matrix $\phi_t(u)$ that is a representation of the probability to detect a photon emitted from location $u \in U$ by a detector $t$. The calculation of $\phi_t(u)$ requires high computational complexity as all the voxels of the input overall volume have to be calculated. In order to reduce the computational complexity a standard functional matrix that is limited to dynamic voxels is used $\phi_{t,dyn}(u)$. The limited standard functional matrix is defined as follows:

$$\phi_{t,dyn}(u) = \begin{cases} \phi_t(u), & u \in \text{dynamic region} \\ 0, & \text{otherwise} \end{cases}$$

Then, for each g ∈ G, the scale on the dynamic region $scale^g(u)$ is evaluated, as described above.

During the following step, as shown at 1312, the dynamic volumetric region $I_{dyn}^g(u)$ is calculated. In particular, in order to calculate $I_{dyn}^g(u)$, a number of sub-iterations take place. First, $I_{dyn}^g(u)$ is calculated using $\phi_{t,dyn}(u)$ as follows:

$$\hat{y}_{dyn,t}^g = s_t T_t^g \sum_u \phi_t(u) I_{dyn}^g(u)$$

Then based on $\hat{y}_{dyn,t}^g$ and $\hat{y}_{stat,t}$ that has been calculated in the step 1310, $\hat{y}_t^g$ is calculated as follows:

$$\hat{y}_t^g = \hat{y}_{stat,t} + \hat{y}_{dyn,t}^g$$

Where $\hat{y}_t^g$ denotes an estimation of the number of photons that are emitted from a certain voxel $u \in U$ and detected by detector t at gate g. It should be noted that unlike $\hat{y}_{dyn,t}^g$, $\hat{y}_{stat,t}$ is not recalculated during the iterative process.

Then, for each gate g, the numerator $num^g(u)$ is evaluated as follows:

$$num^g(u) = \sum_t \frac{y_t^g}{\hat{y}_t^g}\left(s_t T_t^g \phi_t(u) - 1\right)$$

Based on the calculation of the scale that has been calculated before the iterative process and the numerator that is calculated according to radiation emitted from the dynamic region and captured by the detectors, $I_{dyn}^g(u)$ is updated as follows:

$$I_{dyn}^g(u) = I_{dyn}^g(u) + \frac{num^g(u)}{scale^g(u)} \cdot I_{dyn}^g(u)$$

The updated $I_{dyn}^g(u)$ is stored and used during the next iteration, as shown at step 1316.

Then, as shown at 1313, the input overall volume is reconstructed using the updated dynamic region and the static region. As shown at 1314, steps 1311 - 1314 are repeated iteratively until the reconstruction of the input overall volume has reached a desired quality. In the end of each on of the iterations, the dynamic region is updated, as described above.

When the iterative process has been completed, as shown at 1314, a gated reconstructed image can be generated as follows:

$$I^g(u) = I_{stat}(u) + I_{dyn}^g(u)$$

The gated voxels in the dynamic volumetric region 1003 may represent ischemic regions of the heart. The radiation reflected from such ischemic regions may have specific radiation patterns such as a center with low radiation. Thus, such regions can be reconstructed using morphological closing methods or by taking into account the typical shape of the heart (one way is by fitting an ellipsoid to the edges in the image, but other methods may also be used).

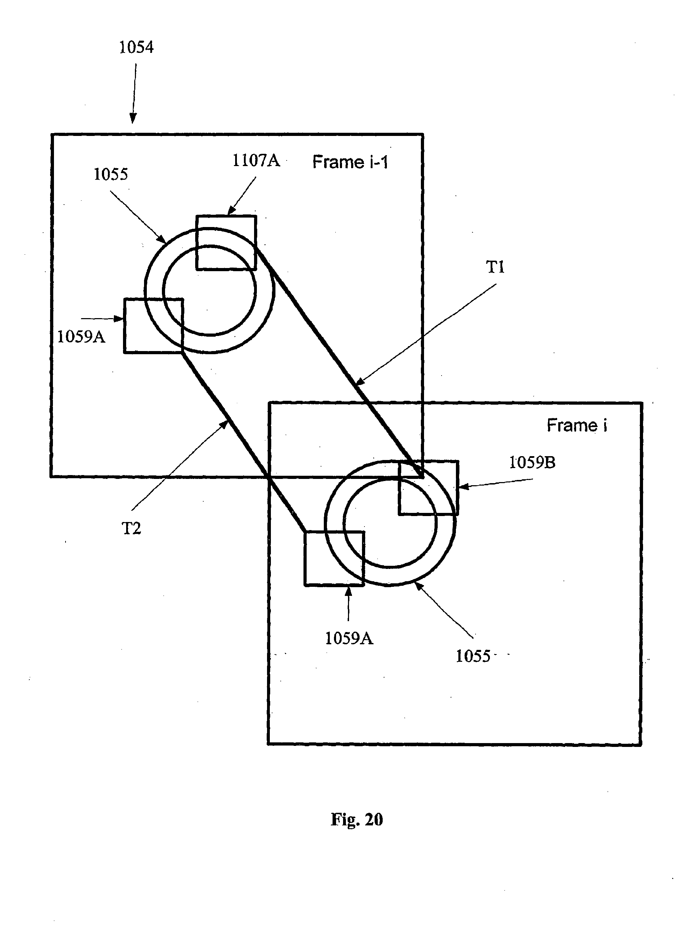

Reference is now made to Figure 20, which is a graphical representation of a position of two selected sub-regions in two sequential frames. As described above, the reconstruction of the dynamic volumetric region is based on time binning of a number of consecutive frames that depict a dynamic organ such as the heart. As each frame is based on a number of gated images, it has high computational load.

In one implementation, the set of frames is a set of sequential images that depict the heart. As all the frames depict the same input overall volume and as the heart has an expected movement pattern, we can use one or more frames to estimate another. In such a manner, fewer frames are calculated and therefore the computational complexity of the reconstruction decreases. Preferably, during the time binning, geometrical information from one or more prior frames assist in the reconstruction of subsequent one or more frames. Such geometric,prior methods are generally well known and therefore, are not described here in greater detail.

For example, Figure 20 depicts two sequential frames, frame i-1 1054 and frame i 1056, which are taken from a sequence of M frames. The sub-regions 1057A and 1059A in frame 54 schematically represent two different regions of the heart 1055. The regions 1057B and 1059B of frame 1056 schematically represent the respective positions of regions 1057A and 1059A in frame 1056. The change in the positions is an outcome of the movement of the heart 1055. The vector T1 represents the movement of the region 1057B relative to the region 1057A and the vector T2 represents the movement of the region 1059B relative to the region 1059A. T1 and T2 can be used to estimate the position of additional one or more frames in some of the implementations. See, Green P, Bayesian Reconstructions from Emission Tomography Data using a Modified EM Algorithm, IEEE Tran. On medical imaging vol.9 No.1, March 1990, pp.84-93 and Fessler J, 2004 NSS/MIC statistical image reconstruction short course notes entitled" Statistical Methods For Image Reconstruction", www.eecs.umich.edu/-fessler/papers/files/talkIO4/mic.notes.Pdf. Preferably, voxels that represent the same anatomical location are stated to be alike. Therefore, voxels from different frames that represent the same anatomical location can form a clique, or a neighborhood, as defined by the Gibbs prior equation. The strength of applying such a geometric prior may be depended on the movement amplitude and on the gate phase.

Dynamically Varying Time-Bin Lengths

There are times when dynamically varying time-bin lengths are desired. For example, Tc-99m-teboroxime has an uptake curve (Figure 5B) which is very steep during the uptake and which becomes less so during the washout. Thus, different time-bin lengths may be desired for different portions of the Tc-99m-teboroxime uptake curve. Similarly, different radiopharmaceuticals have different uptake curves, and dedicated time-bin lengths may be desired for each radiopharmaceutical, and for different portions of their respective uptake curves. Moreover, the cardiac RR cycle has very steep periods, during the rise and fall of the R peak (Figure 5F), followed by periods that are nearly flat as a function of time. Again, time bin lengths of different durations may be employed for the different portions of the RR cycle. Furthermore, while the actual region of interest, for example, the heart, requires imaging at a very high level of accuracy, adjacent regions, for example, the chest muscle, may be of lesser interest, and may be viewed at time bins of greater lengths. Additionally, continuous acquisition mode may require shorter time-bin lengths than stop and shoot mode.

For example, the actual rise and fall of the R peak may be gated at time bins of 10 milliseconds, while the nearly leveled U-wave may be gated at 100 milliseconds. Similarly, while the heart muscle may be gated at an average time bin of 50 milliseconds, the adjacent chest muscle may be gated at time bins of 1 second and longer. It will be appreciated that other values may similarly be employed.

In some implementations, a lookup system of recommended time-bin lengths may be provided, for specifying recommended time-bin lengths as functions of one or more of the following:

a specific region of interest;
an administered radiopharmaceutical;
time elapsed since the administration of the radiopharmaceutical;
cardiac state with respect to an RR cycle;
a view of the detecting unit 12, with respect to the region of interest;
patient general data; and
data acquisition mode.

The lookup system may be, for example, tables or curves.

Thus the dynamic SPECT camera 10 may be configured for time binning at dynamically varying time-bin lengths, by providing communication between the timing mechanism 30 and the lookup system, wherein the timing mechanism is configured for selecting a recommended time-bin length from the lookup system, for each time bin.

Clearly, if the input image has been segmented to gated and non-gated regions, as described in above, only the gated region is gated at time bins at dynamically varying time-bin lengths.

Dynamically Varying Spectral Bins

It is sometimes of value to image only a specific spectral bin so as to eliminate scatter or contributions from other radiopharmaceuticals. Additionally, it may be of value to image several spectral bins simultaneously, for different radiopharmaceuticals, wherein different groups of detecting units are dedicated to different spectral bins.

Thus, the dynamic SPECT camera 10 may be configured for dynamically determining a spectral energy bin for each detecting unit 12, as follows:

providing a spectral selection mechanism 56 (Figure 1A), for enabling a selection of a spectral energy bin to be used for each detecting unit 12, independently from the other detecting units 12; and
a lookup system of recommended spectral energy bin values, as functions of at least one of a specific region of interest, an administered radiopharmaceutical, time since the administration of the radiopharmaceutical, a view of the detecting unit with respect to the region of interest, and patient's details;
wherein the spectral selection mechanism 56 is further configured for dynamically determining the spectral energy bin for each detecting unit, as functions of the specific region of interest, the administered radiopharmaceutical, the time elapsed since the administration of the radiopharmaceutical, the view of the detecting unit with respect to the region of interest, and patients' details, from the lookup system.

The spectral energy bin is designed to include a primary photon energy $\pm 10\%$, or the primary photon energy $\pm 7\%$, or the primary photon energy $\pm 5\%$.

Additionally, at least two radiopharmaceuticals may be administered and viewed by different groups of detecting units, each group being configured for a different spectral energy bin, so as to view each radiopharmaceutical in the same region independently of the other radiopharmaceutical.

The spectral selection mechanism may be a hardware unit or software.

The spectral selection may be performed during data acquisition, or later.

Intracorporeal dynamic SPECT Camera

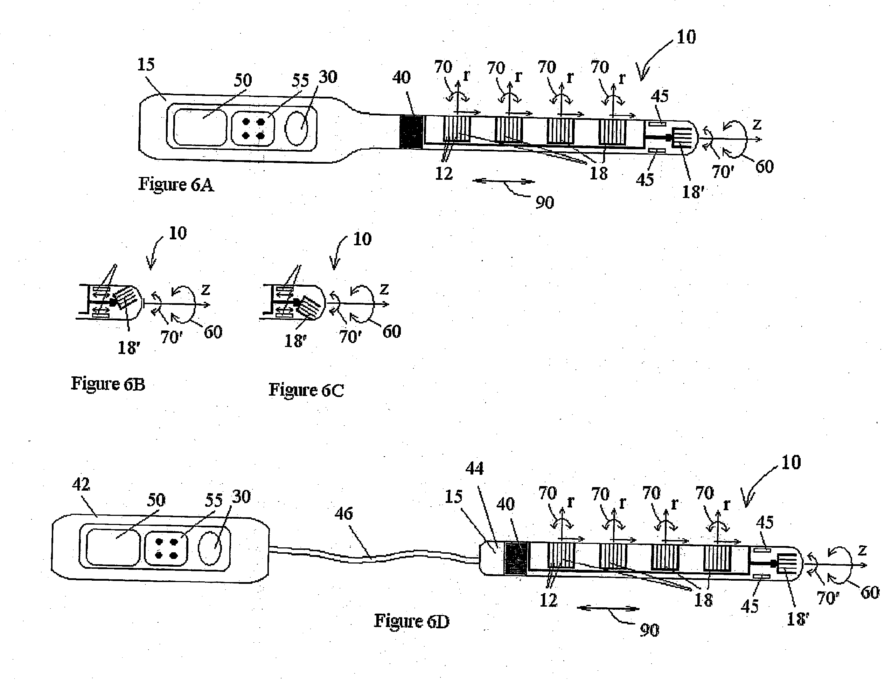

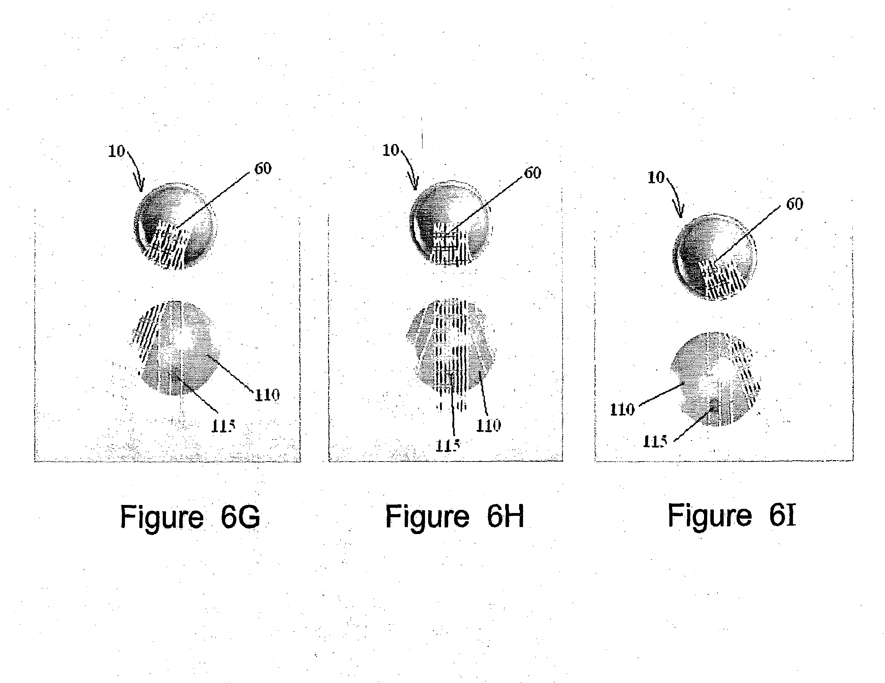

Referring further to the drawings, Figures 6A - 6I describe the dynamic SPECT camera 10 as an intracorporeal dynamic SPECT camera 10, which includes a single assembly 20, preferably configured for oscillatory sweeping motion around its longitudinal axis - the z axis, as described by the arrow 60. The blocks 18 may be further configured for oscillatory sweeping motion in an orthogonal direction, as described by the arrows 70. An end block 18' may be further configured for motion, for example, as described by the arrow 70'. It will be appreciated that other motions are similarly possible, for example, oscillatory lateral motions, or rotational motions. For example, the arrow 90 describes the oscillatory lateral motion along the z axis of the assembly 20.

An ultrasound transducer 45 may be included with the intracorporeal dynamic SPECT camera 10.

Other features of the intracorporeal dynamic SPECT camera 10 are as described for the dynamic SPECT camera 10 of Figures 1A - 1D.

Figure 6A illustrates the intracorporeal dynamic SPECT camera 10 as a single rigid unit, for example, for rectal or vaginal insertion. Figure 6C illustrates the intracorporeal dynamic SPECT camera 10 as having an incorporeal portion 44, an extracorporeal portion 42 and a cable 46, for example, for insertion to the esophagus.

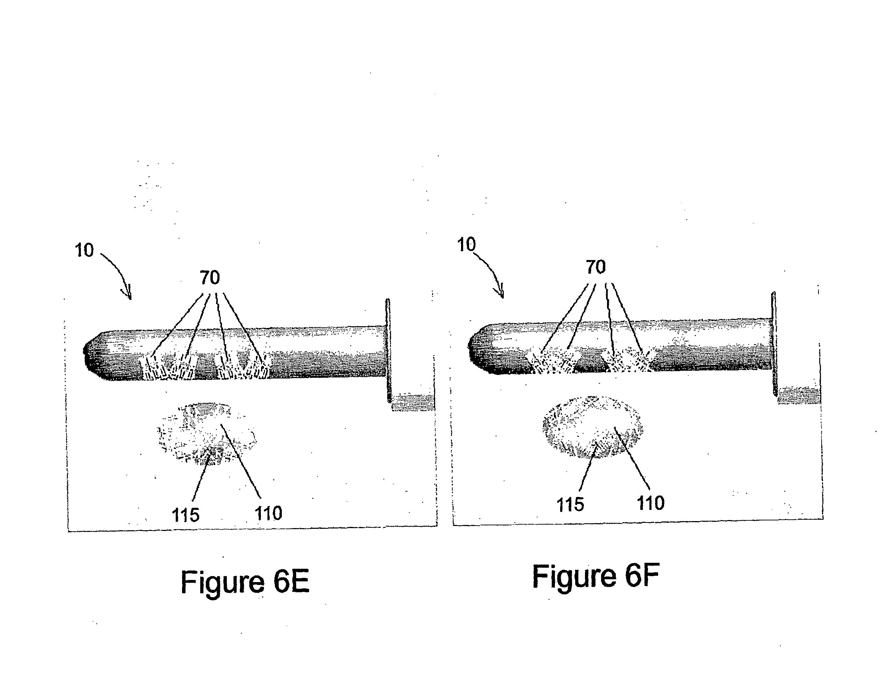

Figures 6F and 6E illustrate motions of the blocks 18, as described by the arrows 70. Figures 6F - 6I illustrate motion of the assembly 20, as described by the arrow 60.

Reconstruction with Object Implantaion

As described above, the reconstruction of the radioactive emission image is based on datasets that have been acquired from a certain overall volume, such as the thorax, with objects having known volume and structure, such as the heart. As the reconstructed volumetric region has a known structure and comprises organs with estimated structure, relative location, and volume, the throughput of the reconstruction can be reduced.

The reconstruction process is an iterative process. During each step, the reconstruction of the overall volume and one or more volumetric regions thereof are being refined. Preferably, one or more object models, which are defined according to an image, such as a CT or an MRI image, an anatomical atlas, or other accurate reconstructions of respective objects, are used to improve and enhance the reconstruction process.

Object implantation proceeds as follows: after a few iterations, which provide a general idea of both:

i. the location and general shape of an organ in question, such as a heart, lungs, a stomach, visceral background elements, etc.; and

ii. an estimation of the expected number of photons that are emitted from different portions of the organ in question,

the general shape and photon counts of the organ in question are replaced by an implanted model, based on a CT image, an MRI image, an anatomical atlas, or the like, thereby providing both:

i. a better definition of edges between the organ in question and the surroundings; and

ii. some analytical evaluation of the expected number of photons that are emitted from different portions of the organ in question, based on the first few iterations, for example, given that the organ is a heart, an average count values for the blood and for the heart muscle, respectively, may be used, based on first few iterations, for the different areas of the model. It will be appreciated that an anatomical construction of voxels may be employed with the voxel implantation.

In this manner, object implantation improves the reconstruction that is based on counting statistics.

It will be appreciated that object implantation may be employed once or several times during the reconstruction process, each time, providing a better starting point for the next iteration.

Object implantation comes to solve the problem that during the first steps of the reconstruction, a blurry radioactive emission image of the overall volume is received, as described above. An organ, such as the heart, can be identified in the blurry radioactive emission image according to a cluster of voxels with expected values in an expected relative position. The value of voxels in such a cluster can be adjusted or changed according to a respective object model. For example, if after a certain number of iterations a cluster of voxels in the upper right section of the overall volume has voxels with a certain average expected value, the cluster can be identified as the heart. As it is known that the number of photons, which are emitted from voxels of the heart is relatively high, the value of voxels in the related cluster are adjusted or changed to have relatively high values, according to the object model. In such a manner, the actual shape of the heart can be reconstructed more efficiently.

Reconstruction using a Minimal Number of Gray Levels

Strictly speaking, variations in radioactive emission activity between different voxels can be infinite, one voxel showing a photon count of 17,584/for a given time period, and another, a photon count of 18,900/for the given time period. Yet, to a doctor, interested in identifying background muscles, heart muscles, or blood, and further, intested in differentiating between healthy heart muscle, ischemic muscle, and dead tissue, a few levels of gray, for example, between 5 or 10 levels or gradations of gray, may be sufficient. Thus, reconstruction need not be carried out in order to evaluate an accurate photon count per voxel, but merely to determine the level of gray, from amongst 5 - 10 levels of gray, per voxel.

As described above, the reconstruction of the radioactive emission image is based on datasets that have been acquired from a certain overall volume. The reconstruction is performed by summing up the photons that are emitted from voxels of the overall volume. The sums of the emitted photons are translated to gray level values, which are used to reconstruct the radioactive emissions image. Preferably, in order to determine whether the reconstruction has reached a desired quality, the number of voxels with activity above a predefined threshold is checked. For example, the number of voxels with activity level that is in the range between the maximal voxel intensity value and 20% therefrom is checked. Other criteria may be determined is order to evaluate whether the reconstruction has reached a desired quality. Threshold values are preferably chosen empirically to yield accurate reconstruction of the overall volume. This analysis applies to gated and ungated regions, alike.

Preferably, the values of the voxels are mapped to a limited number of gray level values, such as 5, 7, 8, 9, or 10. By limiting the number of gray level values a radioactive emissions image, which is more coarsened, is generated and the computational load of the reconstruction process is reduced. Though such a limitation reduces the sharpness and the contrast level of radioactive emission image, the coarsened radioactive emission image still depicts enough information that allows a physician to identify ischemic regions in the overall volume. It should be noted that such a mapping may separately be used on one or more volumetric regions of the overall volume, preferably according to the dynamic characteristics thereof.

Image Acquisition Modes

In some implementations, several image acquisition modes are available, as follows:

In a continuous acquisition mode, also referred to as fanning, data is acquired while the array, the assembly, or the block is in continuous motion. Continuous acquisition mode may apply also to oscillatory motions, although strictly speaking there is a momentary pause with each change of direction. This mode leads to some blurring of the data, but it does not require the array, assembly, or block to stabilize between periods of motion and periods of stationary data acquisition.

In a stop and shoot acquisition mode, incremental travels are followed by stationary acquisition intervals. This mode leads to better resolution, yet it requires a damping period, to allow the array, assembly, or block to stabilize between periods of motion and periods of stationary data acquisition, as discussed hereinbelow, under the heading, "Stability and Damping Time".

Interlacing is a fast stop and shoot acquisition mode with oscillatory motion, for example, sweeping oscillatory motion, wherein on rather than stopping at each predetermined locations, with each sweep, the odd locations are visited on a right sweep and the even locations are visited on the left sweep, or vice vera, so that each sweeping direction stops at different locations.

Prescanning relates to a fast prescan of a subject undergoing diagnosis, to identify a region-of-interest, and thereafter collect higher quality data from the region-of-interest. A prescan, according to one implementation, may be performed by the dynamic SPECT camera 10, preferably, with interlacing, or in a continuous mode, or by any other imaging device, including, for example, ultrasound or MRI.

Stability and Damping Time

Stop and shoot acquisition mode involves discontinuities in motion between travel and shooting modes, and at the beginning of each shooting mode, the assemblies 20 must be allowed to stabilize till vibrations are less than about ±0.25 mm, so as not to interfere with the acquisition.

Prior art SPECT cameras must allow for a damping time of about 5 seconds, but the dynamic SPECT camera 10, according to some implementations, reaches stability in about 1 second or less.

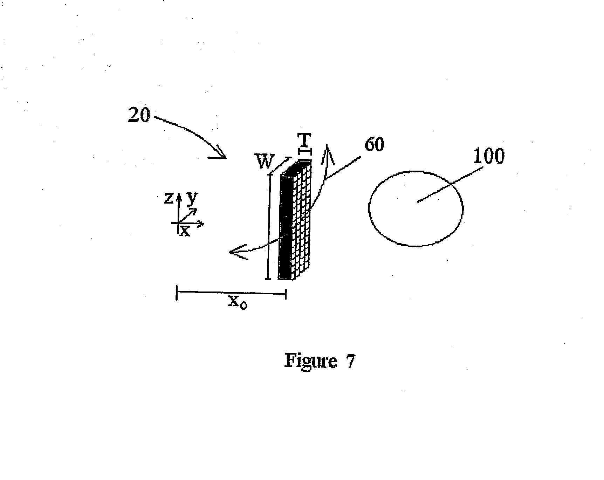

Figure 7 schematically illustrate the assembly 20, according to some implementations. The damping time for the assembly 20 may be described as:

$$Damping\ Time = C\ X\ [\ (1/12)\ M\ (T^2+W^2) + M\ X_0{}^2],$$

wherein:

M is the mass of the assembly 20;
T is the thickness of the assembly 20;
W is the width of the assembly 20;
$X_0$ is the axis of rotation; and
C is a constant that depends on the braking force applied to the assembly 20.

The factor 1/12 is calculated assuming the assembly proximal end is tangential to the sweeping path.

As the damping time equation illustrates, the damping time is highly dependent on both the axis of rotation $X_0$ and the mass of the assembly 20.

In the present case, the axis of rotation is that of the sweeping motion described by the arrow 60 (Figure 1A), which is considerably shorter than an axis of rotation around the body 100.

Similarly, the mass of a single assembly is far less than that of a conventional SPECT camera.

Possible values for the assembly 20, according to some implementations, may be:

Weight of the assembly 20 ≈ 1.5 kg.
Thickness of the assembly 20 ≈ 5 cm.
Width of the assembly 20 ≈ 7 cm.

As such, the assembly is designed with a damping time constant of under 50 msec during which vibrations amplitude subsides to under .25mm.

It will be appreciated that the present example applies to both extracorporeal and intracorporeal dynamic cameras.

Stationary Dynamic SPECT Camera

It may be desired to perform imaging, especially prescanning with a stationary camera, that is without motion, for the following reasons:

1. in continuous acquisition mode, the blurring produced by the motion is eliminated;
2. in stop and shoot acquisition mode, the time spent in motion is avoided, as are the vibrations, associated with the discontinuities between the motions and the stationary intervals.

In general, a stationary camera does not provide sufficient viewing positions and detecting units, yet the camera may be specifically designed to provide those, to a desired level.

Preferably, the assemblies 20 are positioned at optimal positions prior to imaging, and imaging takes place while the camera is stationary.

Thus, in accordance with some implementations, there is provided a stationary dynamic SPECT camera 10, which is described herein with reference to Figures 1A - 1D. The stationary dynamic SPECT camera 10 comprises:

the overall structure 15, which defines proximal and distal ends with respect to a body;
the first plurality of the assemblies 20, arranged on the overall structure 15, forming an array 25 of the assemblies 20, each assembly 20 comprising:

a second plurality of detecting units 12, each detecting unit 12 including:

a single-pixel detector 14, for detecting radioactive emissions; and
a dedicated collimator 16, attached to the single-pixel detector, at the proximal end thereof, for defining a solid collection angle δ for the detecting unit; and

an assembly motion provider 40, configured for providing the assembly 20 with individual assembly motion with respect to the overall structure, prior to the acquisition of radioactive-emission data;
a position-tracker 50, configured for providing information on the position and orientation of each of the detecting units 12, with respect to the overall structure 15, during the individual motion,
the stationary dynamic SPECT camera 10 being configured for acquiring a tomographic reconstruction image of a region of interest while stationary, for the whole duration of the tomographic image acquisition.

Preferably, the region of interest is about 15 X 15 X 15 cubic centimeters, and the tomographic image may be acquired during an acquisition time of 60 seconds, at a spatial resolution of at least 20 X 20 X 20 cubic millimeter.

Additionally, the tomographic image may be acquired during an acquisition time of 30 seconds, at a spatial resolution of at least 20 X 20 X 20 cubic millimeter.

Furthermore, the tomographic image may be acquired during an acquisition time of 60 seconds, at a spatial resolution of at least 10 X 10 X 10 cubic millimeter.

Additionally, the tomographic image may be acquired during an acquisition time of 30 seconds, at a spatial resolution of at least 10 X 10 X 10 cubic millimeter.

Preferably, the structure 15 conforms to the contours of the body 100, for acquisition with substantial contact or near contact with the body.

Additionally, the assemblies 20 in the array 25 are configured to provide stereo views in a plane and cross views.

Anatomic Construction of Voxels

Anatomic construction of voxels avoids the smearing effect of a rigid voxel grid construction, where different tissue types, for example, blood and muscle, appear in a same voxel. This is important especially for perfusion studies, where the perfusion of blood into the tissue is sought.

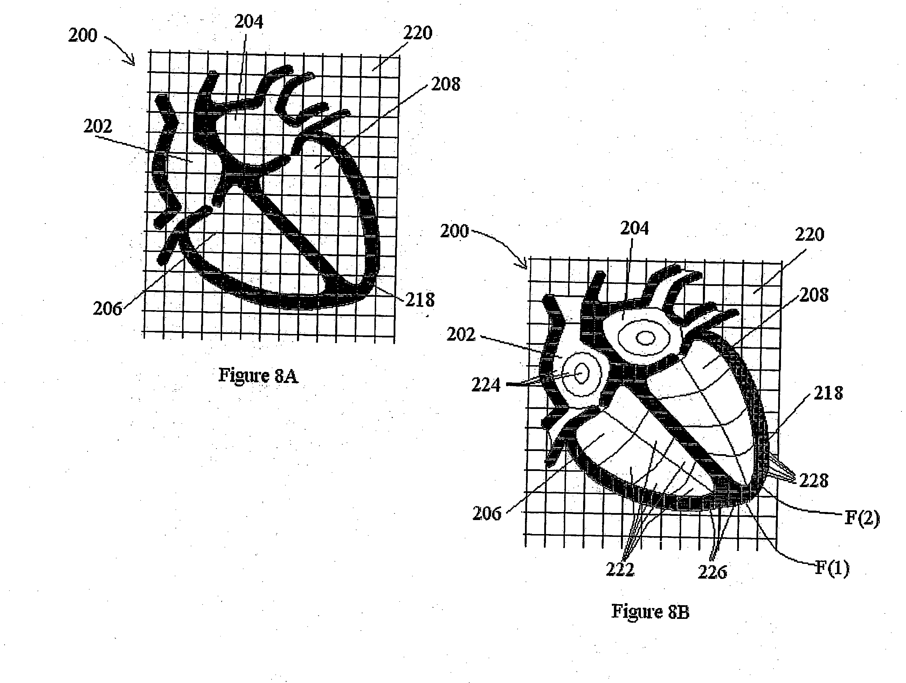

Reference is now made to Figures 8A and 8B, which schematically illustrate a rigid voxel grid construction and an anatomic construction of voxels, respectively, in some implementations.

Figures 8A and 8B illustrate a heart 200, having atria 202 and 204, chambers 206 and 208, and a muscle 218.

As seen in Figure 8A, a rigid voxel construction 202 causes smearing of the different tissue types. However, as seen in Figure 8B, blood and muscle tissues are anatomically divided into different voxels, allowing an accurate study of perfusion. The atria and chambers are divided into an anatomic voxel system 222, or to an anatomic voxel system 224, while the muscle is divided into a relatively coarse voxel system 226, or to a finer voxel system 228, as desired. It will be appreciated that the anatomic voxels may vary in volumetric region. For example, since ischemia is not relevant to the atria and chambers, they may be divided into coarse voxels, while the heart muscle may be divided into fine voxels.

As further seen in Figure 8B, the rigid voxel construction 202 may still applied to the surrounding chest muscle.

It will be appreciated that parametric equations, such as F(1) and F(2) may be created and used in the construction of the anatomic construction of the voxels.

The following describes methods for obtaining the anatomic construction of voxels.

A first method for the anatomic construction of voxels includes:

providing a structural image of a region of interest, such as a heart;
constructing an anatomic system of voxels, for the region of interest, in which voxel boundaries are aligned with boundaries of structural objects of the region of interest, based on the structural image;
performing radioactive-emission imaging of the region of interest, utilizing the anatomic system of voxels; and
performing reconstruction of the radioactive-emission imaging, utilizing the anatomic system of voxels.

Preferably, the structural image is provided by a structural imager, selected from the group consisting of 2-D ultrasound, 3-D ultrasound, planner x-rays, CT x-rays, and MRI.

Additionally, the structural imager is co-registered to a radioactive-emission imaging camera which performs the radioactive-emission imaging.

Moreover, attenuation correction of the radioactive-emission imaging may be performed, based on the structural image.

Furthermore, the structural image and the radioactive-emission image, constructed with the anatomic voxels, may be displayed together.

Alternatively, the structural imager is not co-registered to a radioactive-emission imaging camera which performs the radioactive-emission imaging, and further including corrections for misregistration.

Alternatively still, the structural image is provided from a lookup system, which is preferably corrected for patient's details.

It will be appreciated that the anatomic construction of voxels may be based on fitting the boundaries of the structural objects to parametric equations and utilizing the parametric equations in the constructing of the anatomic system of voxels.

Additionally, the anatomic system of voxels includes voxels of varying volumetric regions, depending on their anatomic position and relevance.

Furthermore, the method includes time binning of the radioactive emissions to time periods not greater than substantially 30 seconds, or not greater than substantially 10 seconds, or not greater than substantially 1 second.

Additionally, the anatomic system of voxels includes voxels of varying volumetric regions, depending on the relevance of their dynamic activity.

An alternative method for the anatomic construction of voxels includes, relates to the use of the radioactive emission imaging itself for the anatomic reconstruction, as follows:

providing a first system of voxels for a region of interest;
obtaining radioactive-emission data from the region of interest;
performing a first reconstruction, based on the radioactive-emission data and the first system of voxels, to obtain a first image;
correcting the first system of voxels, by aligning voxel boundaries with object boundaries, based on the first image; thus obtaining a second system of voxels;
performing a second reconstruction, based on the radioactive-emission data and the second system of voxels, thus obtaining a second image.

Alternatively, a set of radioactive emission data is obtained, possibly with a second injection, in order to concentrate the viewing on the anatomic voxels, as follows:

providing a first system of voxels for a region of interest;
obtaining a first set of radioactive-emission data from the region of interest;
performing a first reconstruction, based on the fist set of the radioactive-emission data and the first system of voxels, to obtain a first image;
correcting the first system of voxels, by aligning voxel boundaries with object boundaries, based on the first image; thus obtaining a second system of voxels, which is anatomically based;
obtaining a second set of radioactive-emission data from the region of interest, based on the second system of voxels, which is anatomically based; and
performing a second reconstruction, based on the second set of the radioactive-emission data and the second system of voxels, thus obtaining a second image.

Anatomic Modeling

Bull's Eye, or polar map, is a semi-automatic method for the quantification and evaluation of coronary artery disease from SPECT tomograms obtained by marking the myocardium with Tl-201 or MIBI-Tc-99. The polar map is computed from cross-sectional slices of the Left Ventricle (LV). For each slice, the center and a radius of search that contains the LV are determined and the LV is divided into radial sectors. The maximum count value of each sector is computed, generating a profile. Profiles are plotted as concentric circle onto the map. The resulting map is a compression of 3D information (LV perfusion) onto a single 2D image.

Yet the bull's eye or polar map is reconstructed from a rigorous geometry of voxels, for example, of 5 X 5 X 5 mm, or 4 X 4 X 4 mm, which cuts across tissue types, thus providing smeared information.

A voxel division that is based on an anatomical structure would be highly preferred, as it would allow the measurements of processes within and across anatomical features, substantially without the smearing effect. For example, if specific voxels are used to define blood regions, and others are used to define muscle regions, than diffusion across boundary membranes and other processes may be evaluated, substantially without a smearing effect.

Anatomical model is based on voxels that follow anatomical structures, and may be shaped for example, as a sphere, a tube, or as a shell segment, rather than as a the standard cube.

When combined with a camera of high resolution and sensitivity and with gated measurements, anatomic modeling would be clearly advantageous over standard, rigorous modeling, especially for kinetic studies are meaningful only with respect to specific tissue types.

In accordance with some implementations, the polar map may be produced with a reduced number of amplitudes, or levels, for example, 7 levels of severity, or 5 levels of severity, from healthy to severe.

**Kinetic Modeling**

As part of the imaging and analysis processes, the camera may be able to produce a time series of 2D or 3D images, showing reconstructed intensity in overall volume and its changes over time.

Likewise, it may be desirable not to reconstruct the entire volumetric region but only limited segments of interest.

In those segments, resolution of segment definition may be very important in order to minimize partial volumetric region effect, which results in a biased estimate of the kinetic process.

In an exemplary embodiment, the analysis of the kinetic process may be after reconstruction of the intensity in the entire volumetric region or in the selected segments has been done for a series of time points. In that case, each segment or location in overall volume (u) has a list of intensity (I) values in time (t), and the list I(u,t) may be further analyzed to fit parametric kinetic model.

Such a parametric kinetic model may be a variety of kinds, depending on the modeling on the biological process. Examples of such models may be found in PCT/IL2005/001173.

In a simplistic example, the model may be

$$I(u,t) = B(t) \bullet \left(1 - e^{-k_1(u)\cdot t}\right) \bullet e^{-k_2(u)\cdot t}$$

where $B(t)$ is a concentration in the blood, whether obtained from imaging a segment which is pure blood (e.g. major blood vessel, or volumetric region within the heart chamber), or may be known from other sources(by injection profile, other invasive or non invasive measurements from the blood, etc). $k_1(u)$ is the time constant representing a process of uptake into the tissue at segment $u$, and $k_2(u)$ is the time constant representing a process of washout from the tissue at segment $u$.

There may be many other models, and for example the equation above may take other forms such as

$$I(u,t) = B(t) * F_1(k_1(u), \tau) * F_2(k_2(u), \tau)$$

where * stands for either multiply operation or convolution in most cases, and $F_1$ and $F_2$ represent processes. In an example, the effect of such process on the intensity may be modeled in linear cases by convolution of the intensity in the blood with an impulse response of a linear process $F_1(k_i(u), \tau)$. Each of these may include one or more time constants $k_i(u)$, and the time profile is described as a function of time $\tau$. There may be one or more such processes $F_i$, for example 1 (e.g. uptake or decay only), 2 (e.g. simultaneous uptake and clearance processes, 3 (e.g. combination with accumulation or metabolism), 4 or more.

A process of fitting may be used between the reconstructed intensity in overall volume and time and the parametric models mentioned above.

In another example, the parametric model may be incorporated *into the reconstruction process*. In this case, it is not necessary to perform reconstruction of intensities *I(u,t)* in overall volume and time and then use that information to extract time constants of biological processes $k_i(u)$.

Instead, the imaging equation

$$y_n(t) \sim Poisson\left(\left[\sum_u \varphi_n(u)I(u,t)\right]\right)$$

may be explicitly replaced with the model of the intensities

$$y_n(t) \sim Poisson\left(\left[\sum_u \varphi_n(u)B(t) * F_1(k_1(u), \tau) * F_2(k_2(u), \tau)\right]\right)$$

(where $y_n(t)$ is the number of photon measured from a viewing position $n$ with a probability function of the view $\varphi_n(u)$).

In this case, the reconstruction process (e.g. by Maximum-Likelihood, Expectation maximization, or other equation solving techniques) is used to recover the best fitting values of $k_i(u)$ , instead of recovering *I(u,t)* and then $k_i(u)$.

In some implementations, the use of a camera directly intended to perform dynamic studies, the ability to avoid interim recovery of intensities in 3D-overall volume in various time periods may be a benefit, as the design of the scanning is optimized for the kinetic parameters reconstruction, and not necessarily to image quality in each time point.

Active Vision

In some implementations, the camera may further include active vision which relates to a_method of radioactive-emission measurements of a body structure, comprising:

performing radioactive-emission measurements of the body structure, at a predetermined set of views;
analyzing the radioactive-emission measurements; and
dynamically defining further views for measurements, based on the analyzing.

Active vision may be used, for example, to better define an edge, by changing a view direction, to direct a saturating detecting unit away from a hot spot, to change the duration at a certain location, when a greater number of counts are required, or when sufficient counts have been obtained.

Reconstruction Stabilizer

The method of reconstruction employed by some implementations may further include a method for stabilizing the reconstruction of an imaged volumetric region, comprising:

performing an analysis of the reliability of reconstruction of a radioactive-emission density distribution of said volumetric region from radiation detected over a specified set of views; and
defining modifications to at least one of a reconstruction process and a data collection process to improve said reliability of reconstruction, in accordance with said analysis.

Additionally, the method may include calculating a measure of said reliability of reconstruction, said measure of reliability of reconstruction being for determining a necessity of performing said modifications.
Furthermore, the method may include:

providing a detection probability matrix defining a respective detection probability distribution of said volumetric region for each of said views; calculating the singular values of said detection probability matrix;
identifying singular values as destabilizing singular values.

Additionally, the method may include calculating a condition number of said probability matrix as a measure of said reliability of reconstruction.
It will be appreciated that this approach may result in non-uniform voxels, wherein voxel volumetric region may increase or decrease as necessary to increase the reliability of the reconstruction

View Selection

In some implementations, a method of optimal view selection is further utilized, as follows:

providing said volumetric region to be imaged;
modeling said volumetric region;
providing a collection of views of said model;
providing a scoring function, by which any set of at least one view from said collection is scorable with a score that rates information obtained from said volumetric region by said set;
forming sets of views and scoring them, by said scoring function; and
selecting a set of views from said collection, based on said scoring function for imaging said volumetric region.

Additionally, zooming in onto a suspected pathology may be performed by a two-step view selection, wherein once the suspected pathology is observed, that region of the volumetric region is modeled anew and a new collection of views is obtained specifically for the suspected pathology.

Experimental Results

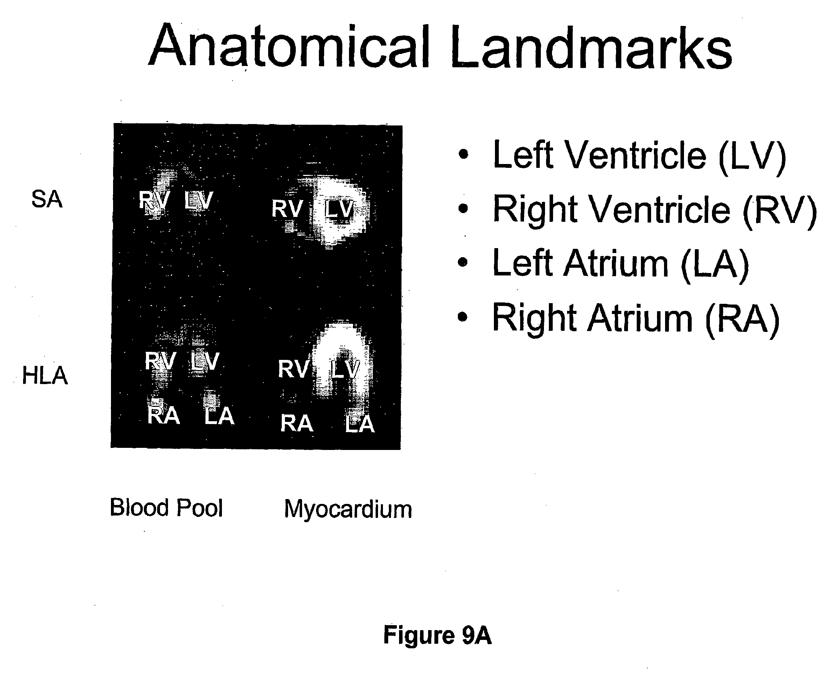

Reference is now made to Figures 9A - 9J, which schematically illustrate cardiac imaging of Tc-99m-Teboroxime, with the dynamic camera 10 in accordance with aspects of some implementations. The significance of the experimental data provided herein is the ability to successfully image Teboroxime, which as Figure 5B illustrates is washed out of the body very quickly.

Figure 9A provides anatomical landmarks, as follows:

- Left Ventricle (LV)
- Right Ventricle (RV)
- Left Atrium (LA)
- Right Atrium (RA)

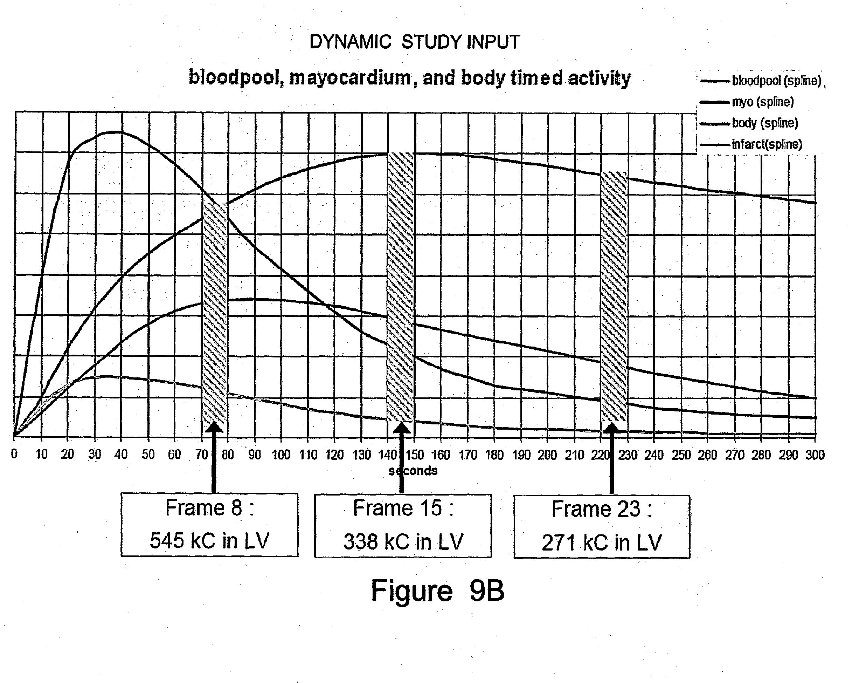

Figure 9B is a dynamic study input of bloodpool, mayocardium, and body timed activity.

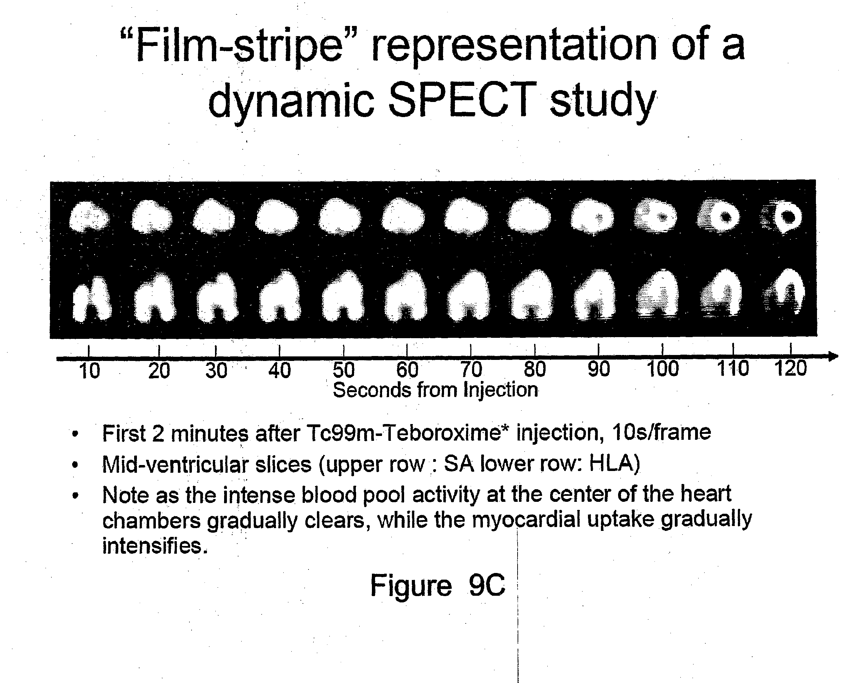

Figure 9C is a Film-stripe representation of a dynamic SPECT study, as follows:

- First 2 minutes after Tc99m-Teboroxime* injection, 10s/frame
- Mid-ventricular slices (upper row : SA lower row: HLA)

Note: as the intense blood pool activity at the center of the heart chambers gradually clears, while the myocardial uptake gradually intensifies.

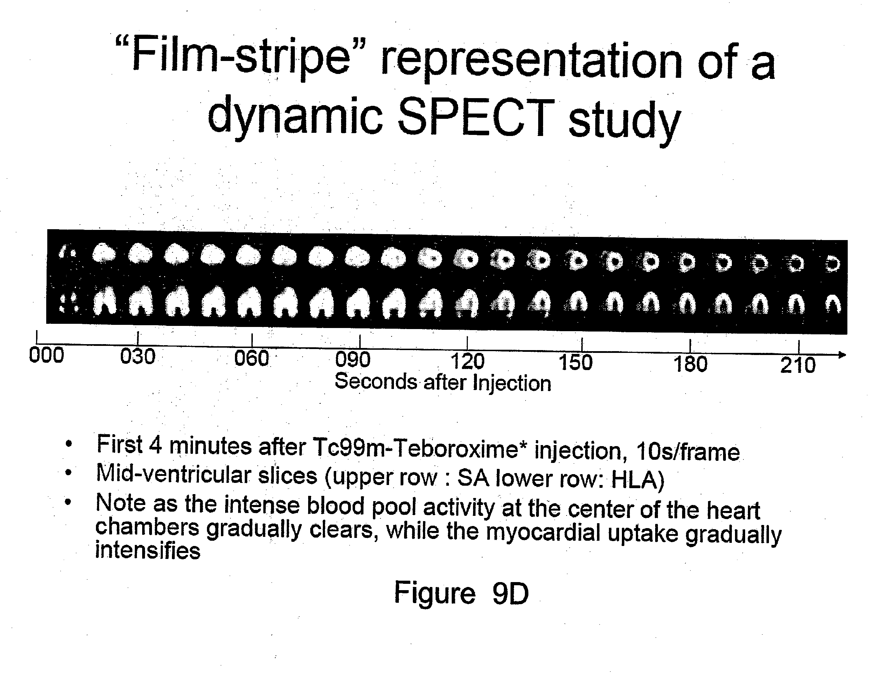

Figure 9D is a Film-stripe representation of a dynamic SPECT study, as follows:

- First 4 minutes after Tc99m-Teboroxime* injection, 10s/frame
- Mid-ventricular slices (upper row : SA lower row: HLA)

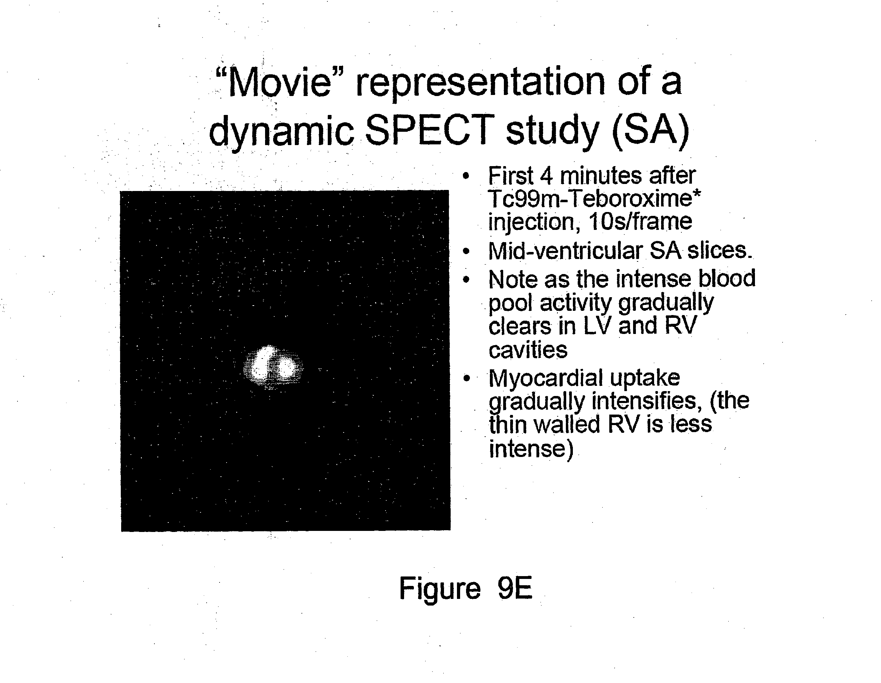

Note: as the intense blood pool activity at the center of the heart chambers gradually clears, while the myocardial uptake gradually intensifies
Figure 9E is a Movie representation of a dynamic SPECT study (SA), as follows:

- First 4 minutes after Tc99m-Teboroxime* injection, 10s/frame
- Mid-ventricular SA slices.

Note: as the intense blood pool activity gradually clears in LV and RV cavities
Note: Myocardial uptake gradually intensifies, (the thin walled RV is less intense)

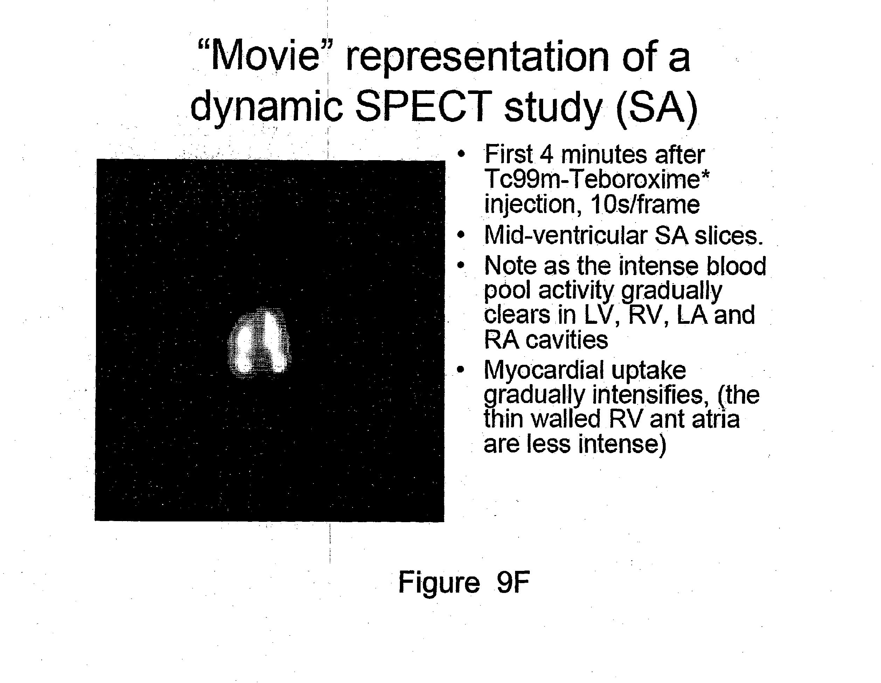

Figure 9F is a Movie representation of a dynamic SPECT study (SA), as follows:

- First 4 minutes after Tc99m-Teboroxime* injection, 10s/frame
- Mid-ventricular SA slices.

Note: as the intense blood pool activity gradually clears in LV, RV, LA and RA cavities
Note: Myocardial uptake gradually intensifies, (the thin walled RV ant atria are less intense)

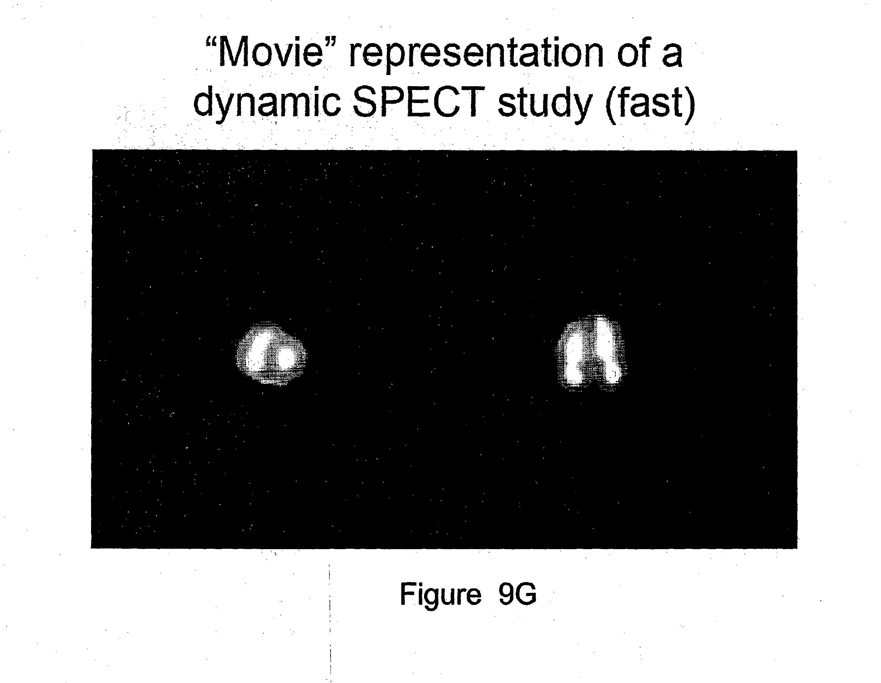

Figure 9G is a Movie representation of a dynamic SPECT study (fast).

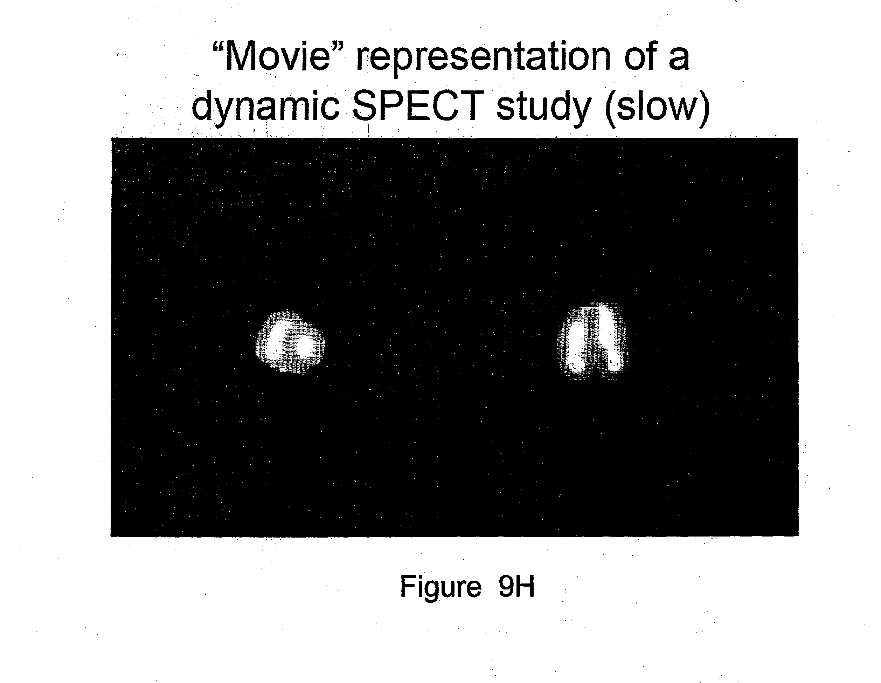

Figure 9H is a Movie representation of a dynamic SPECT study (slow).

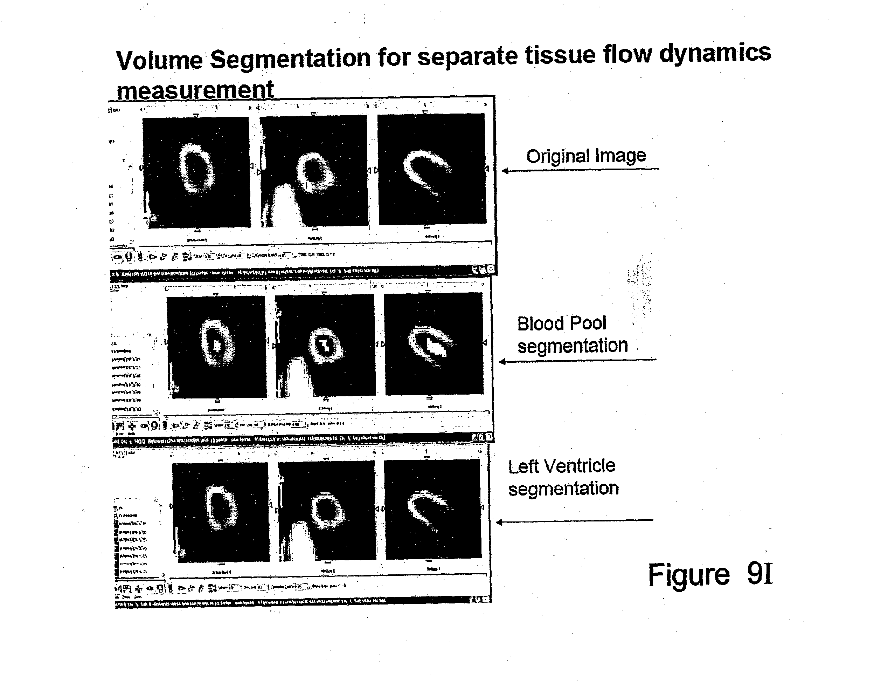

Figure 9I represents volumetric region segmentation for separate tissue flow dynamics measurement

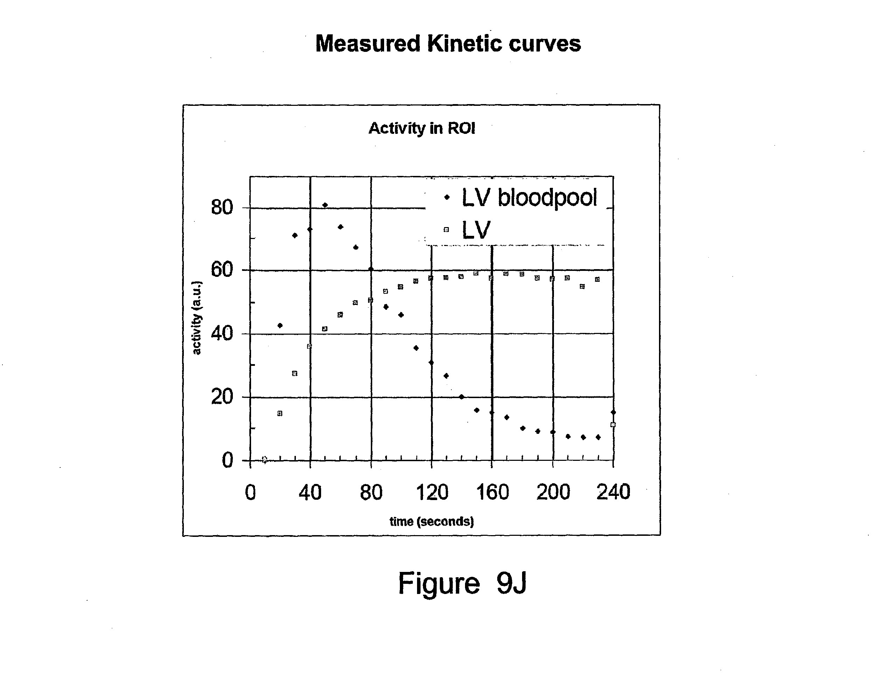

Figure 9J represents measured kinetic curves.

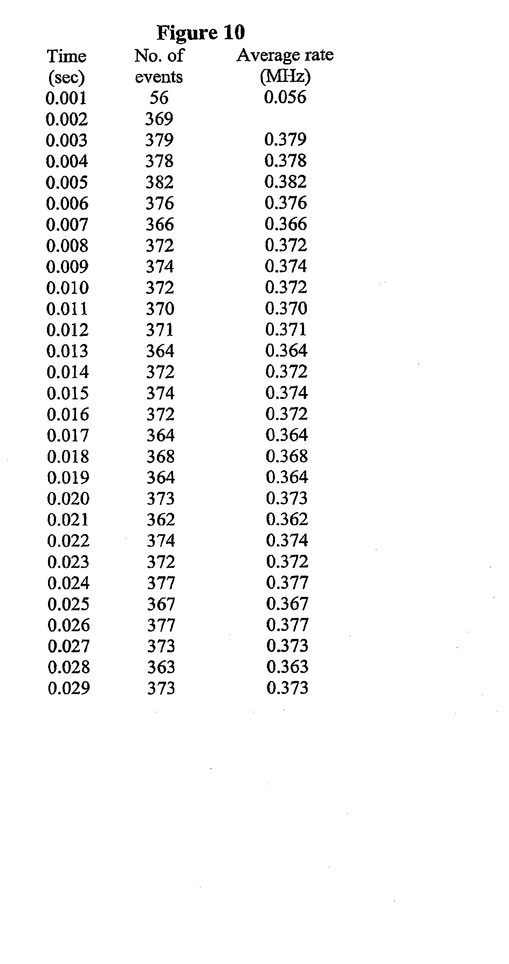

Figure 10 is another experiment, illustrating time binning at a rate of 0.001 seconds.

Electronic Scheme for Fast Throughput

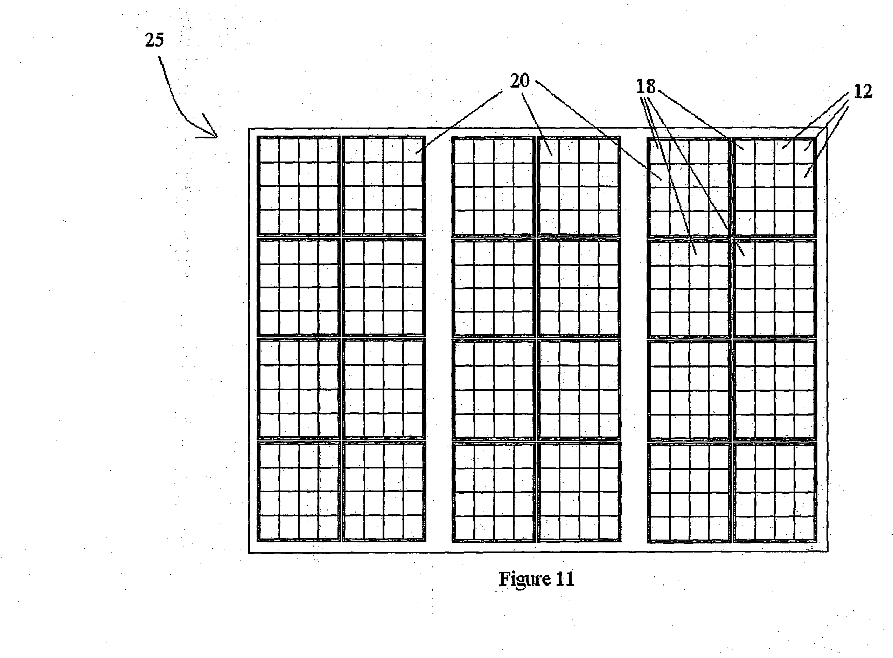

High-sensitivity detecting units, such as the room temperature, solid-state CdZnTe (CZT) detectors utilized in the present embodiments, must be discharged frequently, as their high-sensitivity can lead to rapid saturation. When a given detector saturates, the output count for the associated pixel no longer accurately reflects the number of incoming photons, but rather the maximum number that the detector is capable of absorbing. This inaccuracy may lead to errors during reconstruction. It is therefore important to perform readout often enough to avoid detector saturation.
The data channel from the assembly 20 (or the assembly 20 readout circuitry) to the signal processing components must be fast enough to handle the large quantities of data which are obtained from the detecting units 12.
The electronic scheme of the present embodiments preferably includes one or more of the following solutions for performing frequent detector unit readout, while maintaining high data throughput to prevent data channel saturation.
In a preferred embodiment, the dynamic SPECT camera 10 includes a parallel readout unit for performing parallel readout of emission count data. Parallel readout requires less time than serial readout (in which the pixels are read out in sequence), as multiple pixels may be read out in a single cycle without losing the information of the individual pixel counts. The readout rate can thus be increased without loss of data.
Parallel readout may be performed at many levels. Reference is now made to Figure 11, which illustrates various levels of detector unit organization at which parallel readout may be performed. The present exemplary embodiment shows a single detector array 25, which includes three assemblies 20. Each assembly includes a plurality of blocks 18 of detector units 12. Each detecting unit 12 includes a single-pixel detector (Figure 1D).

The parallel readout unit preferably performs parallel readout at the level of one or more of:

a) detecting units 12, each of the single-pixel detector 14;
b) blocks 18, which include a plurality of detecting units 12;
c) assemblies 20, which include a plurality of blocks 18
d) array 25, which includes a plurality of assemblies 20.

When the parallel readout unit performs parallel readout at the level of the detecting units 12, count values are read out in parallel from each of the electrically insulated single-pixel detector 14. The single-pixel detector 14 is discharged at readout, and the photon collection process begins anew.

When the parallel readout unit performs parallel readout at the level of the block 18, count values from each of the detecting units 12 are read out serially, however multiple blocks 18 are read out in parallel. This approach is less complex to implement than parallel readout of the detecting units 12, although it results in a certain reduction in readout rate to accommodate the serial readout. Again, the single-pixel detectors 14 are discharged at readout.

Similarly, when the parallel readout unit performs parallel readout at the level of the assembly 20, count values from each of the detecting units 12 in the assembly 20 are read out serially, however multiple assemblies 20 are read out in parallel.

Parallel readout preferably includes multiple detection, amplification and signal processing paths for each of the pixels, thereby avoiding saturation due to a single localized high emission area - "hot spot". This is in contrast with the Anger camera, in which multiple collimators are associated with a single-pixel scintillation detector, and saturation of the scintillation detector may occur even due to a localized hot spot.

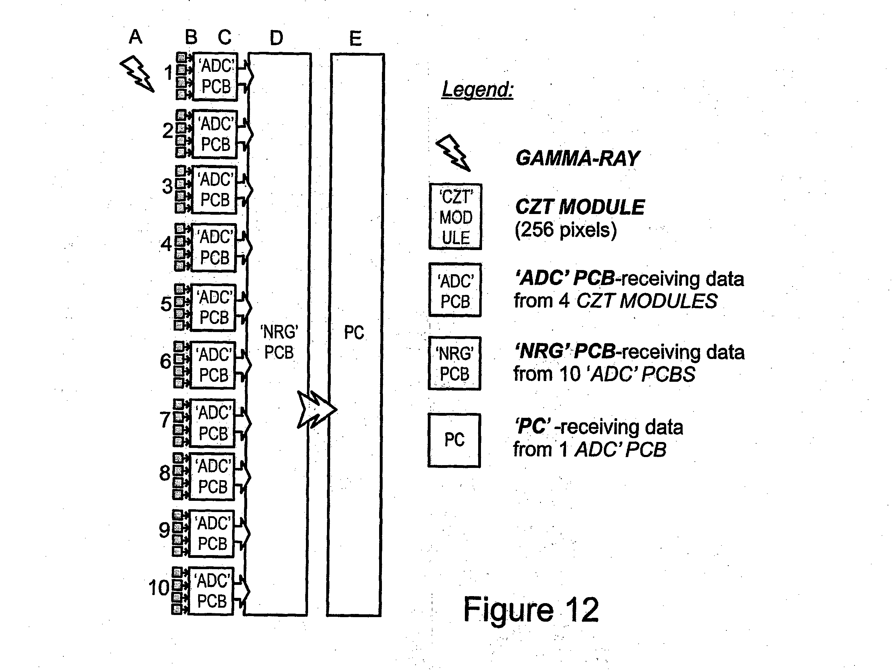

Figure 12 illustrates an exemplary embodiment of parallel readout in the dynamic SPECT camera 10. Radioactive emissions are detected by pixelated CZT crystals, where each crystal is divided into 256 pixels. The crystal is part of a 'CZT MODULE' (B) which also includes two ASICS each receiving events from 128 pixels. The ASIC is an OMS 'XAIM3.4' made by Orbotech Medical Systems, Rehovot, Israel, together with the CZT crystal. The 2 ASICs share a common output and transmit the data to 'ADC PCB' (C) that handles four 'CZT MODULES' (B) in parallel. Thus, a total of 1024 pixels are presently channeled through one ADC board. The system is capable of further increasing the accepted event rate by channeling every two ASICS through a single ADC. The 'ADC PCB' (C) transmits the data to the 'NRG PCB' (D) that handles ten 'ADC PCB's (C) in parallel, but could be further replicated should one want to further decrease "dead time". The 'NRG PCB' (D) transmits the data to the 'PC' (E) where it is stored.

All in all, in the present embodiment, forty CZT MODULEs which contain a total of 10240 pixels transmit in parallel to the PC.

The bottle neck, and hence the only constraint, of the system data flow is the ASICS in the 'CZT MODULE' (B) and the connection to the 'ADC PCB's (C):

1. An ASIC (128 pixels) can process one photon hit within 3.5uSec, or 285,000 events/sec over 128 pixels, i.e. over 2200 events/px/sec-an exceedingly high rate.
2. Two ASICS share the same output, and hence coincident event output of the two ASICS in a 'CZT MODULE' (B) will cause a collision and information loss. The duration of an event output from the ASIC is 1uSec.

When the readout is rapid, the rate at which the radiation emission data is read out of the single-pixel detectors 14 may be greater than the rate at which it may be output to the processor. One known solution for managing a difference data arrival and data processing rates is to use a buffer. The buffer provides temporary storage of the incoming data, which is retrieved at a later time.

A buffered readout configuration can result in the loss of timing information, unless active steps are taken to preserve the time information associated with the collected emission data, for example, as taught hereinabove, under the heading, "The Timing Mechanism 30."

In accordance with some implementations, timing information is preserved. The electrical scheme may include a buffer which stores emission data along with timing information for each data item or group of data items (in the case where emission data from several detectors was obtained at substantially the same time, for example due to parallel readout), and an identification of the associated detector unit. Utilizing a buffer ensures that emission data may be collected from the detectors frequently enough to avoid saturation, even when the data channel throughput is limited. In stop and shoot mode, for example, the emission count data may be stored in the buffer for retrieval while the detector head is moving to the next location. Accurate reconstruction may thus be performed.

The camera readout circuitry is preferably designed to provide fast readout and detecting unit discharge. Fast readout circuitry may include fast analog and digital circuitry, fast A/D converters, pipelined readout, and so forth. After the emission data has been read out of the single-pixel detectors 14, it may be necessary to convey the data to a processor for reconstruction as a single or limited number of data streams. The camera electronic scheme may include a multiplexer, for combining two or more emission data streams into a single data stream. The emission data may thus be conveyed to the processor over one physical link (or alternately over a reduced number of parallel links). For each radioactive emission event, the multiplexer includes both the timing information and an identification of the single-pixel detector 14 supplying the event. The multiplexed data may later be demultiplexed by the processor, and reconstruction may be performed with complete information for each data item, including for example, total counts per single-pixel detector 14, per time bin, single-pixel detector location and orientation, and the time bin. Parallel readout may thus be performed, even when the collected data is to be output over a single data link. Sensitivity Consideration.

It will be appreciated that dynamic imaging with a SPECT camera has been attempted in the past, unsuccessfully, primarily, because prior-art SPECT cameras are not sensitive enough to provide tomographic reconstruction images, for example, of a heart, with sufficient object resolution, for example, 10 X 10 X 10 cubic millimeters, in less than a minute.

As a case in point, US Patent 7,026,623, to Oaknin, et al., filed on January 7, 2004, issued on April 11, 2006, and entitled, "Efficient single photon emission imaging," describes a method of diagnostic imaging in a shortened acquisition time for obtaining a reconstructed diagnostic image of a portion of a body of a human patient who was administered with dosage of radiopharmaceutical substance radiating gamma rays, using an Anger Camera and SPECT imaging. The method includes effective acquisition times from less than 14 minutes to less than 8 minutes. Oaknin, et al., do not claim an effective acquisition time of less than 7 minutes. Yet, in view of the section entitled, "Time Scale Considerations," hereinabove, a sampling rate of 8 about minutes is far too slow for myocardial perfusion studies, where a sampling rate of at least two tomographic reconstruction images per heartbeat, that is, about every 30 seconds, is desired, and furthermore, where processes occur at rates of several seconds, and must be sampled at rates of a second or less, as seen in Figure 5B.

In some implementations, the dynamic SPECT camera 10 achieves sensitivity sufficient for the required sampling rates of between every 30 seconds and every half a second, by combining several features, specifically intended to increase sensitivity, as follows:

a collimator 16 with a solid collection angle δ of at least 0.005 steradians or greater, for a fast collection rate, and high sensitivity, wherein the loss in resolution is compensated by one or a combination of the following factors:

i. motion in a stop and shoot acquisition mode, at very small incremental steps, of between about 0.01 degrees and about 0.75 degrees.
ii. simultaneous acquisition by the assemblies 20, each scanning the same region of interest from a different viewing position, thus achieving both shorter acquisition time and better edge definitions.
iii. the structure 15 conforming to the body contours, for acquisition with substantial contact or near contact with the body.

Definition of a Clinically-Valuable Image

In consequence, the dynamic SPECT camera 10 is capable of producing a "clinically-valuable image" of an intra-body region of interest (ROI) containing a radiopharmaceutical, while fulfilling one or more of the following criteria:

1. the dynamic SPECT camera 10 is capable of acquiring at least one of 5000 photons emitted from the ROI during the image acquisition procedure, such as at least one of 4000, 3000, 2500, 2000, 1500, 1200, 1000, 800, 600, 400, 200, 100, or 50 photons emitted from the ROI. In one particular embodiment, the camera is capable of acquiring at least one of 2000 photons emitted from the ROI during the image acquisition procedure;
2. the dynamic SPECT camera 10 is capable of acquiring at least 200,000 photons, such as at least 500,000, 1,000,000, 2,000,000, 3,000,000, 4,000,000, 5,000,000, 8,000,000, or 10,000,000 photons, emitted from a portion of the ROI having a volume of no more than 500 cc, such as a volume of no more than 500 cc, 400 cc, 300 cc, 200 cc, 150 cc, 100cc, or 50 cc. In one particular embodiment, the camera is capable of acquiring at least 1,000,000 photons emitted from a volume of the ROI having a volume of no more than 200 cc;
3. the dynamic SPECT camera 10 is capable of acquiring an image of a resolution of at least 7x7x7 mm, such as at least 6x6x6 mm, 5x5x5 mm, 4x4x4 mm, 4x3x3 mm, or 3x3x3 mm, in at least 50% of the reconstructed volume, wherein the radiopharmaceutical as distributed within the ROI has a range of emission-intensities I (which is measured as emitted photons / unit time / volume), and wherein at least 50% of the voxels of the reconstructed three-dimensional emission-intensity image of the ROI have inaccuracies of less than 30% of range I, such as less than 25%, 20%, 15%, 10%, 5%, 2%, 1%, or 0.5% of range I. For example, the radiopharmaceutical may emit over a range from 0 photons/second/cc to $10^5$ photons/second/cc, such that the range I is $10^5$ photons/second/cc, and at least 50% of the voxels of the reconstructed three-dimensional intensity image of the ROI have inaccuracies of less than 15% of range I, i.e., less than $1.5 \times 10^4$ photons/second/cc. For some applications, the study produce a parametric image related to a physiological process occurring in each voxel. In one particular embodiment, the image has a resolution of at least 5x5x5 mm, and at least 50% of the voxels have inaccuracies of less than 15% of range I;

4. the dynamic SPECT camera 10 is capable of acquiring an image, which has a resolution of at least 7x7x7 mm, such as at least 6x6x6 mm, 5x5x5 mm, 4x4x4 mm, 4x3x3 mm, or 3x3x3 mm, in at least 50% of the reconstructed volume, wherein the radiopharmaceutical as distributed within the ROI has a range of emission-intensities I (which is measured as emitted photons / unit time / volume), and wherein at least 50% of the voxels of the reconstructed three-dimensional emission-intensity image of the ROI have inaccuracies of less than 30% of range I, such as less than 25%, 20%, 15%, 10%, 5%, 2%, 1%, or 0.5% of range I. For example, the radiopharmaceutical may emit over a range from 0 photons/second/cc to $10^5$ photons/second/cc, such that the range I is $10^5$ photons/second/cc, and at least 50% of the voxels of the reconstructed three-dimensional intensity image of the ROI have inaccuracies of less than 15% of range I, i.e., less than $1.5 \times 10^4$ photons/second/cc. For some applications, the study produces a parametric image related to a physiological process occurring in each voxel. In one particular embodiment, the image has a resolution of at least 5x5x5 mm, and at least 50% of the voxels have inaccuracies of less than 15% of range I;

5. the dynamic SPECT camera 10 is capable of acquiring an image, which has a resolution of at least 20x20x20 mm, such as at least 15x15x15 mm, 10x10x10 mm, 7x7x7 mm, 5x5x5 mm, 4x4x4 mm, 4x3x3 mm, or 3x3x3 mm, wherein values of parameters of a physiological process modeled by a parametric representation have a range of physiological parameter values I, and wherein at least 50% of the voxels of the reconstructed parametric three-dimensional image have inaccuracies less than 100% of range I, such as less than 70%, 50%, 40%, 30%, 25%, 20%, 15%, 10%, 5%, 2%, 1%, or 0.5% of range I. For example, the physiological process may include blood flow, the values of the parameters of the physiological process may have a range from 0 to 100 cc / minute, such that the range I is 100 cc / minute, and at least 50% of the voxels of the reconstructed parametric three-dimensional image have inaccuracies less than 25% of range I, i.e., less than 25 cc / minute. In one particular embodiment, the image has a resolution of at least 5x5x5 mm, and at least 50% of the voxels have inaccuracies of less than 25% of range I; and/or

6. the dynamic SPECT camera 10 is capable of acquiring an image, which has a resolution of at least 7x7x7 mm, such as at least 6x6x6 mm, 5x5x5 mm, 4x4x4 mm, 4x3x3 mm, or 3x3x3 mm, in at least 50% of the reconstructed volume, wherein if the radiopharmaceutical is distributed substantially uniformly within a portion of the ROI with an emission-intensity I +/- 10% (which is defined as emitted photons / unit time / volume), and wherein at least 85% of the voxels of the reconstructed three-dimensional emission-intensity image of the portion of the ROI have inaccuracies of less than 30% of intensity I, such as less than 15%, 10%, 5%, 2%, 1%, 0.5%, 20%, or 25% of intensity I. For example, the radiopharmaceutical may be distributed within a volumetric region with a uniform emission-intensity I of 10^5 photons/second/cc, and at least 85% of the voxels of the reconstructed three-dimensional intensity image of the volumetric region have inaccuracies of less than 15% of intensity I, i.e., less than $1.5 \times 10^4$ photons/second/cc. For some applications, the same definition may apply to a study which produces a parametric image related to a physiological process occurring in each voxel. In one particular embodiment, the image has a resolution of at least 5x5x5 mm, and at least 50% of the voxels have inaccuracies of less than 15% of intensity I.

It is expected that during the life of this patent many relevant dynamic SPECT cameras will be developed and the scope of the term dynamic SPECT camera is intended to include all such new technologies *a priori*.

As used herein the term "substantially" refers to $\pm$ 10 %.

As used herein the term "about" refers to $\pm$ 30 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. In addition, citation or identification of any reference in this application shall not be construed as an admission that such reference is available as prior art to the present invention.

It is expected that during the life of this patent many relevant devices and systems will be developed and the scope of the terms herein, particularly of the terms SPECT detectors, processing unit, communication, and images are intended to include all such new technologies *a priori*.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. In addition, citation or identification of any reference in this application shall not be construed as an admission that such reference is available as prior art to the present invention.

**Claims**

1. A method for reconstructing a radioactive emission image of an overall volume (1001) having first and second volumetric regions (1003, 1002), each volumetric region having respectively independent dynamic and static characteristics, the method comprising:

   a) obtaining (1301) radioactive emissions from the overall volume (1001), including the volumetric regions (1003, 1002), wherein at least one of the volumetric regions (1003, 1002) comprises a body organ (110, 200, 1004) or at least part of another body portion;
   b) reconstructing (1302) an initial radioactive emission image of the overall volume (1001) according to said radioactive emissions;
   c) segmenting (1303, 1310, 1311), performed by a data processor, said initial radioactive emission image to delineate the first and second volumetric regions (1003, 1002); and
   d) separately reconstructing (1304-1306, 1312-1314), performed by a data processor, the first and second volumetric regions (1003, 1002) according to the respectively independent dynamic and static characteristics;
   **characterised in that**:

   said separately reconstructing (1304-1306, 1312-1314) is an iterative process including using object implantation for refining reconstruction of at least one of the first and second volumetric regions (1003, 1002), wherein said object implantation includes:

   providing a model of only a part of said overall volume, said model comprising a general location and shape of, and expected number of photons emitted from, said body organ (110, 200, 1004) or said at least part of body portion;
   replacing, performed by a data processor, at a general location in said initial radioactive emission image corresponding to said model general location, initial radioactive emission image voxels with said model general shape and initial radioactive emission image voxel photon counts, based on said expected number of photons from said model, where said replacing is one or more times during said iterative process, each time for providing a better starting point for performing next iteration of said reconstruction of at least one of the first and second volumetric regions.

2. The method of claim 1, further comprising:

   representing said initial radioactive emission image by a system (202) of voxels;
   providing a structural image (200) of a region of interest in said system (202) of voxels; and
   constructing an anatomical construction (222, 224, 228) of voxels, for said region of interest, in which voxel boundaries are aligned with boundaries of structural objects (202, 204, 206, 208, 218) of said region of interest, based on said structural image (200),
   wherein said separately reconstructing (1304-1306, 1312-1314) is based on said anatomical construction (222, 224, 228) of voxels.

3. The method of claim 2, wherein said structural image is provided by a source selected from the group consisting of 2-D ultrasound, 3-D ultrasound, planner x-rays, CT x-rays, MRI, and an anatomical atlas.

4. The method of claim 3, wherein said structural image is co-registered to said initial radioactive emission image.

5. The method of claim 2, wherein said anatomical construction (222, 224, 228) of voxels includes voxels of varying volumetric regions, depending on their anatomic position and relevance.

6. The method of claim 1, wherein said obtaining (1301) radioactive emissions further includes obtaining timing data by a method selected from the group consisting of time stamping, time binning, and a combination thereof.

7. The method of claims 6, wherein said timing data are employed in gating, and further wherein different gating is employed for the first and second volumetric regions (1003, 1002), based on the respectively independent dynamic and static characteristics.

8. The method of claim 7, wherein each of said gating may be of a dynamically varying duration.

9. The method of claim 1, wherein said obtaining (1301) radioactive emissions includes obtaining without timing data, by prescanning, for defining the first and second volumetric regions (1003, 1002), and further including:

obtaining a second set of radioactive emissions from the overall volume (1001), including the volumetric regions (1003, 1002), after said reconstructing (1302) said initial radioactive emission image, said second set including timing data,
wherein said separately reconstructing (1304-1306, 1312-1314) includes separately reconstructing the first and second volumetric regions (1003, 1002) according to the respectively independent dynamic and static characteristics, from said second set.

10. The method of claim 9, wherein said timing data are employed in gating, and further wherein different gating is employed for the first and second volumetric regions (1003, 1002), based on the respectively independent dynamic and static characteristics.

11. The method of claim 10, wherein each of said gating may be of a dynamically varying duration.

12. The method of claim 1, further comprising displaying said radioactive emission image and allowing a system user to mark at least one of the first and second volumetric regions (1003, 1002), said segmenting (1301, 1310, 1311) being performed according to said mark.

13. The method of claim 1, wherein said segmenting (1301, 1310, 1311) said initial radioactive emission image is based on the respectively independent dynamic and static characteristics.

14. The method of claim 1, wherein the volumetric regions (1003, 1002) comprise independent sub-volumetric regions, wherein during said separately reconstructing (1304-1306, 1312-1314), each one of said independent sub-volumetric regions is separately reconstructed.

15. The method of claim 1, wherein said radioactive emissions are obtained (1301) using a single photon emission computed tomography (SPECT) camera (10).

**Patentansprüche**

1. Verfahren zum Rekonstruieren eines Bildes radioaktiver Emissionen eines Gesamtvolumens (1001) mit ersten und zweiten Volumenbereichen (1003, 1002), wobei jeder Volumenbereich jeweils unabhängige dynamische und statische Charakteristika aufweist, wobei das Verfahren umfasst:

a) Erhalten (1301) von radioaktiven Emissionen aus dem Gesamtvolumen (1001), einschließlich der Volumenbereiche (1003, 1002), wobei zumindest einer der Volumenbereiche (1003, 1002) ein Körperorgan (110, 200, 1004) oder zumindest einen Teil eines weiteren Körperabschnitts umfasst;
b) Rekonstruieren (1302) eines anfänglichen Bildes radioaktiver Emissionen des Gesamtvolumens (1001) gemäß den radioaktiven Emissionen;
c) Segmentieren (1303, 1310, 1311) des anfänglichen Bildes radioaktiver Emissionen durch einen Datenprozessor, um die ersten und zweiten Volumenbereiche (1003, 1002) abzugrenzen; und
d) separates Rekonstruieren (1304-1306, 1312-1314) der ersten und zweiten Volumenbereiche (1003, 1002) gemäß den jeweils unabhängigen dynamischen und statischen Charakteristika durch einen Datenprozessor; **dadurch gekennzeichnet, dass**:

das separate Rekonstruieren (1304-1306, 1312-1314) ein iterativer Prozess ist, der das Verwenden von Objektimplantation beinhaltet, um die Rekonstruktion von zumindest einem der ersten und zweiten Volumenbereiche (1003, 1002) zu verfeinern,
wobei die Objektimplantation beinhaltet:

Bereitstellen eines Modells von nur einem Teil des Gesamtvolumens, wobei das Modell eine allgemeine Position und Form des Körperorgans (110, 200, 1004) oder zumindest des Teils des Körperabschnitts und eine antizipierte Anzahl von daraus emittierten Photonen umfasst;
Ersetzen von Voxeln des anfänglichen Bildes radioaktiver Emissionen an einer allgemeinen Position im anfänglichen Bild radioaktiver Emissionen, die der allgemeinen Position des Modells entspricht, durch die Voxelphotonenzählungen der allgemeinen Form des Modells und des anfänglichen Bildes radioaktiver Emissionen auf Basis der antizipierten Anzahl von Photonen aus dem Modell durch einen Datenprozessor, wobei das Ersetzen einmal oder mehrmals während des iterativen Prozesses erfolgt, jeweils um einen besseren Ausgangspunkt für das Durchführen einer nächsten Iteration der Rekonstruktion zumindest eines der ersten und zweiten Volumenbereiche bereitzustellen.

2. Verfahren nach Anspruch 1, das ferner umfasst:

Darstellen des anfänglichen Bildes radioaktiver Emissionen durch ein System (202) von Voxeln;
Bereitstellen eines Strukturbildes (200) eines Bereichs von Interesse im System (202) von Voxeln; und
Konstruieren einer anatomischen Konstruktion (222, 224, 228) von Voxeln für den Bereich von Interesse, wobei die Voxelgrenzen mit Grenzen von Strukturobjekten (202, 204, 206, 208, 218) des Bereichs von Interesse ausgerichtet sind, auf Basis des Strukturbildes (200),
wobei das separate Rekonstruieren (1304-1306, 1312-1314) auf der anatomischen Konstruktion (222, 224, 228) von Voxeln basiert.

3. Verfahren nach Anspruch 2, wobei das Strukturbild von einer Quelle bereitgestellt wird, die aus der Gruppe ausgewählt ist, bestehend aus 2D-Ultraschall, 3D-Ultraschall, Planner-Röntgen, CT-Röntgen, MRT und einem anatomischen Atlas.

4. Verfahren nach Anspruch 3, wobei das Strukturbild mit dem anfänglichen Bild radioaktiver Emissionen abgeglichen wird.

5. Verfahren nach Anspruch 2, wobei die anatomische Konstruktion (222, 224, 228) von Voxeln das Variieren von Volumenbereichen je nach deren anatomischen Position und Relevanz beinhaltet.

6. Verfahren nach Anspruch 1, wobei das Erhalten (1301) von radioaktiven Emissionen ferner das Erhalten von Zeitnahmedaten mit einem Verfahren beinhaltet, das aus der Gruppe ausgewählt ist, bestehend aus Zeitstempelung, Zeit-Binning und einer Kombination davon.

7. Verfahren nach Anspruch 6, wobei die Zeitnahmedaten beim Gating verwendet werden, und ferner wobei ein unterschiedliches Gating für die ersten und zweiten Volumenbereiche (1003, 1002) auf Basis der jeweils unabhängigen dynamischen und statischen Charakteristika verwendet wird.

8. Verfahren nach Anspruch 7, wobei jedes Gating eine dynamisch variierende Dauer aufweisen kann.

9. Verfahren nach Anspruch 1, wobei das Erhalten (1301) von radioaktiven Emissionen das Erhalten ohne Zeitnahmedaten durch Vorabtastung beinhaltet, um die ersten und zweiten Volumenbereiche (1003, 1002) zu definieren, und das ferner beinhaltet:

Erhalten eines zweiten Satzes von radioaktiven Emissionen aus dem Gesamtvolumen (1001), einschließlich der Volumenbereiche (1003, 1002), nach dem Rekonstruieren (1302) des anfänglichen Bildes radioaktiver Emissionen, wobei der zweite Satz Zeitnahmedaten enthält,
wobei das separate Rekonstruieren (1304-1306, 1312-1314) das separate Rekonstruieren der ersten und zweiten Volumenbereiche (1003, 1002) gemäß den jeweils unabhängigen dynamischen und statischen Charakteristika aus dem zweiten Satz beinhaltet.

10. Verfahren nach Anspruch 9, wobei die Zeitnahmedaten beim Gating verwendet werden, und ferner wobei ein unterschiedliches Gating für die ersten und zweiten Volumenbereiche (1003, 1002) auf Basis der jeweils unabhängigen dynamischen und statischen Charakteristika verwendet wird.

**11.** Verfahren nach Anspruch 10, wobei jedes Gating eine dynamisch variierende Dauer aufweisen kann.

**12.** Verfahren nach Anspruch 1, das ferner das Anzeigen des Bildes radioaktiver Emissionen und das Zulassen, dass ein Systembenutzer zumindest einen der ersten und zweiten Volumenbereiche (1003, 1002) markiert, umfasst, wobei das Segmentieren (1301, 1310, 1311) gemäß der Markierung durchgeführt wird.

**13.** Verfahren nach Anspruch 1, wobei das Segmentieren (1301, 1310, 1311) des anfänglichen Bildes radioaktiver Emissionen auf den jeweils unabhängigen dynamischen und statischen Charakteristika basiert.

**14.** Verfahren nach Anspruch 1, wobei die Volumenbereiche (1003, 1002) unabhängige Teilvolumenbereiche umfassen, wobei jeder der unabhängigen Teilvolumenbereiche während des separaten Rekonstruierens (1304-1306, 1312-1314) separat rekonstruiert wird.

**15.** Verfahren nach Anspruch 1, wobei die radioaktiven Emissionen unter Verwendung einer Einzelphotonen-Emissionscomputertomographie-(SPECT-)Kamera (10) erhalten (1301) werden.

## Revendications

**1.** Procédé pour reconstruire une image d'émission radioactive d'un volume global (1001) comportant des première et deuxième régions volumétriques (1003, 1002), chaque région volumétrique présentant des caractéristiques dynamiques et statiques respectivement indépendantes, le procédé comprenant

    a) l'obtention (1301) d'émissions radioactives à partir du volume global (1001), comprenant les régions volumétriques (1003, 1002), dans lequel au moins l'une des régions volumétriques (1003, 1002) comprend un organe corporel (110, 200, 1004) ou au moins une partie d'une autre partie corporelle ;
    b) la reconstruction (1302) d'une image d'émission radioactive initiale du volume global (1001) conformément aux dites émissions radioactives ;
    c) la segmentation (1303, 1310, 1311), effectuée par un processeur de données, de ladite image d'émission radioactive initiale pour représenter les première et deuxième régions volumétriques (1003, 1002) ; et
    d) la reconstruction séparée (1304 à 1306, 1312 à 1314), effectuée par un processeur de données, des première et deuxième régions volumétriques (1003, 1002) conformément aux caractéristiques dynamiques et statiques respectivement indépendantes ; **caractérisé en ce que**
    ladite reconstruction séparée (1304 à 1306, 1312 à 1314) est un processus itératif comprenant l'utilisation d'une implantation d'objet pour affiner la reconstruction d'au moins l'une des première et deuxième régions volumétriques (1003, 1002),
    dans lequel ladite implantation d'objet comprend :

        la fourniture d'un modèle uniquement d'une partie dudit volume global, ledit modèle comprenant un emplacement général et une forme générale, et un nombre attendu de photons émis à partir dudit organe corporel (110, 200, 1004) ou ladite au moins une partie de partie corporelle ;
        le remplacement, effectué par un processeur de données, à un emplacement général dans ladite image d'émission radioactive initiale correspondant au dit emplacement général de modèle, des voxels d'image d'émission radioactive initiale par lesdits comptes de photons de voxels de la forme générale de modèle et de l'image d'émission radioactive initiale, sur la base dudit nombre attendu de photons dudit modèle, dans lequel ledit remplacement est effectué une ou plusieurs fois pendant ledit processus itératif, à chaque fois pour fournir un meilleur point de départ pour effectuer une itération suivante de ladite reconstruction d'au moins l'une des première et deuxième régions volumétriques.

**2.** Procédé selon la revendication 1, comprenant en outre :

    la représentation de ladite image d'émission radioactive initiale par un système (202) de voxels ;
    la fourniture d'une image structurelle (200) d'une région présentant un intérêt dans ledit système (202) de voxels ; et
    la construction d'une construction anatomique (222, 224, 228) de voxels, pour ladite région présentant un intérêt, dans lequel les frontières des voxels sont alignées avec les frontières des objets structurels (202, 204, 206, 208, 218) de ladite région présentant un intérêt, sur la base de ladite image structurelle (200),
dans lequel ladite reconstruction séparée (1304 à 1306, 1312 à 1314) est basée sur ladite construction anatomique (222, 224, 228) de voxels.

3. Procédé selon la revendication 2, dans lequel ladite image structurelle est fournie par une source sélectionnée dans le groupe consistant en un ultrason bidimensionnel, un ultrason tridimensionnel, des rayons X de planificateur, des rayons X de CT, IRM, et un atlas anatomique.

4. Procédé selon la revendication 3, dans lequel ladite image structurelle est alignée avec ladite image d'émission radioactive initiale.

5. Procédé selon la revendication 2, dans lequel ladite construction anatomique (222, 224, 228) de voxels comprend des voxels de régions volumétriques variables, en fonction de leur position et de leur intérêt anatomiques.

6. Procédé selon la revendication 1, dans lequel ladite obtention (1301) d'émissions radioactives comprend en outre l'obtention de données de synchronisation par un procédé sélectionné dans le groupe consistant en un horodatage, un regroupement temporel, et une combinaison de ceux-ci.

7. Procédé selon la revendication 6, dans lequel lesdites données de synchronisation sont utilisées pour la synchronisation, et en outre dans lequel différentes synchronisations sont utilisées pour les première et deuxième régions volumétriques (1003, 1002), sur la base des caractéristiques dynamiques et statiques respectivement indépendantes.

8. Procédé selon la revendication 7, dans lequel chacune desdites synchronisations peut avoir une durée dynamiquement variable.

9. Procédé selon la revendication 1, dans lequel ladite obtention (1301) d'émissions radioactives comprend l'obtention sans données de synchronisation, par un balayage préalable, pour définir les première et deuxième régions volumétriques (1003, 1002), et comprenant en outre :

l'obtention d'un deuxième ensemble d'émissions radioactives à partir du volume global (1001), comprenant les régions volumétriques (1003, 1002), après ladite reconstruction (1302) de ladite image d'émission radioactive initiale, ledit deuxième, ensemble comprenant des données de synchronisation,
dans lequel ladite reconstruction séparée (1304 à 1306, 1312 à 1314) comprend la reconstruction séparée des première et deuxième régions volumétriques (1003, 1002) conformément aux caractéristiques dynamiques et statiques respectivement indépendantes, à partir dudit deuxième ensemble.

10. Procédé selon la revendication 9, dans lequel lesdites données de synchronisation sont utilisées pour la synchronisation, et en outre dans lequel différentes synchronisations sont utilisées pour les première et deuxième régions volumétriques (1003, 1002), sur la base des caractéristiques dynamiques et statiques respectivement indépendantes.

11. Procédé selon la revendication 10, dans lequel chacune desdites synchronisations peut avoir une durée dynamiquement variable.

12. Procédé selon la revendication 1, comprenant en outre l'affichage de ladite image d'émission radioactive et le marquage éventuel, par un utilisateur du système, d'au moins l'une des première et deuxième régions volumétriques (1003, 1002), ladite segmentation (1301, 1310, 1311) étant effectuée conformément à ladite marque.

13. Procédé selon la revendication 1, dans lequel ladite segmentation (1301, 1310, 1311) de ladite image d'émission radioactive initiale est basée sur les caractéristiques dynamiques et statiques respectivement indépendantes.

14. Procédé selon la revendication 1, dans lequel les régions volumétriques (1003, 1002) comprennent des régions sous-volumétriques indépendantes, dans lequel, pendant ladite reconstruction séparée (1304 à 1306, 1312 à 1314), chacune desdites régions sous-volumétriques indépendantes est reconstruite séparément.

15. Procédé selon la revendication 1, dans lequel lesdites émissions radioactives sont obtenues (1301) en utilisant une caméra de tomodensitométrie d'émission à photon unique (SPECT) (10).

Figure 1B

Figure 1D

Figure 1C

Figure 1A

Figure 2A

Figure 2B

Figure 3D

Figure 3A

Figure 3C

Figure 3B

Figure 4C

Figure 4F

Figure 4B

Figure 4E

Figure 4A

Figure 4D

Figure 5A

Figure 5B

45

EP 1 971 257 B1

81

82       82

|← RR Interval →|← RR Interval →|← RR Interval →|

83

89

84

91

89

83

1 2 3 4 5 6 8 10 12 14 16 2 4 6 8 10 12 14 16 2 4 6 8 10 12 14 16

Figure 5C

81

96   97     96   97     96   97     96

85

87    87    87    87

86    86    86    86

Figure 5D

point of alignment 94          point of alignment 94          graduation time scale 92

coarse-graduation          discarded zone 95
time scale 98

fine-graduation
time scale 93

1 2 3 4 5 6 7 8 9 10 11 12 13 14 15 16   1 2 3 4 5 6 7

index 84

**Figure 5E**

RR Interval

QRS Complex                                        R

ST
Segment   T-Wave

P-Wave        U-Wave

81

PR

Q  S

QT

QRS

**Figure 5F**

EP 1 971 257 B1

Figure 6A

Figure 6B

Figure 6C

Figure 6D

Figure 6F

Figure 6E

Figure 6I

Figure 6H

Figure 6G

Figure 7

Figure 8A

Figure 8B

EP 1 971 257 B1

# Anatomical Landmarks

SA

HLA

Blood Pool    Myocardium

- Left Ventricle (LV)
- Right Ventricle (RV)
- Left Atrium (LA)
- Right Atrium (RA)

**Figure 9A**

EP 1 971 257 B1

DYNAMIC STUDY INPUT

bloodpool, mayocardium, and body timed activity

Legend:
- bloodpool (spline)
- myo (spline)
- body (spline)
- infarct(spline)

Frame 8 :
545 kC in LV

Frame 15 :
338 kC in LV

Frame 23 :
271 kC in LV

Figure 9B

EP 1 971 257 B1

# "Film-stripe" representation of a dynamic SPECT study

Seconds from Injection

- First 2 minutes after Tc99m-Teboroxime* injection, 10s/frame
- Mid-ventricular slices (upper row : SA lower row: HLA)
- Note as the intense blood pool activity at the center of the heart chambers gradually clears, while the myocardial uptake gradually intensifies.

Figure 9C

EP 1 971 257 B1

# "Film-stripe" representation of a dynamic SPECT study

Seconds after Injection

- First 4 minutes after Tc99m-Teboroxime* injection, 10s/frame
- Mid-ventricular slices (upper row : SA lower row: HLA)
- Note as the intense blood pool activity at the center of the heart chambers gradually clears, while the myocardial uptake gradually intensifies

Figure 9D

EP 1 971 257 B1

# "Movie" representation of a dynamic SPECT study (SA)

- First 4 minutes after Tc99m-Teboroxime* injection, 10s/frame
- Mid-ventricular SA slices.
- Note as the intense blood pool activity gradually clears in LV and RV cavities
- Myocardial uptake gradually intensifies, (the thin walled RV is less intense)

## Figure 9E

EP 1 971 257 B1

# "Movie" representation of a dynamic SPECT study (SA)

- First 4 minutes after Tc99m-Teboroxime* injection, 10s/frame
- Mid-ventricular SA slices.
- Note as the intense blood pool activity gradually clears in LV, RV, LA and RA cavities
- Myocardial uptake gradually intensifies, (the thin walled RV ant atria are less intense)

Figure 9F

EP 1 971 257 B1

# "Movie" representation of a dynamic SPECT study (fast)

Figure 9G

# "Movie" representation of a dynamic SPECT study (slow)

Figure 9H

EP 1 971 257 B1

# Volume Segmentation for separate tissue flow dynamics measurement

Original Image

Blood Pool segmentation

Left Ventricle segmentation

Figure 9I

**Measured Kinetic curves**

Activity in ROI

- ◆ LV bloodpool
- ▫ LV

activity (a.u.)

time (seconds)

Figure 9J

## Figure 10

| Time (sec) | No. of events | Average rate (MHz) |
|---|---|---|
| 0.001 | 56 | 0.056 |
| 0.002 | 369 | |
| 0.003 | 379 | 0.379 |
| 0.004 | 378 | 0.378 |
| 0.005 | 382 | 0.382 |
| 0.006 | 376 | 0.376 |
| 0.007 | 366 | 0.366 |
| 0.008 | 372 | 0.372 |
| 0.009 | 374 | 0.374 |
| 0.010 | 372 | 0.372 |
| 0.011 | 370 | 0.370 |
| 0.012 | 371 | 0.371 |
| 0.013 | 364 | 0.364 |
| 0.014 | 372 | 0.372 |
| 0.015 | 374 | 0.374 |
| 0.016 | 372 | 0.372 |
| 0.017 | 364 | 0.364 |
| 0.018 | 368 | 0.368 |
| 0.019 | 364 | 0.364 |
| 0.020 | 373 | 0.373 |
| 0.021 | 362 | 0.362 |
| 0.022 | 374 | 0.374 |
| 0.023 | 372 | 0.372 |
| 0.024 | 377 | 0.377 |
| 0.025 | 367 | 0.367 |
| 0.026 | 377 | 0.377 |
| 0.027 | 373 | 0.373 |
| 0.028 | 363 | 0.363 |
| 0.029 | 373 | 0.373 |

Figure 11

Figure 12

Fig. 13 (Prior art)

**Apparatus for reconstructing a radioactive emission image**

990

991 → Segmentation module ↔ Image reconstruction module ← 992

993

1002

1003

**Fig. 14**

EP 1 971 257 B1

1001

1002

1003

III

III

1004

## Fig. 15

1001A

1004

1002A

1003A

## Fig. 16

```
           ┌─────────────────────────────────────────┐
      ──────▶│           Fetch a dataset               │
  1301       └─────────────────────────────────────────┘
                              │
                              ▼
           ┌─────────────────────────────────────────┐
      ──────▶│    Generate an initial estimation image │
  1302       └─────────────────────────────────────────┘
                              │
                              ▼
           ┌─────────────────────────────────────────┐
      ──────▶│  Segment the initial estimation image   │◀──┐
  1303       │    to gated and non-gated regions       │   │
           └─────────────────────────────────────────┘   │
                              │                           │
                              ▼                           │
           ┌─────────────────────────────────────────┐   │
           │           Use time binning               │   │
      ──────▶│  For updating the gated and non-gated   │   │
  1304       │          regions separately             │   │
           └─────────────────────────────────────────┘   │
                              │                           │
                              ▼                           │
           ┌─────────────────────────────────────────┐   │
           │  Reconstruct the overall volume using    │   │
      ──────▶│    the updated gated and non-gated      │   │
  1305       │               regions                   │   │
           └─────────────────────────────────────────┘   │
                              │                           │
                              ▼                           │
                        ◇ Is the ◇                        │
      ──────▶      reconstruction ───────────────────────┘
  1306             ◇ acceptable ◇
                              │
                              ▼
                         ( END )
```

**Fig. 17**

| Non-gated voxels | Frame I Voxels | ● ● ● | Frame M Voxels |

1101  1002A  1002G

$|I_c$

Fig. 18

1301 — Fetch a dataset

1302 — Generate an initial estimation image

1310 — Segment the non-gated region

1311 — Segment the gated region

1312 — Use time binning for updating the gated region

1313 — Reconstruct a overall volume using the updated gated region and the non-gated region

1314 — Is the reconstruction acceptable

END

**Fig. 19**

**Fig. 20**

**EP 1 971 257 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6628984 B, Weinberg
- US 6242743 B, DeVito
- US 5939724 A, Eisen
- US 6525320 B, Juni
- US 6271525 B, Majewski
- US 6271524 B, Wainer
- US 6212423 B, Krakovitz
- WO 0010034 A
- US 2003010539 A, Turner
- US 5722405 A, Goldberg
- US 7026623 B, Oaknin
- IL 2005001173 W

**Non-patent literature cited in the description**

- **GILLAND 0 R et al.** A 3D model of non-uniform attenuation and detector response for efficient iterative reconstruction in SPECT. *PHYSICS IN MEDICINE AND BIOLOGY,* March 1994, vol. 39 (3), 547-561
- Simultaneous Reconstruction and Motion Estimation for Gated Cardiac ECT. **DAVID R GILLAND et al.** IEEE TRANSACTIONS ON NUCLEAR SCIENCE. IEEE SERVICE CENTER, 01 October 2002, vol. 49
- **HONGDI LI et al.** A HOTlinkinetworked PC data acquisition and image reconstruction system for a high resolution whole-body PET with respiratory or ECG gated performance ho. *2002 IEEE NUCLEAR SCIENCE SYMPOSIUM CONFERENCE RECORD/2002 IEEE NUCLEAR SCIENCE SYMPOSIUM AND MEDICAL IMAGING CONFERENCE,* 10 November 2002, vol. 2, 1135-1139
- **XIAOLONG OUYANG et al.** Incorporation of correlated structural images in the PET image reconstruction. *IEEE TRANSACTION ON MEDICAL IMAGING USA,* December 1994, vol. 13 (4
- **LEAHY R et al.** Computer tomography in: Handbook of Image and Video Processing, BovikA. Academic press, 2000, 771-787
- **J. KAY.** The EM algorithm in medical imaging. *Stat. Meth. Med. Res.,* January 1997, vol. 6 (1), 55-75
- Statistical image reconstruction methods for transmission tomography. **J. A. FESSLER.** Handbook of Medical Imaging. 2000, 1-70
- **R. M. LEAHY et al.** *Statistical approaches in quantitative positron emission tomography,* April 2000, vol. 10 (2), 147-65
- **M. DEFRISE.** A short reader's guide to 3D tomographic reconstruction. *Computerized Medical Imaging and Graphics,* March 2001, vol. 25 (2), 1 13-6
- **VANDENBERGHE, Y. D'ASSELER et al.** Iterative reconstruction algorithms in nuclear medicine. *Computerized Medical Imaging and Graphics,* March 2001, vol. 25 (2), 105-11
- **G. L. ZENG.** *Image reconstruction, a tutorial, Computerized Medical Imaging and Graphics,* March 2001, vol. 25 (2), 97-103
- **R. M. LEWITT et al.** Overview of methods for image reconstruction from projections in emission computed tomography. *Proc. IEEE,* October 2003, vol. 91 (9), 1588-611
- **GARCIA et al.** *Am. J. Cardiol. 51st Annual Scientific Session,* 2002
- **M. KASS ; A. WITKIN ; D. TERZOPOULOS.** *Snakes: active contour models, International Conference on Computer Vision,* 1987, 259-268
- **GREEN P.** Bayesian Reconstructions from Emission Tomography Data using a Modified EM Algorithm. *IEEE Tran. On medical imaging,* March 1990, vol. 9 (1), 84-93
- **FESSLER J.** NSS/MIC statistical image reconstruction short course notes entitled. *Statistical Methods For Image Reconstruction,* 2004

73